(19) Europäisches Patentamt
European Patent Office
Office européen des brevets

(11) **EP 1 010 693 B1**

(12) **FASCICULE DE BREVET EUROPEEN**

(45) Date de publication et mention de la délivrance du brevet:
**24.09.2003 Bulletin 2003/39**

(21) Numéro de dépôt: **99403190.4**

(22) Date de dépôt: **17.12.1999**

(51) Int Cl.[7]: **C07D 233/22**, C07D 233/10, C07D 405/06, C07D 233/20, A61K 31/4164, A61K 31/4178, A61P 25/00

(54) **Nouveaux composés imidazoliniques, leur procédé de préparation et les compositions pharmaceutiques qui les contiennent**

Imidazoline Derivate,ihre Herstellung und pharmazeutische Zusammensetzungen die sie enthalten

Imidazoline derivatives, preparation and pharmaceutical compositions containing them

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **18.12.1998 FR 9815999**

(43) Date de publication de la demande:
**21.06.2000 Bulletin 2000/25**

(73) Titulaire: **LES LABORATOIRES SERVIER**
**92200 Neuilly sur Seine (FR)**

(72) Inventeurs:
- **Cordi, Alex**
  **92150 Suresnes (FR)**
- **Lacoste, Jean-Michel**
  **92310 Sevres (FR)**
- **Millan, Mark**
  **78230 Le Pecq (FR)**
- **Newman-Tancredi, Adrian**
  **78230 Le Pecq (FR)**
- **Gobert, Alain**
  **92500 Rueil-Malmaison (FR)**

(56) Documents cités:
**EP-A- 0 125 410**
**EP-A- 0 166 937**
**EP-A- 0 846 688**
**WO-A-94/07866**
**US-A- 5 151 526**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 010 693 B1

Printed by Jouve, 75001 PARIS (FR)

## Description

La présente invention concerne de nouveaux dérivés imidazoliniques, leur procédé de préparation et les compositions pharmaceutiques qui les contiennent, ainsi que leur utilisation en tant qu'antagonistes α2-adrénergiques et bloqueurs de la recapture de monoamines (sérotonine et/ou noradrénaline).

Le système nerveux adrénergique joue un rôle important à plusieurs niveaux, par exemple artériel, veineux, cardiaque, rénal et au niveau du système nerveux autonome central et périphérique. Dès lors, les produits capables d'interagir avec les récepteurs adrénergiques peuvent induire un grand nombre de réponses physiologiques comme la vasoconstriction, la vasodilatation, l'augmentation ou la diminution du rythme cardiaque, la variation de la force de contraction du muscle cardiaque et des activités métaboliques. Différents composés adrénergiques ont été utilisés dans le passé pour modifier ces réponses physiologiques ou d'autres.

Les composés décrits dans la présente invention, outre le fait qu'ils sont nouveaux, possèdent un profil d'antagonistes α2-adrénergiques et de bloqueurs de la recapture de monoamines, les rendant utiles dans le traitement de la dépression (Drug News & Perspective, 4 (4), 1991). Le problème majeur posé par les antidépresseurs est leur long délai d'efficacité, lié à leur propre mode d'action. Des études ont montré que l'association d'un antagoniste α2-adrénergique avec un inhibiteur de la recapture des monoamines permettait de réduire ce délai (Commun. Psychopharmacol, 4, pp. 95-100, 1980). La combinaison des deux effets sur une seule molécule pourrait donner naissance à une nouvelle génération d'antidépresseurs beaucoup plus efficaces. Parmi ces composés, le napamezole (US 5017584, EP0125410) est décrit comme possédant à la fois une activité antagoniste α2-adrénergique et bloqueur de la recapture de monoamines.

Les composés de la présente invention, de structures nouvelles, présentent un profil sélectif d'antagonistes α2-adrénergiques et un pouvoir d'inhibiteurs de la recapture des monoamines surprenant bien supérieur au napamezole.

La présente invention concerne plus particulièrement les composés de formule (I):

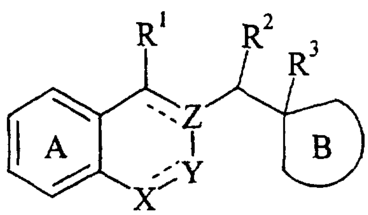

(I)

dans laquelle :

- A représente un noyau benzénique substitué ou non par 1 à 4 groupements identiques ou différents choisis parmi alkyle ($C_1$-$C_6$) linéaire ou ramifié, alkoxy ($C_1$-$C_6$) linéaire ou ramifié, hydroxy, polyhalogénoalkyles ($C_1$-$C_6$) linéaire ou ramifié, cyano, nitro, amino, alkylamino, dialkylamino, thioalkyle, sulfonylalkyle, sulfinylalkyle, carboxy, alkyloxycarbonyle, alkylcarbonyloxy, formyle, carbamoyle, carboxamide, phényle, benzyle, ou atomes d'halogène,
- B représente un cycle imidazolinique tel que représenté dans les formules (Ia) ou (Ib):

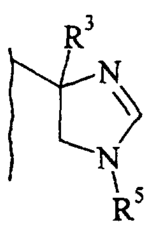

(Ia)

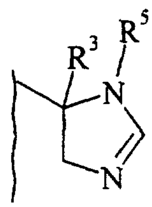

(Ib)

- X représente

- un groupement $CR^6$ ou $CR^6R^7$ (où $R^6$ et $R^7$, identiques ou différents, représentent un atome d'hydrogène ou un groupement alkyle ($C_1$-$C_6$) linéaire ou ramifié),
- un atome d'azote ou un groupement $NR^8$ (dans lequel $R^8$ représente un atome d'hydrogène ou un groupement alkyle ($C_1$-$C_6$) linéaire ou ramifié ou benzyle),
- un atome d'oxygène,
- un atome de soufre ou un groupement SO ou $SO_2$,

♦ Y représente un groupement CH ou $CH_2$ ou une liaison simple (et dans ce cas le cycle contenant X, Y et Z est représenté par la formule (Ic):

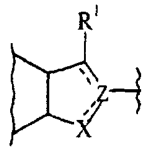

(Ic) )

♦ Z représente un atome de carbone ou un groupement $CR^4$ dans lequel $R^4$ représente un atome d'hydrogène ou un groupement alkyle ($C_1$-$C_6$) linéaire ou ramifié,

♦ $R^1$, $R^2$, $R^3$, identiques ou différents, représentent un atome d'hydrogène ou un groupement alkyle ($C_1$-$C_6$) linéaire ou ramifié,
les groupements ($R^2$ et $R^4$) ou ($R^1$ et $R^4$) pouvant former un cyclopropane,

♦ $R^5$ représente un atome d'hydrogène, un groupement alkyle ($C_1$-$C_6$) linéaire ou ramifié ou benzyle,

♦ la représentation ---- signifie que les liaisons peuvent être simples ou doubles, étant entendu que la valence des atomes est respectée,

étant entendu que par alkyle on entend un groupement alkyle linéaire ou ramifié contenant de 1 à 6 atomes de carbone, leurs tautomères, énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels $R^5$ représente un atome d'hydrogène.

Les cycles B préférés sont ceux représentés par la formule (Ia).

De façon avantageuse, l'invention concerne les composés de formule (I) pour lesquels $R^1$ et $R^2$ représentent simultanément un atome d'hydrogène, $R^3$ représente un atome d'hydrogène ou un groupement méthyle, Z représente un atome de carbone, un groupement CH ou C-$CH_3$, ou ($R^1$ et $R^4$) ou ($R^2$ et $R^4$) représentent un cyclopropane.

Les systèmes cycliques

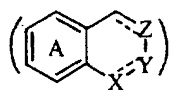

préférés sont le 3,4-dihydronaphtalène, le 1,2,3,4-tétra hydronaphtalène, l'indane, l'indène ou le benzofurane. Plus particulièrement, l'invention concerne les composés 3,4-dihydronaphtalène, 1,2,3,4-tétrahydronaphtalène, indane, indène ou benzofurane non substitués ou substitués par un ou plusieurs substituants, identiques ou différents, choisis parmi l'atome d'halogène ou les groupements méthyle, méthoxy ou $CF_3$.

De façon encore plus préférentielle, l'invention concerne les composés de formule (I) qui sont

- le 4-(3,4-dihydro-2-naphtalénylméthyl)-4,5-dihydro-*1H*-imidazole,
- le 4-[(8-chloro-3,4-dihydro-2-naphtalényl)méthyl]-4,5-dihydro-*1H*-imidazole,
- le 4-(benzo[*b*]furan-2-ylméthyl)-4,5-dihydro-*1H*-imidazole,
- le 4-{spiro[cyclopropan-2:2'-(1',2',3',4'-tétrahydronaphtalèn)]-1-yl}-4,5-dihydro-*1H*-imidazole (isomère 1),
- le 4-{spiro[cyclopropan-2:2'-(1',2',3',4'-tétrahydronaphtalèn)]-1-yl}-4,5-dihydro-*1H*-imidazole (isomère 2),
- le 4-[1,2,3,4-tétrahydro-2-naphtalénylméthyl]-4,5-dihydro-*1H*-imidazole (isomère 1),
- le 4-[1,2,3,4-tétrahydro-2-naphtalénylméthyl]-4,5-dihydro-*1H*-imidazole (isomère 2),
- le 4-[1,2,3,4-tétrahydro-2-naphtalénylméthyl]-4,5-dihydro-*1H*-imidazole (isomère 3),
- le 4-[1,2,3,4-tétrahydro-2-naphtalénylméthyl]-4,5-dihydro-*1H*-imidazole (isomère 4),
- le 4-(1,3-dihydro-*1H*-2-indénylméthyl)-4,5-dihydro-*1H*-imidazole.
- le 4-[(5-fluoro-2,3-dihydro-*1H*-indèn-2-yl)méthyl-4,5-dihydro-*1H*-imidazole,
- le 4-[(5,6-difluoro-2,3-dihydro-*1H*-indèn-2-yl)méthyl-4,5-dihydro-*1H*-imidazole,
- le 4-[(7-fluoro-3,4-dihydro-2-naphtalényl)méthyl]-4,5-dihydro-*1H* imidazole,
- le 4-[(8-méthoxy-3,4-dihydro-2-naphtalényl)méthyl]-4,5-dihydro-*1H*-imidazole,
- le 4-[(3,4-dihydro-4,4-diméthyl-2-naphtalényl)méthyl]-4,5-dihydro-*1H*-imidazole,
- le 4-[(8-chloro-2-naphtyl)méthyl]-4,5-dihydro-*1H*-imidazole,
- le 4-(spiro[cyclopropan-2':2"-(5",6"-dilluoro-indan)]-1'-yl}-4,5-dihydro-*1H*-imidazole (diastéréoisomère 1),
- le 4-{spiro[cyclopropan-2':2"-(5",6"-difluoro-indan)]-1'-yl}-4,5-dihydro-*1H*-imidazole (diastéréoisomère 2).

Les tautomères, énantiomères et diastéréoisomères ainsi que les sels d'addition à un acide ou à une base pharmaceutiquement acceptable des composés préférés de l'invention font partie intégrante de l'invention.

La présente invention concerne également le procédé de préparation des composés de formule (I) caractérisé en ce que l'on utilise comme produit de départ le composé de formule (II):

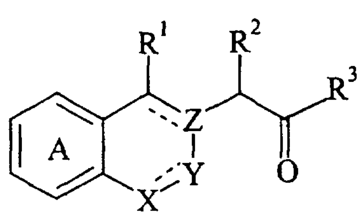

(II)

dans laquelle A, X, Y, Z, $R^1$, $R^2$, $R^3$ et la représentation ---- sont définis comme précédemment, que l'on soumet soit aux conditions de la réaction de Strecker (KCN, $NH_4Cl$), soit à l'action d'une amine $R_aNH_2$ (dans laquelle $R_a$ représente un atome d'hydrogène ou un groupement protecteur encombré permettant une meilleure séparation, lorsqu'il y a lieu, des diastéréoisomères formés) et de cyanure de triméthylsilyle afin de conduire au composé de formule (III):

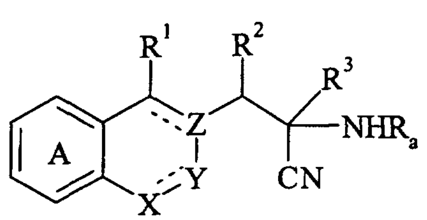

(III)

dans laquelle A, X, Y, Z, $R^1$, $R^2$, $R^3$, $R_a$ et la représentation ---- sont définis comme précédemment, composé de formule (III) qui peut être par ailleurs obtenu, lorsque $R_a$ représente un atome d'hydrogène, à partir du composé de formule (IV) :

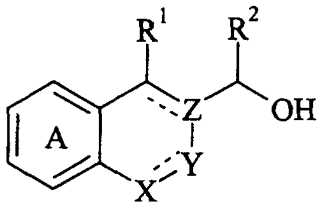

(IV)

dans laquelle A, X, Y, Z, $R^1$, $R^2$ et la représentation ---- sont définis comme précédemment, que l'on soumet à un agent d'halogénation comme $CBr_4/P(Ph)_3$ pour conduire au composé de formule (V):

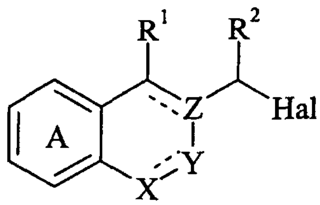

(V)

dans laquelle A, X, Y, Z, $R^1$, $R^2$ et la représentation ---- ont la même définition que précédemment et Hal représente un atome d'halogène,
sur lequel on condense un dérivé de formule (VI):

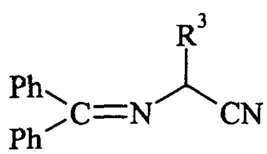

(VI)

dans laquelle $R^3$ est tel que défini précédemment, pour conduire au composé de formule (VII):

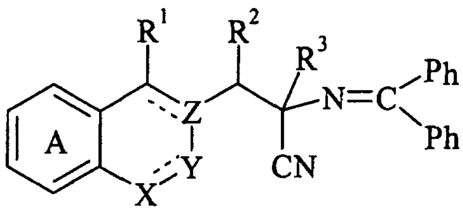

(VII)

dans laquelle A, X, Y, Z, $R^1$, $R^2$, $R^3$ et la représentation ---- sont tels que définis précédement, que l'on soumet à une hydrolyse acide pour conduire au composé de formule (III/a), cas particulier des composés de formule (III):

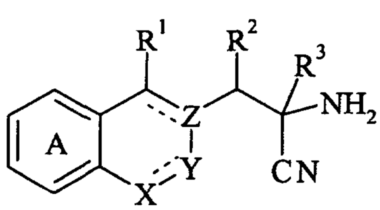

(III/a)

dans laquelle A, X, Y, Z, $R^1$, $R^2$, $R^3$ et la représentation ---- sont définis comme précédemment, composé de formule (III) que l'on soumet à l'action de $LiAlH_4$ ou $AlH_3$ ou à une hydrogénation catalytique au nickel ou cobalt, ou palladium sur charbon ou ruthénium ou rhodium pour obtenir le composé de formule (VIII) :

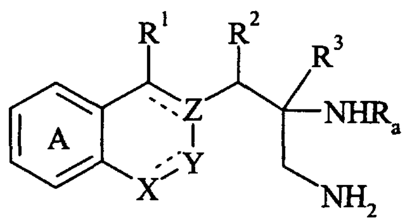

(VIII)

dans laquelle A, X, Y, Z, $R^1$, $R^2$, $R^3$, $R_a$ et la représentation ---- ont la même définition que précédemment, que l'on déprotège dans le cas ou $R_a$ est différent d'un atome d'hydrogène en milieu acide acétique aqueux, ou sous hydrogène en présence de palladium sur charbon avec une quantité catalytique d'acide chlorhydrique, en présence de formiate d'ammonium, ou en présence d'une quantité catalytique de palladium sur charbon dans un solvant tel que l'éthanol, pour conduire au composé de formule (IX) :

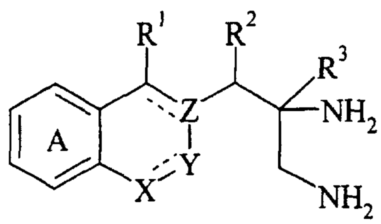

(IX)

dans laquelle A, X, Y, Z, $R^1$, $R^2$, $R^3$ et la représentation ---- sont définis comme précédemment, qui est mis en réaction avec de l'acétate de formamidine ou de l'orthoformiate de méthyle, pour aboutir au composé de formule (I/a), cas particulier des composés de formule (I):

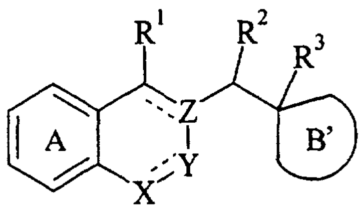

(I/a)

dans laquelle A, X, Y, Z, $R^1$, $R^2$, $R^3$, et la représentation ---- ont la même signification que précédemment, et B' représente un cycle imidazolinique non substitué tel que représenté dans les formules (Ia/a) ou (Ib/a) :

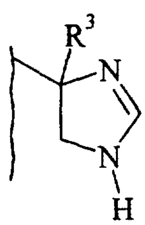

(Ia/a)

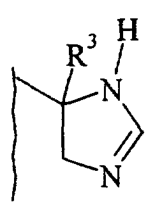

(Ib/a)

que l'on peut soumettre, en présence d'un système basique, à l'action d'un composé de formule (X) :

$$R^5_a\text{-J} \quad (X)$$

dans laquelle $R^5_a$ représente un groupement alkyle ($C_1$-$C_6$) linéaire ou ramifié ou benzyle et J représente un groupe

partant comme un atome d'halogène ou un groupement tosyle, afin d'obtenir le composé de formule (I/b), cas particulier des composés de formule (I) :

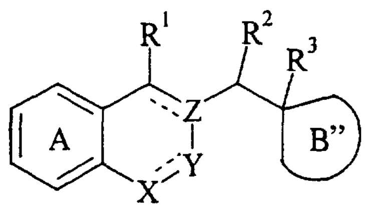

(I/b)

dans laquelle A, X, Y, Z, $R^1$, $R^2$, $R^3$, et la représentation ---- ont la même définition que précédemment, et B" représente un cycle imidazolinique substitué tel que représenté dans les formules (Ia/b) ou (Ib/b) :

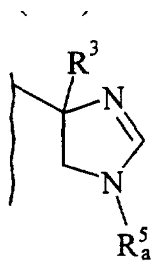

(Ia/b)

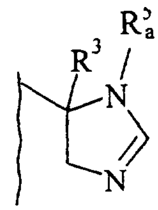

(Ib/b)

où $R^5_a$ est tel que défini précédemment,
les composés de formule (I/a) et (I/b) formant l'ensemble des composés de formule (I) et pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

La présente invention concerne également le procédé de préparation des composés de formule (I/a) caractérisé en ce que l'on utilise comme produit de départ le composé de formule (II') :

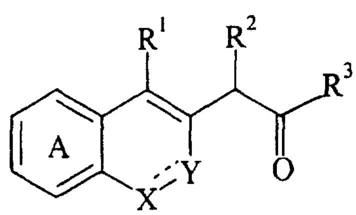

(II')

dans laquelle A, X, Y, $R^1$, $R^2$, $R^3$ et la représentation ---- sont définis comme précédemment,
que l'on condense sur un composé aminé chiral de formule (XI):

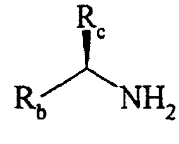

(XI)

dans laquelle $R_b$ et $R_c$, différents, représentent un groupement alkyle ou aryle,
pour obtenir le composé de formule (XII) :

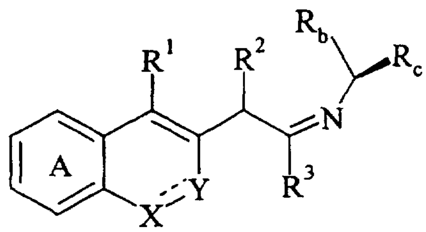

(XII)

dans laquelle A, X, Y, $R^1$, $R^2$, $R^3$, $R_b$, $R_c$ et la représentation ---- ont la même définition que précédemment, que l'on soumet à l'action de N-1-isocyano-cyclohexene et d'acide benzoique pour conduire au composé de formule (XIII) :

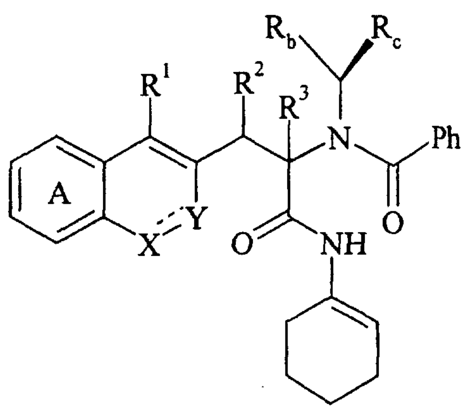

(XIII)

dans laquelle A, X, Y, $R^1$, $R^2$, $R^3$, $R_b$, $R_c$ et la représentation ---- sont tels que définis précédemment, composé de formule (XIII) que l'on chromatographie sur silice pour conduire aux diastéréoisomères de formules ($XIII_a$) et ($XIII_b$) :

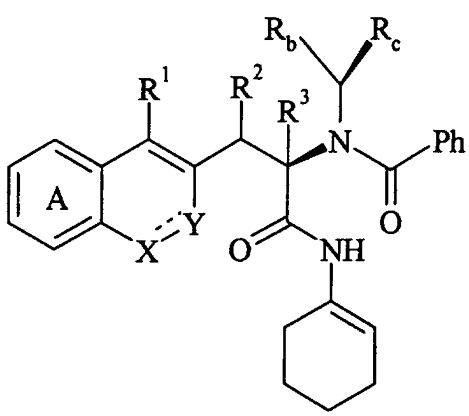

($XIII_a$)

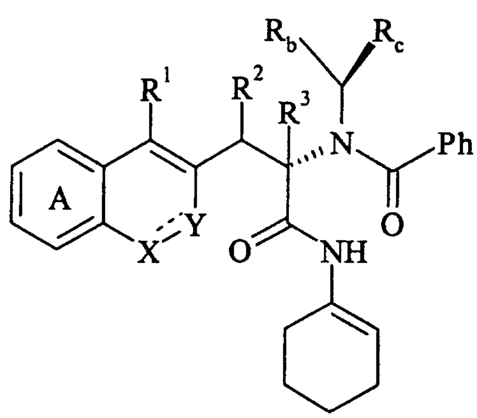

($XIII_b$)

dans lesquelles A, X, Y, $R^1$, $R^2$, $R^3$, $R_b$, $R_c$ et la représentation ---- sont définis comme précédemment, composé de formule ($XIII_a$) que l'on soumet à l'action d'un acide fort tel que l'acide chlorhydrique concentré pour obtenir le composé de formule ($XIV_a$) :

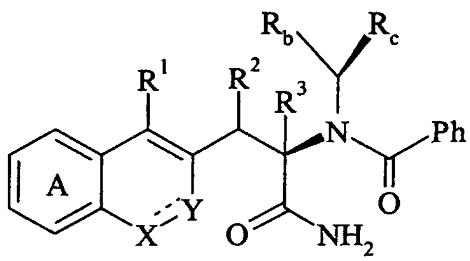

($XIV_a$)

dans laquelle A, X, Y, $R^1$, $R^2$, $R^3$, $R_b$, $R_c$ et la représentation ---- sont tels que définis précédemment,

- que l'on soumet à une hydrogénation catalytique pour conduire au composé de formule ($XV_a$) :

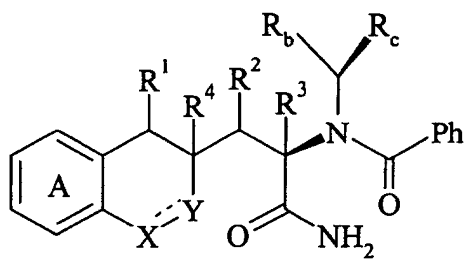

($XV_a$)

dans laquelle A, X, Y, $R^1$, $R^2$, $R^3$, $R^4$, $R_b$, $R_c$ et la représentation ---- sont tels que définis précédemment, composé de formule ($XV_a$) que l'on chromatographie sur silice pour conduire aux diastéréoisomères de formules ($XV_a$') et ($XV_a$'') :

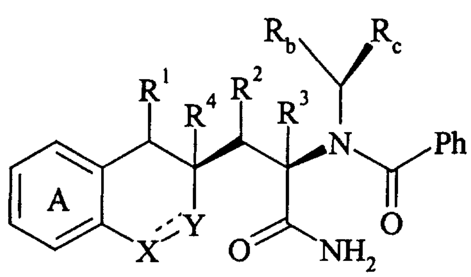

($XV_a$')

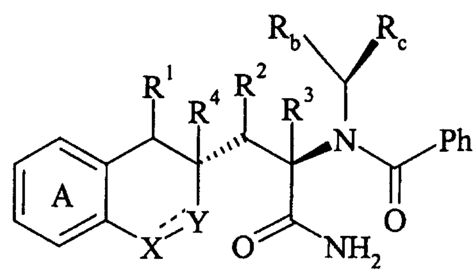

($XV_a$'')

dans lesquelles A, X, Y, $R^1$, $R^2$, $R^3$, $R^4$, $R_b$, $R_c$ et la représentation ---- ont la même signification que précédemment, composé de formule ($XV_a$') que l'on soumet successivement à l'action de $BH_3$, puis à une hydrogénolyse pour conduire au composé de formule ($XVI_a$') :

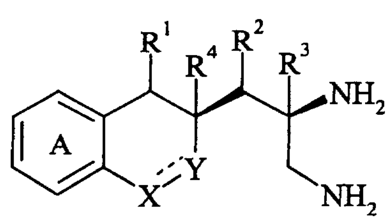

($XVI_a$')

dans laquelle A, X, Y, $R^1$, $R^2$, $R^3$, $R^4$ et la représentation ---- sont tels que définis précédemment, qui est mis en réaction avec de l'acétate de formamidine ou de l'orthoformiate de méthyle pour obtenir le composé de formule ($XVII_a$'), cas particulier des composés de formule (I/a) :

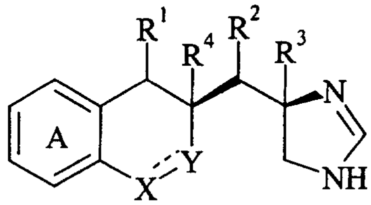

(XVII$_a$')

dans laquelle A, X, Y, $R^1$, $R^2$, $R^3$, $R^4$ et la représentation ---- sont tels que définis précédemment, la même suite réactionnelle permettant d'obtenir :

➢ le composé de formule (XVII$_a$"), cas particulier des composés de formule (I/a), obtenu à partir du diastéréoisomère (XV$_a$"):

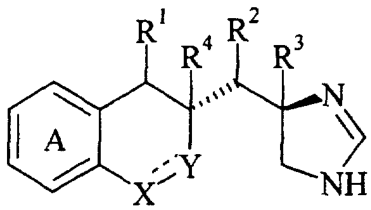

(XVII$_a$")

dans laquelle A, X, Y, $R^1$, $R^2$, $R^3$, $R^4$ et la représentation ---- sont tels que définis précédemment,

➢ le composé de formule (XVII$_b$'), cas particulier des composés de formule (I/a), obtenu à partir du diastéréoisomère (XIII$_b$):

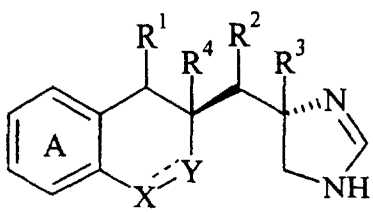

(XVII$_b$')

dans laquelle A, X, Y, $R^1$, $R^2$, $R^3$, $R^4$ et la représentation ---- sont tels que définis précédemment,

➢ le composé de formule (XVII$_b$"), cas particulier des composés de formule (I/a), obtenu à partir du diastéréoisomère (XIII$_b$) :

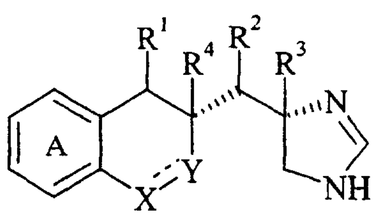

(XVII$_b$")

dans laquelle A, X, Y, $R^1$, $R^2$, $R^3$, $R^4$ et la représentation ---- sont tels que définis précédemment,

- ou composé de formule (XIV$_a$) que l'on soumet successivement à l'action d'un agent réducteur comme $BH_3$, puis à une hydrogénolyse pour conduire au composé de formule (XVIII$_a$) :

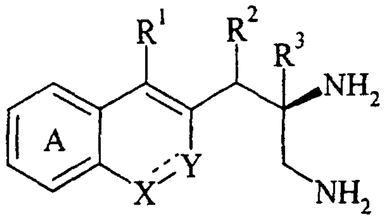

(XVIII$_a$)

dans laquelle A, X, Y, $R^1$, $R^2$, $R^3$ et la représentation ---- sont tels que définis précédemment, qui est mis en réaction avec de l'acétate de formamidine ou de l'orthoformiate de méthyle pour obtenir le composé de formule (XIX$_a$), cas particulier des composés de formule (I/a) :

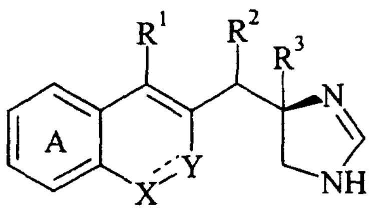

(XIX$_a$)

dans laquelle A, X, Y, $R^1$, $R^2$, $R^3$, et la représentation ---- sont tels que définis précédemment, la même suite réactionnelle permettant d'obtenir le composé de formule (XIX$_b$), cas particulier des composés de formule (I/a) obtenu à partir du composé (XIV$_b$) correspondant :

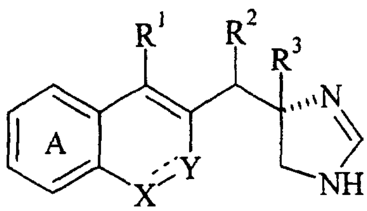

(XIX$_b$)

dans laquelle A, X, Y, $R^1$, $R^2$, $R^3$ et la représentation ---- sont tels que définis précédemment, les composés de formules (XVII$_a$'), (XVII$_a$"), (XVII$_b$'), (XVII$_b$"), (XIX$_a$) et (XIX$_b$) pouvant être purifiés selon une technique classique de séparation que l'on transforme, si on le souhaite, en leurs sels d'addition à un acide ou à une base pharmaceutiquement acceptable. Les composés de formule (II) et (II') sont obtenus par exemple :

♦ à partir du composé de formule (XX) :

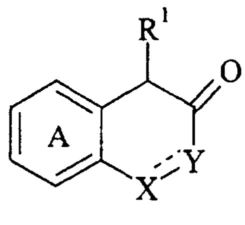

(XX)

dans laquelle A, X, Y, $R^1$ et la représentation ---- sont tels que définis précédemment,

- que l'on soumet à une réaction de Wittig-Homer suivie d'une éventuelle réduction afin de conduire au composé de formule (XXI) :

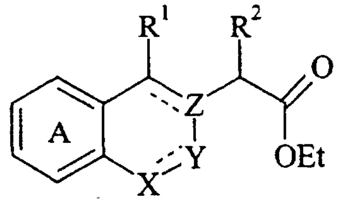

(XXI)

dans laquelle A, X, Y, Z, $R^1$, $R^2$ et la représentation ---- sont tels que définis précédemment, que l'on soumet à une réduction pour obtenir le composé de formule (XXII), cas particulier des composés de formule (II) :

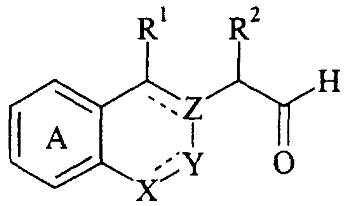

(XXII)

dans laquelle A, X, Y, Z, $R^1$, $R^2$ et la représentation ---- sont tels que définis précédemment,

- ou que l'on soumet à une réaction de Wittig suivie d'une éventuelle réduction afin de conduire au composé de formule (XXIII), cas particulier des composés de formule (II):

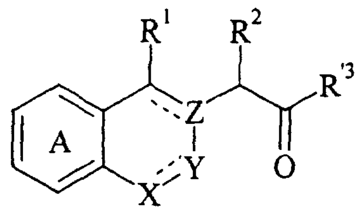

(XXIII)

dans laquelle A, X, Y, Z, $R^1$, $R^2$, et la représentation ---- ont la même signification que précédemment et $R'^3$ représente un groupement alkyle ($C_1$-$C_6$) linéaire ou ramifié,

♦ ou à partir du composé de formule (XXIV) :

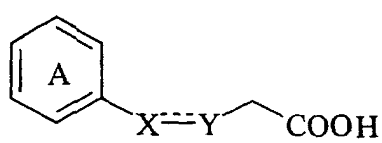

(XXIV)

dans laquelle A, X, Y et la représentation ---- sont tels que définis précédemment, qui est cyclisé (après transformation en chlorure d'acide correspondant) en présence d'$AlCl_3$ pour conduire au composé de formule (XXV) :

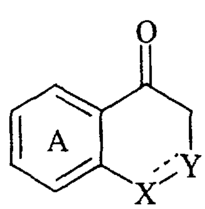

(XXV)

dans laquelle A, X, Y et la représentation ---- ont la même définition que précédemment,

- sur lequel on condense en milieu basique le diéthylchlorophosphate afin de conduire au composé de formule (XXVI) :

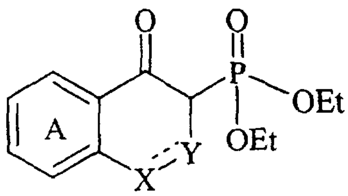

(XXVI)

dans laquelle A, X, Y et la représentation ---- sont définis comme précédemment, sur lequel on condense le composé de formule (XXVII) :

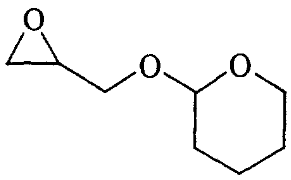

(XXVII)

pour conduire au composé de formule (XXVIII):

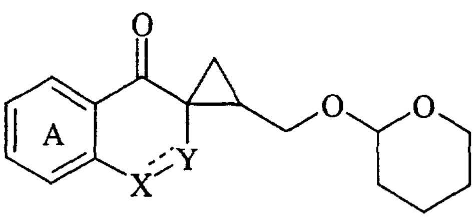

(XXVIII)

dans laquelle A, X, Y et la représentation ---- ont la même signification que précédemment,
qui est soumis à l'action d'un système réducteur afin de conduire au composé de formule (XXIX)

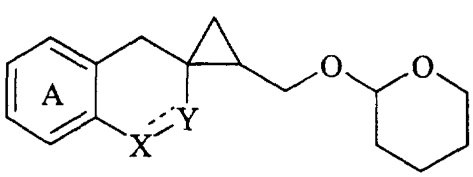

(XXIX)

dans laquelle A, X, Y et la représentation ---- sont définis comme précédemment,
qui peut être hydrolysé dans les conditions de la réaction ou bien isolé et hydrolysé dans une étape consécutive en composé de formule (XXX):

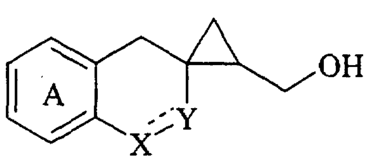

(XXX)

dans laquelle A, X, Y et la représentation ---- sont définis comme précédemment,

- ou composé de formule (XXV) qui est soumis à l'action du 2-[(diéthoxyphosphoryl)oxy]acrylate d'éthyle en milieu basique pour conduire au composé de formule (XXXI) :

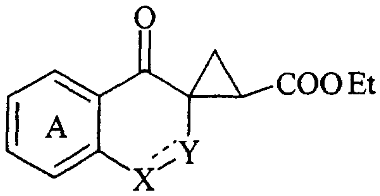

(XXXI)

dans laquelle A, X, Y et la représentation ---- sont définis comme précédemment,
que l'on place en présence d'un agent réducteur comme $NaBH_3CN$ et d'un acide de Lewis tel que l'iodure de zinc pour obtenir le composé de formule (XXXII) :

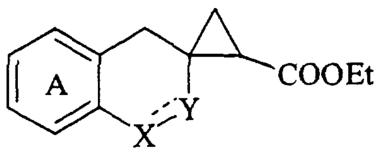

(XXXII)

dans laquelle A, X, Y et la représentation ---- sont tels que définis précédemment,
que l'on soumet à l'action d'un agent réducteur comme $LiAlH_4$ pour conduire au composé de formule (XXVIII) que l'on oxyde pour obtenir le composé de formule (XXXIII), cas particulier des composés de formule (II):

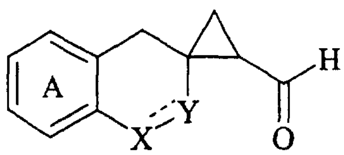

(XXXIII)

dans laquelle A, X, Y et la représentation ---- ont la même signification que précédemment.

Les composés de l'invention et les compositions pharmaceutiques les contenant s'avèrent être utiles pour le traitement de la dépression.

En effet, les composés de la présente invention sont des antagonistes spécifiques α2-adrénergiques et agissent également comme de puissants inhibiteurs de recapture de la sérotonine et de la noradrénaline.

A ce titre, ils peuvent être utilisés en thérapeutique pour le traitement de la dépression, de l'obésité, des attaques de panique, de l'anxiété, des troubles obsessionnels compulsifs, des troubles cognitifs, des phobies, des troubles impulsifs de l'abus et du sevrage de drogue, des dysfonctionnements sexuels et de la maladie de Parkinson.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon le sexe, l'âge et le poids du patient, la voie d'administration, la nature de l'indication thérapeutique, ou des traitements éventuellement associés et s'échelonne entre 1 et 1000 mg par 24 heures en 1 ou plusieurs prises.

Les exemples suivants illustrent l'invention et ne la limitent en aucune façon.

La nomenclature adoptée pour les exemples qui suivent est la substitution en 4 du noyau imidazolinique, mais les exemples comprennent également les tautomères substitués en 5. A titre d'exemple, le composé de l'Exemple 1 (4-(3,4-dihydro-2-naphtalénylméthyl)-4,5-dihydro-*1H*-imidazole, fumarate) s'écrit aussi 5-(3,4-dihydro-2-naphtalénylméthyl)-4,5-dihydro-*1H*-imidazole, fumarate.

**EXEMPLE 1: 4-(3,4-Dihydro-2-naphtalénylméthyl)-4,5-dihydro-*1H*-imidazole, fumarate**

*Stade 1 : 2-(3,4-Dihydro-naphtalèn-2 yl)acétate d 'éthyle*

A une suspension de 6 g (0,25 mole) de NaH dans 600 ml de THF anhydre, maintenue à 0°C, sous atmosphère d'azote est ajoutée goutte à goutte une solution de 56 g (0,25 mole) de triéthylphosphonoacétate dans 70 ml de tétrahydrofurane (THF). Le milieu est ensuite agité à 10°C pendant 30 minutes, refroidi à 0°C puis traité, goutte à goutte, par une solution de 36,52 g (0,25 mole) de β-tétralone dans 50 ml de THF anhydre. Après 3 heures d'agitation à 20°C, le mélange est hydrolysé, à 0°C, par 200 ml d'eau. Le solvant est évaporé sous pression réduite et le résidu est extrait par du dichlorométhane ($CH_2Cl_2$) (3 x 200 ml). Les phases organiques réunies sont lavées par une solution de NaCl saturée, séchées et concentrées pour donner un résidu huileux brun. Une distillation sous vide partiel permet d'obtenir le composé du titre sous forme de liquide incolore.

Point d'ébullition (0,03mm Hg): 88-91°C

| Microanalyse élémentaire : | | |
|---|---|---|
| | C% | H% |
| Théorique | 77,75 | 7,46 |
| Trouvée | 77,59 | 7,57 |

*Stade 2 : 2-(3,4-Dihydro-naphtalèn-2-yl)acétaldéhyde*

Une solution de 18,60 g (0,086 mole) de l'ester obtenu au stade 1 dans 360 ml de $CH_2Cl_2$, refroidie à -60°C, est traitée goutte à goutte par une solution molaire d'hydrure de diisobutyl aluminium dans du $CH_2Cl_2$ (170 ml). Après 2 heures d'agitation à -60°C, le mélange est hydrolysé, à cette température, par additions successives de solutions de $NH_4Cl$ 10% (35 ml) et HCl 1N (42 ml). La température du milieu est remontée à 20°C en 1 heure puis le précipité formé est essoré et lavé par du $CH_2Cl_2$. Le filtrat est lavé par de l'eau puis par une solution saturée de NaCl, séchée et évaporée. Le résidu huileux obtenu est chromatographié sur silice en éluant avec un mélange cyclohexane/EtOAc = 95/5. Le composé attendu est isolé sous forme d'huile incolore et utilisé tel quel pour la réaction suivante.

*Stade 3 : 2-Amino-3-(3,4-dihydro-naphtalèn-2-yl)propionitrile*

A une solution de 3,44 g (20 mmol) de l'aldéhyde obtenu au stade 2 dans 100 ml de $CH_2Cl_2$, sont additionnés successivement, à 20°C, 0,25 g de $ZnI_2$ puis, lentement, 2,18 g (22 mmol) de cyanure de triméthylsilyle. Après 20 heures d'agitation à 20°C, le mélange est concentré sous vide. Le résidu obtenu est traité par 200 ml d'une solution de $NH_3$ dans $CH_3OH$ (7N) et le milieu est agité pendant 4 heures dans un récipient bouché puis concentré sous vide. Le résidu est repris par HCl 1N et la suspension lavée par 50 ml d'$Et_2O$; la phase aqueuse est alcalinisée par NaOH 6N et extraite par du $CH_2Cl_2$ (3 x 50 ml). Les phases organiques réunies sont lavées par une solution saturée de NaCl, séchées et concentrées pour conduire au composé attendu sous forme d'huile, utilisée directement pour la réaction suivante.

*Stade 4 : 3-(3,4-Dihydro-2-naphtalènyl)-propane-1,2-diamine*

Un mélange composé de 2,57 g (13 mmol) du nitrile obtenu au stade 3, 120 ml de $CH_3OH$, 2 g de Nickel de Raney et 50 ml d'une solution de $NH_3$ dans $CH_3OH$ (7N) est hydrogéné sous une pression d'une atmosphère pendant 16 heures. Après filtration sur célite, la solution est concentrée et le résidu chromatographié sur silice en éluant avec un mélange $CH_2Cl_2/CH_3OH/NH_4OH$ conc. = 90/10/1 pour donner le composé attendu sous forme d'une huile incolore.

| Microanalyse élémentaire : | | | |
|---|---|---|---|
| | C% | H% | N% |
| Théorique | 77,18 | 8,97 | 13,85 |
| Trouvée | 76,80 | 8,42 | 13,26 |

*Stade 5 : 4-(3,4-Dihydro-2-naphtalènylméthyl)-4,5-dihydro-*1H*-imidazole, fumarate*

Un mélange composé de 0,505 g (2,5 mmol) du composé obtenu au stade 4 et de 0,286 g (2,75 mmol)

d'acétate de formamidine dans 20 ml d'EtOH est agité à 20°C pendant 16 heures. Après évaporation du solvant, le résidu est repris dans 10 ml de $CH_2Cl_2$; le milieu est traité par 5 ml de NaOH 2 N et extrait par $CH_2Cl_2$ (3 x 20 ml). Les phases organiques réunies sont séchées et concentrées ; l'huile résiduelle est dissoute dans 10 ml d'EtOH et le mélange traité par une solution de 0,29 g (2,5 mmol) d'acide fumarique dans 5 ml d'EtOH. Le composé du titre est isolé après recristallisation.

Point de fusion : 165-166°C

| Microanalyse élémentaire : | | | |
|---|---|---|---|
| | C% | H% | N% |
| Théorique | 65,84 | 6,14 | 8,53 |
| Trouvée | 66,01 | 6,13 | 8,41 |

Les Exemples 2 et 3 sont obtenus après dédoublement du composé obtenu au stade 4 de l'Exemple 1, au moyen de l'acide (+)-dibenzoyl-D-tartrique ou de l'acide (-)-dibenzoyl-L-tartrique par recristallisations successives dans du MeOH puis neutralisation du sel par NaOH 1N et extraction par $CH_2Cl_2$, puis en procédant comme dans le stade 5 de l'Exemple 1.

**EXEMPLE 2 : (*4*R)-4-(3,4-dihydro-2-naphtalénylméthyl)-4,5-dihydro-*1H*- imidazole, fumarate**

*Stade 1 : (2R)-3-(dihydro-2-naphtalényl)-propane-1,2-diamine*

*Stade 2 : (4R)-4-(3,4-dihydro-2-naphtalénylméthyl)-4,5-dihydro-1H-imidazole, fumarate*

Point de fusion : 164-165°C

| Microanalyse élémentaire : | | | |
|---|---|---|---|
| | C% | H% | N% |
| Théorique | 65,84 | 6,14 | 8,53 |
| Trouvée | 65,71 | 6,26 | 8,57 |

**EXEMPLE 3 : (*4S*)-4-(3,4-dihydro-2-naphtalényl)-4,5-dihydro-*1H*-imidazole, fumarate**

*Stade 1 : (2S)-3-(3,4-dihydro-2-naphtalényl)-propane-1,2-diamine*

*Stade 2: (4S)-4-(3,4-dihydro-2-naphtalénylméthyl)-4,5-dihydro-*1H*-imidazole, fumarate*

Point de fusion : 164-165°C

| Microanalyse élémentaire: | | | |
|---|---|---|---|
| | C% | H% | N% |
| Théorique | 65,84 | 6,14 | 8,53 |
| Trouvée | 65,93 | 6,19 | 8,58 |

**EXEMPLE 4: 4-[(6-Méthyl-3,4-dihydro-2-naphtalényl)méthyl]-4,5-dihydro-*1H*-imidazole, fumarate**

*Stade 1 : 2-(6-Méthyl-3,4-dihydro-naphtalèn-2yl)acétate d'éthyle*

On procède comme dans le stade 1 de l'Exemple 1.

Point d'ébullition (0,03mm Hg) : 99-102°C

| Microanalyse élémentaire: | | |
|---|---|---|
| | C% | H% |
| Théorique | 78,23 | 7,88 |
| Trouvée | 78,63 | 8,06 |

<u>Stade 2</u> *: 3-(6-Méthyl-3,4-dihydro-naphtalèn-2-yl)propane-1,2-diamine*

On procède comme dans les stades 2, 3 et 4 de l'Exemple 1.

| Microanalyse élémentaire : | | | |
|---|---|---|---|
| | C% | H% | N% |
| Théorique | 77,73 | 9,32 | 12,95 |
| Trouvée | 77,56 | 9,45 | 12,85 |

<u>Stade 3</u> *: 4-[(6-Méthyl-3,4-dihydro-2-naphtalényl)méthyl]-4,5-dihydro-*1H*-imidazole, fumarate*

<u>Point de fusion</u> : 177-178°C

| Microanalyse élémentaire : | | | |
|---|---|---|---|
| | C% | H% | N% |
| Théorique | 66,65 | 6,48 | 8,18 |
| Trouvée | 66,30 | 6,65 | 8,12 |

**<u>EXEMPLE 5</u>: 4-[(7-Méthyl-3,4-dihydro-2-naphtalényl)méthyl]-4,5-dihydro-*1H*imidazole, fumarate**

<u>Stade 1</u>*: 2-(7-Méthyl-3,4-dihydro-naphtalèn-2-yl)acétate d'éthyle*

On procède comme dans le stade 1 de l'Exemple 1.

<u>Point d'ébullition</u> (0,03mm Hg) : 101-103°C

| Microanalyse élémentaire: | | |
|---|---|---|
| | C% | H% |
| Théorique | 78,23 | 7,88 |
| Trouvée | 78,09 | 7,80 |

<u>Stade 2</u>*: 3-(7-Méthyl-3,4-dihydro-naphtalèn-2-yl)propane-1,2-diamine*

On procède comme dans les stades 2,3 et 4 de l'Exemple 1.

| Microanalyse élémentaire: | | | |
|---|---|---|---|
| | C% | H% | N% |
| Théorique | 77,73 | 9,32 | 12,95 |
| Trouvée | 78,06 | 9,23 | 12,44 |

<u>Stade 3</u> *: 4-[(7-Méthyl-3,4-dihydro-2-naphtalènyl)méthyl]-4,5-dihydro-*1H*-imidazole, fumarate*

<u>Point de fusion</u> : 137-139°C

| Microanalyse élémentaire : | | | |
|---|---|---|---|
| | C% | H% | N% |
| Théorique | 66,65 | 6,48 | 8,18 |
| Trouvée | 66,27 | 6,40 | 8,23 |

**EXEMPLE 6 : 4-[(7-Méthoxy-3,4-dihydro-2-naphtalényl)méthyl]-4,5-dihydro-*1H*-imidazole, fumarate**

*Stade 1: 2-(7-Méthoxy-3,4-dihydro-naphtalèn-2-yl)acétate d'éthyle*

On procède comme dans le stade 1 de l'Exemple 1.

| Microanalyse élémentaire: | | |
|---|---|---|
| | C% | H% |
| Théorique | 73,15 | 7,37 |
| Trouvée | 73,12 | 7,34 |

*Stade 2 : 3-(7-Méthoxy-3,4-dihydro-naphtalèn-2-yl)propane-1,2-diamine*

On procède comme dans les stades 2, 3 et 4 de l'Exemple 1.

| Microanalyse élémentaire : | | | |
|---|---|---|---|
| | C% | H% | N% |
| Théorique | 72,38 | 8,68 | 12,06 |
| Trouvée | 72,74 | 8,68 | 11,79 |

*Stade 3 : 4-[(7-Méthoxy-3,4-dihydro-2-naphtalènyl )méthyl]-4,5-dihydro-1H-imidazole, fumarate*

Point de fusion : 136-138°C

| Microanalyse élémentaire : | | | |
|---|---|---|---|
| | C% | H% | N% |
| Théorique | 63,68 | 6,19 | 7,82 |
| Trouvée | 63,43 | 6,40 | 7,65 |

**EXEMPLE 7 : 4-[(7-Fluoro-3,4-dihydro-2-naphtalényl)méthyl]-4,5-dihydro-*1H*-imidazole, fumarate**

*Stade 1 : 2-(7-Fluoro-3,4-dihydro-naphtalèn-2-yl)acétate d'éthyle*

On procède comme dans le stade 1 de l'Exemple 1.
Point d'ébullition (0,02mm Hg) : 92-94°C

| Microanalyse élémentaire : | | |
|---|---|---|
| | C% | H% |
| Théorique | 71,78 | 6,45 |
| Trouvée | 72,07 | 6,63 |

*Stade 2 : 3-(7-Fluoro-3,4-dihydro-naphtalèn-2-yl)propane-1,2-diamine*

On procède comme dans les stades 2,3 et 4 de l'Exemple 1.

| Microanalyse élémentaire : | | | |
| --- | --- | --- | --- |
| | C% | H% | N% |
| Théorique | 70,88 | 7,78 | 12,72 |
| Trouvée | 70,97 | 7,59 | 12,56 |

*Stade 3 : 4-[(7-Fluoro-3,4-dihydro-2-naphtalényl)méthyl]-4,5-dihydro-*1H-*imidazole, fumarate*

Point de fusion : 155-157°C

| Microanalyse élémentaire : | | | |
| --- | --- | --- | --- |
| | C% | H% | N% |
| Théorique | 62,42 | 5,53 | 8,09 |
| Trouvée | 62,27 | 5,77 | 8,06 |

**EXEMPLE 8: 4-[7-Trifluorométhyl-3,4-dihydro-2-naphtalényl)méthyl]-4,5-dihydro-*1H*-imidazole, fumarate**

*Stade 1 : 2-(7-Trifluorométhyl-3,4-dihydro-naphtalèn-2-yl)acétate d'éthyle*

On procède comme dans le stade 1 de l'Exemple 1.

| Microanalyse élémentaire: | | |
| --- | --- | --- |
| | C% | H% |
| Théorique | 63,38 | 5,32 |
| Trouvée | 63,61 | 5,73 |

*Stade 2 : 3-(7-Trifluorométhyl-3,4-dihydro-naphtalèn-2-yl)propane-1,2-diamine*

On procède comme dans les stades 2, 3 et 4 de l'Exemple 1.

| Microanalyse élémentaire : | | | |
| --- | --- | --- | --- |
| | C% | H% | N% |
| Théorique | 62,21 | 6,34 | 10,36 |
| Trouvée | 62,28 | 6,18 | 10,34 |

*Stade 3 :4-[7-Trifluorométhyl-3,4-dihydro-2-naphtalényl)méthyl]-4,5-dihydro-*1H-*imidazole, fumarate*

Point de fusion: 169-171°C

| Microanalyse élémentaire : | | | |
| --- | --- | --- | --- |
| | C% | H% | N% |
| Théorique | 57,78 | 4,83 | 7,07 |
| Trouvée | 57,72 | 5,25 | 7,03 |

**EXEMPLE 9 : 4-[(8-Fluoro-3,4-dihydro-2-naphtalényl)méthyl)-4,5-dihydro-*1H*-imidazole, fumarate**

*Stade 1 : 2-(8-Fluoro-3,4-dihydro-naphtalèn-2-yl)acétate d'éthyle*

On procède comme dans le stade 1 de l'Exemple 1.

| Microanalyse élémentaire : | | |
|---|---|---|
| | C% | H% |
| Théorique | 71,78 | 6,45 |
| Trouvée | 71,67 | 6,73 |

*Stade 2: 3-(8-Fluoro-3,4-dihydro-naphtalèn-2-yl)propane-1,2-diamine*

On procède comme dans les stades 2,3 et 4 de l'Exemple 1.

| Microanalyse élémentaire : | | | |
|---|---|---|---|
| | C% | H% | N% |
| Théorique | 70,88 | 7,78 | 12,72 |
| Trouvée | 71,05 | 7,86 | 12,48 |

*Stade 3 : 4-[(8-Fluoro-3,4-dihydro-2-naphtalényl)méthyl]-4,5-dihydro-*1H*-imidazole, fumarate*

Point de fusion : 183-185°C

| Microanalyse élémentaire: | | | |
|---|---|---|---|
| | C% | H% | N% |
| Théorique | 62,42 | 5,53 | 8,09 |
| Trouvée | 62,24 | 5,63 | 8,04 |

**EXEMPLE 10 : 4-[(8-Chloro-3,4-dihydro-2-naphtalényl)méthyl)-4,5-dihydro-*1H*-imidazole, fumarate**

*Stade 1: 2-(8-Chloro-3,4-dihydro-naphtalèn-2-yl)acétate d'éthyle*

On procède comme dans le stade 1 de l'Exemple 1.
Point d'ébullition (0,02mm Hg) : 112-114°C

*Stade 2 : 3-(8-Chloro-3,4-dihydro-naphtalèn-2-yl)propane-1,2-diamine*

On procède comme dans les stades 2,3 et 4 de l'Exemple 1.

| Microanalyse élémentaire : | | | | |
|---|---|---|---|---|
| | C% | H% | N% | Cl% |
| Théorique | 65,95 | 7,24 | 11,83 | 14,97 |
| Trouvée | 65,72 | 7,05 | 11,92 | 14,96 |

*Stade 3 : 4-[(8-Chloro-3,4-dihydro-2-naphtalényl)méthyl]-4,5-dihydro-*1H*-imidazole, fumarate*

Point de fusion : 191-195°C

| Microanalyse élémentaire : | | | | |
|---|---|---|---|---|
| | C% | H% | N% | Cl% |
| Théorique | 59,59 | 5,28 | 7,72 | 9,77 |
| Trouvée | 59,55 | 5,36 | 7,66 | 9,89 |

**EXEMPLE 11: 4-[(8-Méthyl-3,4-dihydro-2-naphtalényl)méthyl]-4,5-dihydro-*1H*-imidazole, fumarate**

*Stade 1 : 2-(8-Méthyl-3,4-dihydro-naphtalèn-2-yl)acétate d'éthyle*

On procède comme dans le stade 1 de l'Exemple 1.

Point d'ébullition (0,02mm Hg) : 95-97°C

*Stade 2 : 3-(8-Méthyl-3,4-dihydro-naphtalèn-2-yl)propane-1,2-diamine*

On procède comme dans les stades 2,3 et 4 de l'Exemple 1.

| Microanalyse élémentaire: | | | |
|---|---|---|---|
| | C% | H% | N% |
| Théorique | 77,73 | 9,32 | 12,95 |
| Trouvée | 77,94 | 9,17 | 12,65 |

*Stade 3 : 4-[(8-Méthyl-3,4-dihydro-2-naphtalényl)méthy]-4,5-dihydro-*1H*-imidazole, fumarate*

Point de fusion : 182-184°C

| Microanalyse élémentaire : | | | |
|---|---|---|---|
| | C% | H% | N% |
| Théorique | 66,65 | 6,49 | 8,18 |
| Trouvée | 66,08 | 6,44 | 7,98 |

**EXEMPLE 12: 4-[(8-Méthoxy-3,4-dihydro-2-naphtalényl)méthyl]-4,5-dihydro-*1H*-imidazole, fumarate**

*Stade 1 : 2-(8-Méthoxy-3,4-dihydro-naphtalèn-2-yl)acétate d'éthyle*

On procède comme dans le stade 1 de l'Exemple 1.

| Microanalyse élémentaire : | | |
|---|---|---|
| | C% | H% |
| Théorique | 73,15 | 7,37 |
| Trouvée | 73,50 | 7,34 |

*Stade 2 : 3-(8-Méthoxy-3,4-dihydro-naphtalèn-2-yl)prapane-1,2-diamine*

On procède comme dans les stades 2, 3 et 4 de l'Exemple 1.

| Microanalyse élémentaire: | | | |
|---|---|---|---|
| | C% | H% | N% |
| Théorique | 72,38 | 8,68 | 12,06 |
| Trouvée | 72,67 | 8,49 | 11,96 |

*Stade 3 : 4-[(8-Méthoxy-3,4-dihydro-2-naphtalényl)méthyl]-4,5-dihydro-*1H*-imidazole, fumarate*

Point de fusion : 206-208°C

| Microanalyse élémentaire : | | | |
|---|---|---|---|
| | C% | H% | N% |
| Théorique | 63,68 | 6,19 | 7,82 |
| Trouvée | 63,00 | 6,26 | 7,43 |

**EXEMPLE 13: 4-(*2H*-3-Chroménylméthyl)-4,5-dihydro-*1H*-imidazole**

*Stade 1 : 3-(2H-3-Chroménÿl)-1,2-propanediamine*

On procède comme dans les stades 1, 2, 3 et 4 de l'Exemple 1.

| Microanalyse élémentaire : | | | |
|---|---|---|---|
| | C% | H% | N% |
| Théorique | 70,56 | 7,90 | 13,72 |
| Trouvée | 70,28 | 7,77 | 13,85 |

*Stade 2 : 4-(2H-3-Chroménylméthyl)-4,5-dihydro-*1H*-imidazole*

Point de fusion : 148-150°C

| Microanalyse élémentaire : | | | |
|---|---|---|---|
| | C% | H% | N% |
| Théorique | 61,81 | 5,49 | 8,48 |
| Trouvée | 61,57 | 5,60 | 8,50 |

**EXEMPLE 14: 4-(3,4-Dihydro-2-naphtalénylméthyl)-4-méthyl-4,5-dihydro-*1H*-imidazole, fumarate**

*Stade 1 : 2-Amino-2-méthyl-3-(3,4-dihydro-naphtalèn-2-yl)propionitrile*

A une solution de 6,50 g (35 mmol) de 1-(3,4-dihydronaphtalèn-2-yl)propan-2-one dans un mélange de 41 ml de $CH_3OH$ et 20 ml d'eau sont ajoutés successivement, en une fois, 2,34 g (36 mmol) de KCN et 1,93 g (36 mmol) de $NH_4Cl$. Après 72 heures d'agitation à 20°C, le milieu est dilué par $H_2O$, extrait par $CH_2Cl_2$ (4 x 50 ml). Les solvants sont évaporés, le résidu est repris par $Et_2O$ et à nouveau extrait par HCl 1N (3 x 40 ml). Les phases aqueuses réunies sont alcalinisées par NaOH 6N puis extraites par $CH_2Cl_2$ (3 x 50 ml). Après séchage et concentration des phases organiques, le produit du titre est obtenu sous forme d'huile jaune utilisée directement pour la réaction suivante.

*Stade 2 : 3-(3,4-Dihydro-naphtalèn-2-yl)-2-méthyl-propane-1,2-diamine*

Une solution de 5 g (23,6 mmol) du composé obtenu au stade précédent dans 20 ml de THF anhydre est additionnée goutte à goutte à une suspension de 1,90 g (50 mmol) de $LiAlH_4$ dans 50 ml de THF anhydre, en maintenant la température inférieure à 10°C. Après 1 heure d'agitation à 20°C, le milieu est refroidi à 0°C et hydrolysé par additions successives de 11 ml d'isopropanol et 11 ml d'une solution saturée de NaCl. Le précipité formé est essoré et rincé par du THF ; le filtrat est concentré sous vide et le résidu est repris par HCl 1N (50 ml) puis lavé à l'éther (10 ml). La phase aqueuse est basifiée par NaOH 6N et extraite par $CH_2Cl_2$ (3 x 50 ml). Les phases organiques sont séchées, concentrées et le résidu obtenu est chromatographié sur silice, avec un mélange $CH_2Cl_2/CH_3OH/NH_4OH$ conc. = 90/10/1 comme éluant. Le produit du titre est isolé sous forme d'huile.

*Stade 3 : 4-(3,4-Dihydro-2-naphtalénylméthyl)-4-méthyl-4,5-dihydro-*1 H*-imidazole, fumarate*

On procède comme dans le stade 5 de l'Exemple 1 à partir du composé obtenu au stade 2.
Point de fusion : 146-148°C

| Microanalyse élémentaire: | | | |
|---|---|---|---|
| | C% | H% | N% |
| Théorique | 66,65 | 6,48 | 8,18 |
| Trouvée | 66,24 | 6,50 | 8,14 |

**EXEMPLE 15: 4-[(3,4-Dihydro-4,4-diméthyl-2-naphtalényl)méthyl]-4,5-dihydro-*1H*-imidazole, hémifumarate**

On procède comme dans l'Exemple 14 à partir du (4,4-diméthyl-3,4-dihydronaphtalén-2-yl)acétaldéhyde.
Point de fusion : 178-179°C

| Microanalyse élémentaire: | | | |
|---|---|---|---|
| | C% | H% | N% |
| Théorique | 71,86 | 7,36 | 9,21 |
| Trouvée | 71,93 | 7,40 | 9,13 |

**EXEMPLE 16: 4-[1,2,3,4-Tétrahydro-2-naphtalénylméthyl]-4,5-dihydro-*1H*-imidazole, fumarate**

*Stade 1 : (1,2,3,4-Tétrahydro-naphtalèn-2-yl)acétate d'éthyle*

Un mélange de 26 g (0,12 mole) de l'ester obtenu dans le stade 1 de l'Exemple 1 et de 2,50 g de Pd/C 10% dans 250 ml d'EtOH est hydrogéné sous 1 bar, à 20°C pendant une nuit. Après filtration du catalyseur et lavage à l'éthanol, le filtrat est concentré sous vide pour conduire au composé attendu sous forme d'une huile incolore.
Point d'ébullition (0,02mm Hg) : 90-94°C

| Microanalyse élémentaire: | | |
|---|---|---|
| | C% | H% |
| Théorique | 77,03 | 8,31 |
| Trouvée | 77,49 | 8,16 |

*Stade 2 : (1,2,3,4-Tétrahydro-naphtalèn-2-yl)acétaldéhyde*

On procède comme dans le stade 2 de l'Exemple 1 à partir du composé obtenu au stade 1.

| Microanalyse élémentaire: | | |
|---|---|---|
| | C% | H% |
| Théorique | 82,72 | 8,10 |
| Trouvée | 82,89 | 7,98 |

*Stade 3 : 3-(1,2,3,4-Tétrahydro-naphtalèn-2-yl)propane-1,2-diamine*

On procède comme dans les stades 3 et 4 de l'Exemple 1 à partir du composé obtenu au stade 2.

| Microanalyse élémentaire : | | | |
|---|---|---|---|
| | C% | H% | N% |
| Théorique | 76,42 | 9,87 | 13,71 |
| Trouvée | 76,10 | 9,76 | 13,95 |

*Stade 4 : 4-[1,2,3,4-Tétrahydro-2-naphtalènylméthyl]4,5-dihydro-*1H*-imidazole, fumarate*

On procède comme dans le stade 5 de l'Exemple 1 à partir du composé obtenu au stade 3.

Point de fusion: 158-159°C

| Microanalyse élémentaire : | | | |
|---|---|---|---|
| | C% | H% | N% |
| Théorique | 65,44 | 6,71 | 8,48 |
| Trouvée | 65,66 | 6,82 | 8,52 |

**EXEMPLE 17 : 4-[1,2,3,4-tétrahydro-2-naphtalénylméthyl]-4,5-dihydro-*1H*-imidazole, fumarate (isomère 1)**

*Stade 1 : 4-(1,2,3,4-Tétrahydro-2-naphtalénylméthyl)-1-trityl-4,5-dihydro*-1H-*imidazole*

A une solution du composé obtenu dans l'Exemple 16 (stade 4) sous forme d'amine libre (1,80 g, 8,40 mmol) dans 50 ml de benzène, sous azote, sont ajoutés successivement, à 20°C, 1,25 ml (9 mmol) de triéthylamine et 2,36 g (8,50 mmol) de chlorure de trityle. Après 15 heures d'agitation à 20°C le mélange est lavé par $H_2O$, la phase organique est séchée et concentrée sous vide pour conduire à un résidu huileux. Une chromatographie sur colonne chirale, en éluant avec une solution de triétylamine dans le toluène (0,5%) permet de séparer les 4 isomères sous forme de solides blancs.

*Stade 2 : 4[1,2,3,4-tétrahydro-2-naphtalénylméthyl]-4,5-dihydro*-1H-*imidazole, fumarate (isomère 1)*

Une solution d'un des isomères obtenus au stade 1 (550 mg, 1,2 mmol) dans un mélange $AcOH/H_2O$ = 90/1 est chauffée à reflux pendant 2h30. Après refroidissement le mélange est concentré sous vide ; le résidu est repris par $CH_2Cl_2$ (10 ml) et traité par une solution de NaOH 1N. La phase organique est lavée par une solution saturée de NaCl, séchée et évaporée pour conduire à un résidu huileux qui est chromatographié sur silice, en éluant avec un mélange $CH_2Cl_2/CH_3OH/N_4OH$ = 90/10. L'huile obtenue (170 mg, 0,8 mmol) est dissoute dans EtOH et traitée par 93 mg (0,8 mmol) d'acide fumarique en solution dans 5 ml d'EtOH. Après concentration de la solution et recristallisation dans de l'iPrOH, le composé du titre est obtenu sous forme de prismes incolores.

Point de fusion : 155-156°C

| Microanalyse élémentaire : | | | |
|---|---|---|---|
| | C% | H% | N% |
| Théorique | 65,44 | 6,71 | 8,48 |
| Trouvée | 65,24 | 6,92 | 8,53 |

En procédant comme dans le stade 2 de l'Exemple 17 à partir des autres isomères, on obtient les Exemples 18 à 20 :

**EXEMPLE 18 : 4-[1,2,3,4-tétrahydro-2-naphtalénylméthyl]-4,5-dihydro-*1H*-imidazole, fumarate (isomère 2)**

Point de fusion : 155°C

| Microanalyse élémentaire : | | | |
|---|---|---|---|
| | C% | H% | N% |
| Théorique | 65,44 | 6,71 | 8,48 |
| Trouvée | 65,28 | 6,90 | 8,41 |

**EXEMPLE 19 :4-[1,2,3,4-tétrahydro-2-naphtalénylméthyl]-4,5-dihydro-*1H*-imidazole, fumarate (isomère 3)**

Point de fusion : 159°C

| Microanalyse élémentaire : | | | |
|---|---|---|---|
| | C% | H% | N% |
| 'Théorique | 65,44 | 6,71 | 8,48 |
| Trouvée | 65,69 | 7,01 | 8,49 |

**EXEMPLE 20 : 4-(1,2,3,4-tétrahydro-2-naphtalénylméthyl]-4,5-dihydro-*1H*-imidazole, fumarate (isomère 4)**

Point de fusion : 159°C

| Microanalyse élémentaire : | | | |
|---|---|---|---|
| | C% | H% | N% |
| Théorique | 65,44 | 6,71 | 8,48 |
| Trouvée | 64,92 | 7,01 | 8,48 |

**EXEMPLE 21: 4-(*1H*-Indén-2-ylméthyl)-4,5-dihydro-*1H*-imidazole, fumarate**

On procède comme dans l'Exemple 1 à partir de la 2-indanone.

Point de fusion: 161-162°C

| Microanalyse élémentaire : | | | |
|---|---|---|---|
| | C% | H% | N% |
| Théorique | 64,96 | 5,77 | 8,91 |
| Trouvée | 65,34 | 5,76 | 8,82 |

**EXEMPLE 22 : 4-(2,3-Dihydro-*1H*-indén-2-ylméthyl)-4,5-dihydro-*1H*-imidazole, hémifumarate**

On procède comme dans l'Exemple 16 à partir du (*1H*-indén-2-yl)acétate d'éthyle.

Point de fusion : 208-210°C

| Microanalyse élémentaire : | | | |
|---|---|---|---|
| | C% | H% | N% |
| Théorique | 69,75 | 7,04 | 10,85 |
| Trouvée | 69,79 | 6,69 | 10,73 |

**EXEMPLE 23 : 4-(Benzo[*b*]furan-2-ylméthyl)-4,5-dihydro-*1H*-imidazole, fumarate**

*Stade 1: 2-(Bromométhyl)benzo[b]furane*

A une solution, refroidie à 0°C, de 10,40 g (70 mmol) de 2-hydroxyméthyl-benzo[*b*]furane et 46 g (139 mmol) de $CBr_4$ dans 220 ml d'éther, sont ajoutés, en une fois sous forte agitation, 36,40 g (139 mmol) de triphénylphosphine. Après 4 heures d'agitation à 20°C, le précipité formé est essoré, lavé par $Et_2O$ ; le filtrat est séché, concentré, puis le résidu est chromatographié sur silice en éluant avec un mélange cyclohexane/$CH_2Cl_2$ = 80/20 pour donner le composé attendu sous forme d'huile.

| Microanalyse élémentaire : | | | |
|---|---|---|---|
| | C% | H% | N% |
| Théorique | 51,22 | 3,34 | 37,86 |
| Trouvée | 50,98 | 3,25 | 38,02 |

*<u>Stade 2 :</u> 2-Amino-3-benzo[*b*]furan-2-yl propanenitrile*

A une solution refroidie à 0°C, contenant 4 g (18 mmol) de N-(diphénylméthylène) aminoacétonitrile, 0,53 g (1,6 mmol) de bromure de tétrabutyl ammomium dans un mélange de 55 ml de toluène et 4,40 g de NaOH à 40%, sont additionnés, goutte à goutte, sous azote, 4,40 g (22 mmol) du composé obtenu au stade 1. Après une nuit d'agitation à 20°C, le milieu est dilué par de l'eau, extrait par $CH_2Cl_2$ (3 x 80 ml) ; les phases organiques réunies sont lavées par $H_2O$ puis par une solution saturée de NaCl et concentrées sous vide. Le résidu est repris dans 100 ml d'éther et traité, sous forte agitation, par 100 ml d'HCl 1N. Le mélange est agité à 20°C pendant 12 heures puis décanté, la phase aqueuse est alcalinisée par NaOH 6N puis extraite par $CH_2Cl_2$ (3 x 50 ml) et les phases organiques sont lavées par une solution saturée de NaCl, séchées et concentrées. Le composé du titre est obtenu sous forme d'huile brune utilisée sans autre purification pour la réaction suivante.

*<u>Stade 3</u> : 3-Benzo[b]furan-2-yl-1,2-propanediamine*

On procède comme dans le stade 2 de l'Exemple 14 à partir du nitrile obtenu dans le stade 2.

| Microanalyse élémentaire : | | | |
|---|---|---|---|
| | C% | H% | N% |
| Théorique | 69,45 | 7,42 | 14,72 |
| Trouvée | 69,61 | 7,43 | 14,34 |

*<u>Stade 4</u> : 4-(Benzo[*b*]furan-2-ylméthyl)-4,5-dihydro-*1H*-imidazole, fumarate*

On procède comme dans le stade 5 de l'Exemple 1 à partir de la diamine obtenue au stade 3.

<u>Point de fusion</u> : 154-156°C

| Microanalyse élémentaire : | | | |
|---|---|---|---|
| | C% | H% | N% |
| Théorique | 60,76 | 5,10 | 8,86 |
| Trouvée | 60,54 | 5,19 | 8,82 |

En procédant comme dans l'Exemple 23, on obtient les Exemples 24 et 25 :

**<u>EXEMPLE 24:</u> 4-[(5-Chloro-2,3-dihydro-*1H*-2-indényl)méthyl]-4,5-dihydro-*1H*-imidazole, hémifumarate**

| Microanalyse élémentaire : | | | |
|---|---|---|---|
| | C% | H% | N% |
| Théorique | 65,20 | 6,20 | 10,14 |
| Trouvée | 64,82 | 6,20 | 9,90 |

**<u>EXEMPLE 25</u> : 4-(2-Naphtylméthyl)-4,5-dihydro-*1H*-imidazole, fumarate**

<u>Point de fusion</u> : 149-150°C

| Microanalyse élémentaire : | | | |
|---|---|---|---|
| | C% | H% | N% |
| Théorique | 66,25 | 5,56 | 8,58 |
| Trouvée | 65,97 | 5,65 | 8,44 |

**EXEMPLE 26: 4-[1a,2,3,7b-Tétrahydro-*1H*-cyclopropa[*a*]naphtalèn-1-ylméthyl]-4,5-dihydro-*1H*-imidazole, fumarate**

*Stade 1: 1-(1a,2,3,7b-Tétrahydro-*1H-*cyclopropa[a]naphtalèn-1-yl) acétate* d'éthyle

A une solution de 7,20 g (33 mmol) du composé obtenu dans l'Exemple 1 (stade 1) dans 75 ml de dichloro-1,2 éthane, refroidie à -25°C, sont additionnés successivement, goutte à goutte, 200 ml (20 mmol) d'une solution de diéthylzinc dans l'hexane (1M) et une solution de 71 g (400 mmol) de chloroiodométhane dans 125 ml de dichloro-1,2-éthane. La température du milieu est remontée à 10°C et l'agitation maintenue à cette température pendant 4 heures. Après refroidissement à 0°C, le mélange est traité par 50 ml de $NH_4Cl$ saturée puis par 100 ml d'eau et extrait par $Et_2O$ (3 x 200 ml). Les phases organiques réunies sont lavées par une solution saturée de NaCl, séchées et concentrées sous vide pour conduire au composé du titre, sous forme d'huile.

| Microanalyse élémentaire : | | |
|---|---|---|
| | C% | H% |
| Théorique | 78,23 | 7,88 |
| Trouvée | 78,53 | 7,64 |

*Stade 2: 2-(1a,2,3,7b-Tétrahydro-*1H-*cyclopropa[a]naphtalèn-1-yl)acétaldéhyde*

On procède comme dans le stade 2 de l'Exemple 1 à partir du composé obtenu au stade 1.

| Microanalyse élémentaire : | | |
|---|---|---|
| | C% | H% |
| Théorique | 83,83 | 7,58 |
| Trouvée | 83,72 | 7,63 |

*Stade 3 : 4-[1a,2,3,7b-Tétrahydro-*1H-*cyclopropa[a]naphtalèn-1-ylméthyl]-4,5-dihydro-*1H-*imidazole, fumarate*

On procède comme dans les stades 1, et 3 de l'Exemple 14 à partir de l'aldéhyde obtenu au stade 2. Après réaction avec l'acétate de formamidine, le composé obtenu dans l'Exemple 26 est constitué d'un mélange de diastéréoisomères (50/50) qui sont séparés par chromatographie sur silice en éluant avec un mélange $CH_2Cl_2/CH_3OH/NH_4OH$ = 95/5/0,5. Chaque diastéréoisomère isolé est salifié de façon classique, en présence d'acide fumarique et recristallisé dans un mélange acétone/iPrOH.

**EXEMPLE 27 : (*4R*)-4-[(*1aR, 7bR*)-1a,2,3,7b-Tétrahydro-*1H*-cyclopropa[*a*] naphtalèn-1-ylméthyl]-4,5-dihydro-*1H*-imidazole, fumarate**

**et *(4S)*-4-[*(1aS,7bS)*-1a,2,3,7b-Tétrahydro-*1H*-cyclopropa[*a*] naphtalèn-1-ylméthyl]-4,5-dihydro-*1H*-imidazole, fumarate**

Point de fusion : 192°C

| Microanalyse élémentaire : | | | |
|---|---|---|---|
| | C% | H% | N% |
| Théorique | 66,65 | 6,48 | 8,18 8 |
| Trouvée | 66,50 | 6,52 | 8,08 |

**EXEMPLE 28: (*4S*)-4-[(*1aR,7bR*)-1a,2,3,7b-Tétrahydro-*1H*-cyclopropa[*a*] naphtalèn-1-ylméthyl]-4,5-dihydro-*1H*-imidazole, fumarate**

**et (*4R*)-4-[(*1aS, 7bS*)-1a,2,3,7b-Tétrahydro-*1H*-cyclopropa[*a*] naphtalèn-1-ylméthyl]-4,5-dihydro-*1H*-imidazole, fumarate**

Point de fusion : 204°C

| Microanalyse élémentaire : | | | |
|---|---|---|---|
| | C% | H% | N% |
| Théorique | 70,61 | 6,96 | 9,47 |
| Trouvée | 70,74 | 7,01 | 9,47 |

**EXEMPLE 29 : 4-{Spiro[cyclopropan-2:2'-(1',2',3',4'-tétrahydro naphtalèn)]-1-yl)-4,5-dihydro-*1H*-imidazole, fumarate (isomère 1)**

*Stade 1 : Diéthyl(1-oxo-1,2,3,4-tétrahydro-2-naphtalényl)phosphonate*

Une solution d'a-tétralone (10 g, 68 mmol) dans du THF anhydre (120 ml) est ajoutée goutte à goutte à une solution de diisopropylamidure de Lithium 1M (75 mmol, 75 ml) agitée à -65°C sous azote. Après 45 minutes d'agitation, on ajoute à l'énolate formé du chlorophosphate de diéthyle (12,90 g, 75 mmol) et le milieu est remonté progressivement à 0°C sur une période de 50 minutes. Après refroidissement à -70°C, le milieu est transféré sur une solution de diisopropylamidure de Lithium 2M (150 mmol, 75 ml). La solution obtenue est agitée 2 heures à 10°C puis traitée avec de l'acide acétique (272 mmol, 15,5 ml) dans de l'éther (250 ml). Le milieu est filtré et le filtrat concentré. Le composé du titre est obtenu par purification sur colonne flash (éluant : cyclohexane/EtOAc = 60/40).

*Stade 2 : 2'-(Tétrahydropyran-2-yloxyméthyl)-spiro[1'-2]cyclopropan-3,4-dihydro-1-oxo-naphtalène*

Une solution du composé obtenu au stade 1 (27 g, 95 mmol) dans du toluène (60 ml) est additionnée à une suspension de NaH (4,40 g, 109,2 mmol) dans du toluène (160 ml) à 20°C sous azote. Le milieu est agité à température ambiante pendant 1 heure puis 30 g (190 mmol) de 2-oxiranylméthoxy-tétrahydropyrane sont ajoutés. La réaction est portée à reflux durant 4 jours puis le milieu est refroidi, hydrolysé à l'eau et extrait à l'éther. La phase organique est lavée avec une solution de NaCl, séchée sur $MgSO_4$ et concentrée sous vide. Le résidu obtenu est chromatographié sur colonne flash (éluant : cyclohexane/EtOAc = 80/20) et permet d'isoler le composé du titre sous forme d'une huile rouge pâle.

| Microanalyse élémentaire : | | |
|---|---|---|
| | C% | H% |
| Théorique | 75,50 | 7,74 |
| Trouvée | 75,43 | 7,82 |

*Stade 3 : {Spiro[cyclopropan-2:2'-(1',2',3',4'-tétrahydronaphtalèn)]-1-yl}-méthanol*

A une solution du composé obtenu au stade 2 (2,50 g, 8,7 mmol) dans du 1,2-dichloroéthane (45 ml) à température ambiante sous azote sont additionnés du iodure de zinc solide (4,16 g, 13 mmol) et du cyanoborohydrure de sodium (4,10 g, 62,2 mmol). Le milieu réactionnel est chauffé à 80-85°C pendant 3 heures puis refroidi et versé sur une solution de $NH_4Cl$ saturée glacée contenant 10% en volume de HCl 5N (180 ml). Le milieu est extrait 3 fois à l'AcOEt et les phases organiques sont séchées sur $MgSO_4$ et évaporées. L'alcool du titre est purifié par chromatographie sur colonne flash (éluant: cyclohexane/EtOAc = 70/30) sous forme d'huile incolore.

*Stade 4 : {Spiro[cyclopropan-2:2'-(1',2',3',4'-tétrahydronaphtalèn)]-1-yl)- carboxaldéhyde*

A une solution de pyridine (226,5 mmol, 18,5 ml) dans du $CH_2Cl_2$ (360 ml) à 0°C sous azote sont additionnés 11,6 g (116,2 mmol) de $CrO_3$. Après 1 heure d'agitation à température ambiante, l'alcool obtenu au stade 3 (3,64 g, 19,4 mmol) en solution dans du $CH_2Cl_2$ (80 ml) est additionné et le milieu est agité encore 2 heures à température

ambiante, puis filtré et le filtrat évaporé sous pression réduite. Le résidu est dilué avec de l'éther, lavé avec NaOH 1N, HCl 1N et une solution saturée de NaCl puis séché sur $MgSO_4$ et évaporé sous pression réduite pour conduire à l'aldéhyde du titre sous forme d'huile.

*Stade 5 : {Spiro[cyclopropan-2:2'-(1',2',3',4'-tétrahydronaphtalèn)]-1-yl}-aminoacétonitrile*

A une solution agitée vigoureusement sous azote contenant 2,63 g (14,1 mmol) du composé obtenu au stade 4, 430 ml de MeOH et 20 ml d'eau, sont successivement additionnés 760 mg (14,2 mmol) de KCN et 920 mg (14,2 mmol) de $NH_4Cl$. Après agitation 12 heures à 20°C, la solution est diluée dans du $CH_2Cl_2$ et extraite 3 fois au $CH_2Cl_2$. La phase organique est lavée avec une solution saturée de NaCl, séchée sur $MgSO_4$ et concentrée. Le résidu obtenu est traité par 30 ml d'une solution méthanolique d'ammoniaque 7N et agité en système clos 12 heures à 20°C. Après évaporation sous pression réduite, le produit du titre est obtenu sous forme d'une huile brune.

*Stade 6 : 1{Spiro[cyclopropan-2:2'-(1',2',3',4'-tétrahydronaphtalèn)-1-yl}-éthane-1,2-diamine*

Une solution du composé obtenu au stade 5 (2,90 g, 13,7 mmol) dans du THF anhydre (60 ml) est additionnée goutte à goutte à une suspension de $LiAlH_4$ (570 mg, 15 mmol) dans 60 ml de THF à -20°C sous azote. Le milieu est agité 2 heures à -10°C puis hydrolysé par additions successives de $H_2O$ (3,5 ml), NaOH 35% (7 ml), $H_2O$ (7,3 ml). La suspension obtenue est filtrée et le filtrat évaporé. L'huile obtenue est purifiée par chromatographie sur colonne flash et conduit à un mélange de 2 diastéréoisomères séparés par HPLC (Kromasil 100,5 C18-265mm-$CH_3OH/H_2O/CF_3COOH$: 350/650/5).

*Stade 7: 4-{Spiro[cyclopropan-2:2'-(1',2',3',4'-tétrahydro naphtalèn)]-1-yl}-4,5-dihydro-*1H*-imidazole, fumarate (isomère 1)*

Un mélange contenant l'un des deux diastéréoisomères obtenus au stade 6 (170 mg, 0,8 mmol) et de l'acétate de formamidine (94 mg, 0,9 mmol) dans de l'éthanol (5 ml) est agité à 20°C sous azote pendant 12 heures. Le solvant est ensuite évaporé et le résidu repris dans HCl 1N. La phase acide est lavée avec $Et_2O$ et basifiée avec une solution de soude. Le milieu est extrait avec $CH_2Cl_2$ et la phase organique est lavée, séchée sur $MgSO_4$ et évaporée. Le résidu solide obtenu est dissout dans de l'acétone (10 ml) et traité par une solution d'acide fumarique (81,5 mg, 0,7 mmol) dans de l'isopropanol (4 ml). Après évaporation et recristallisation dans un mélange acétone/isopropanol, le composé du titre est obtenu sous forme d'une poudre blanche.
Point de fusion : 149°C

**EXEMPLE 30 : 4-{Spiro[cyclopropan-2:2'-(1',2',3',4'-tétrahydro naphtalèn)]-1-yl}-4,5-dihydro-*1H*-imidazole, fumarate (isomère 2)**

On procède comme dans le stade 7 de l'Exemple 29 à partir de l'autre diastéréoisomère isolé au stade 6 de l'Exemple 29.
Point de fusion : 164°C

| Microanalyse élémentaire : | | | |
|---|---|---|---|
| | C% | H% | N% |
| Théorique | 66,65 | 6,48 | 8,18 |
| Trouvée | 66,59 | 6,65 | 8,22 |

**EXEMPLE 31 : 4-[(8-Chloro-1,2,3,4-tétrahydro-2-naphtalényl)méthyl]-4,5-dihydro-*1H*-imidazole, fumarate**

On procède comme dans l'Exemple 16 à partir du 2-(8-chloro-3,4-dihydro-2-naphtalényl)acétate d'éthyle.
Point de fusion : 230-233°C

| Microanalyse élémentaire : | | | | |
|---|---|---|---|---|
| | C% | H% | N% | %Cl |
| Théorique | 59,26 | 5,80 | 7,68 | 9,72 |
| Trouvée | 59,44 | 5,95 | 7,64 | 9,93 |

**EXEMPLE 32: 4-[(5-Fluoro-2,3-dihydro-*1H*-indèn-2-yl)méthyl]-4,5-dihydro-*1H*-imidazole, hémifumarate**

On procède comme dans l'Exemple 23 à partir du 2-hydroxyméthyl-5-fluoroindane.

Point de fusion : 187-190°C

| Microanalyse élémentaire : | | | |
|---|---|---|---|
| | C% | H% | N% |
| Théorique | 65,20 | 6,20 | 10,14 |
| Trouvée | 64,82 | 6,20 | 9,90 |

**EXEMPLE 33: 4-[(4-Fluoro-2,3-dihydro-*1H*-indèn-2-yl)méthyl]-4,5-dihydro-*1H*-imidazole, hémifumarate**

On procède comme dans l'Exemple 23 à partir du 2-hydroxyméthyl-4-fluoroindane.

Point de fusion : 212-215°C

| Microanalyse élémentaire : | | | |
|---|---|---|---|
| | C% | H% | N% |
| Théorique | 65,20 | 6,20 | 10,14 |
| Trouvée | 64,34 | 6,32 | 9,89 |

**EXEMPLE 34: 4-[(5,6-Difluoro-2,3-dihydro-*1H*-indèn-2-yl)méthyl]-4,5-dihydro-*1H*-imidazole, hémifumarate**

On procède comme dans l'Exemple 23 à partir du 2-hydroxyméthyl-5,6-difluoroindane.

Point de fusion : 201-202°C

| Microanalyse élémentaire: | | | |
|---|---|---|---|
| | C% | H% | N% |
| Théorique | 61,22 | 5,48 | 9,52 |
| Trouvée | 61,04 | 5,54 | 9,47 |

**EXEMPLE 35 : 4-[(5,6-Diméthyl-2,3-dihydro-*1H*-indèn-2-yl)méthyl]-4,5-dihydro-1*H*-imidazole, fumarate**

On procède comme dans l'Exemple 23 à partir du 2-hydroxyméthyl-5,6-diméthylindane.

Point de fusion : 198-200°C

| Microanalyse élémentaire : | | | |
|---|---|---|---|
| | C% | H% | N% |
| Théorique | 66,26 | 7,02 | 8,13 |
| Trouvée | 66,37 | 7,07 | 8,09 |

**EXEMPLE 36: 4-[(5-Fluoro-1-benzofuran-2-yl)méthyl]-4,5-dihydro-*1H*-imidazole, fumarate**

On procède comme dans l'Exemple 23 à partir du 2-hydroxyméthyl-5-fluorobenzo[*b*]furane.

Point de fusion : 148-151°C

| Microanaivse élémentaire : | | | |
|---|---|---|---|
| | C% | H% | N% |
| Théorique | 57,49 | 4,52 | 8,38 |
| Trouvée | 57,51 | 4,68 | 8,27 |

**EXEMPLE 37 : 4-(3,4-Dihydro-2-naphtalénylméthy)-1-méthyl-4,5-dihydro-*1H*-imidazole, 1,5 fumarate**

A une solution de 320 mg (1,5 mmol) du composé obtenu dans l'Exemple 1 (après neutralisation du sel par NaOH 2N) dans 10 ml de $CH_2Cl_2$, refroidie à -60°C, sont ajoutés successivement 0,205 ml (1,5 mmol) de triéthylamine puis 0,165 ml (1,75 mmol) de diméthylsulfate. La température est ensuite remontée à 20°C et le milieu agité à cette température pendant 12 heures. Le mélange est lavé parNaOH 0,1N (5 ml) puis par une solution saturée de NaCl. Après séchage et évaporation du solvant, le résidu est chromatographié sur silice en éluant avec un mélange $CH_2Cl_2$/$CH_3OH$/$NEt_3$ = 90/10/0,5. La fraction de tête est dissoute dans 5 ml d'EtOH puis traitée par une solution de 71 mg (0,61 mmol) d'acide fumarique dans 5 ml d'EtOH. Après concentration, le composé du titre est isolé par recristallisation.

Point de fusion : 190-192°C

| Microanalyse élémentaire : | | | |
|---|---|---|---|
| | C% | H% | N% |
| Théorique | 62,99 | 6,05 | 7,00 |
| Trouvée | 63,22 | 6,10 | 6,97 |

**EXEMPLE 38 : 4-[(7-Fluoro-1,2,3,4-tétrahydro-2-naphtalényl)méthyl]-4,5-dihydro-*1H*-imidazole, fumarate (diastéréoisomère 1)**

*Stade 1 : N-{2-(1-Cyclohexen-1-ylamino)-1-[(7-fluoro-3,4-dihydro-2-naphtalényl)méthyl]-2-oxoéthyl}-N-[(1R)-1-phényléthyl]benzamide*

Une solution de 15 g (78 mmoles) de 7-fluoro-3,4-dihydro-2-naphtalène carboxaldéhyde (obtenu selon le même procédé que dans les stades 1 et 2 de l'Exemple 1 à partir de la 7-fluoro-1,2,3,4-tétrahydronaphtalèn-2-one) et 9,45 g (78 mmol) de (1R)-1-phényléthylamine dans 150 ml de méthanol est agitée à 20°C sous atmosphère d'azote pendant 1 heure. Puis sont additionnés successivement 9,76 g (80 mmol) d'acide benzoïque et 8,56 g (80 mmol) de 1-cyclohexényl-isonitrile ; le mélange est alors agité à 20°C pendant 15 heures. Après concentration sous vide, le résidu est repris par EtOAc (300 ml) ; la solution est lavée par NaOH aq. 0,1 N puis HCl aq. 0,1 N et enfin NaCl aq. saturée. La phase organique est concentrée sous vide ; une chromatographie du résidu huileux, en éluant avec un mélange cyclohexane/EtOAc = 80/20 permet d'isoler 2 diastéréoisomères (1 et 2) du composé du titre, sous forme d'huiles.
Rf (1) = 0,25    Rf (2) =0,15

*Stade 2 : N-{2-Amino-1-[(7-fluoro-3,4-dihydro-2-naphtalényl)méthyl]-2-oxoéthyl)-N-[(1R)-1-phényléthyl]benzamide*

Une solution de 3,50 g (6,70 mmoles) du diastéréoisomère 2 du stade 1 dans 50 ml de THF est traitée par 1,20 ml de HCl concentré. Après agitation pendant 1 heure à 20°C, le mélange est neutralisé par addition de $NaHCO_3$ solide, filtré et concentré sous vide. Une chromatographie sur $SiO_2$, en éluant ave un mélange cyclohexane/EtOAc = 40/60 permet d'obtenir le composé du titre sous forme de mousse.

| Microanalyse élémentaire : | | | |
|---|---|---|---|
| | C% | H% | N% |
| Théorique | 76,00 | 6,15 | 6,33 |
| Trouvée | 76,20 | 6,30 | 5,95 |

*Stade 3 : N-{2-Amino-1-[(7-fluoro-1,2,3,4-tétrahydro-2-naphtalényl )méthyl]-2-oxoéthyl}-N-[(1R)-1-phényléthyl]benzamide*

Une suspension de 2,70 g (6,10 mmol) du composé obtenu au stade 2 et de 1 g de $Pd(OH)_2$ dans 120 ml d'EtOH est hydrogénée sous une atmosphère pendant 12 heures. Après filtration du catalyseur, le solvant est évaporé sous vide. Le résidu obtenu est chromatographié sur $SiO_2$ en éluant avec une solution cyclohexane/EtOAc = 40/60, pour donner le composé du titre sous forme de 2 diastéréoisomères.

*Diastéréoisomère 1 :*

Point de fusion: 145-146°C

| Microanalyse élémentaire : | | | |
|---|---|---|---|
| | C% | H% | N% |
| Théorique | 75,65 | 6,58 | 6,30 |
| Trouvée | 75,46 | 6,62 | 6,48 |

*Stade 4 : N-{2-Amino-1-[(7-fluoro-1,2,3,4-tétrahydro-2-naphtalényl)méthyl] éthyl)-N-benzyl-N-[(1R)-1-phényléthyl]amine*

A une solution maintenue sous azote de 1,20 g (2,70 mmol) du composé obtenu au stade 3 (diastéréoisomère 1) dans 30 ml de THF anhydre sont ajoutés goutte à goutte sous forte agitation, 7 ml (7 mmol) d'une solution molaire de $BH_3$/THF. Le milieu est ensuite chauffé à 70°C pendant 1 heure, puis refroidi, traité par 8 ml de $CH_3OH$ et agité à 20°C pendant 1 heure. Après concentration sous vide, le résidu est chromatographié sur silice en éluant avec une solution $CH_2Cl_2/CH_3OH$ = 95/5 pour conduire au composé du titre sous forme d'huile.

*Stade 5 : 3-(7-Fluoro-1,2,3,4-tétrahydro-2-naphtalényl)-1,2-propanediamine*

A une suspension fortement agitée de 0,35 g (0,84 mmol) du composé obtenu au stade 4 et de 0,25 g de Pd/C à 10 % dans 25 ml de $CH_3OH$ sont ajoutés 0,45 g de formiate d'ammonium. Après 1 heure d'agitation sous reflux, le mélange est refroidi et filtré ; le catalyseur est lavé par $CH_3OH$ et les filtrats réunis sont concentrés sous vide pour conduire au composé du titre, sous forme d'huile, utilisée sans purification dans la réaction suivante.

*Stade 6 : 4-[(7-Fluoro-1,2,3,4-tétrahydro-2-naphtalényl)méthyl]-4,5-dihydro-*1H*-imidazole, fumarate (diastéréoisomère 1)*

On procède comme dans le stade 5 de l'Exemple 1 à partir du composé obtenu au stade 5 ci-dessus.
Point de fusion: 174-175°C

| Microanalyse élémentaire : | | | |
|---|---|---|---|
| | C% | H% | N% |
| Théorique | 62,06 | 6,08 | 8,04 |
| Trouvée | 62,25 | 6,39 | 8,08 |

**EXEMPLE 39 : 4-[(7-Fluoro-1,2,3,4-tétrahydro-2-naphtalényl)méthyl]-4,5-dihydro-1H-imidazole, fumarate (diastéréoisomère 2)**

On procède comme dans l'Exemple 38 en prenant au stade 3 le diastéréoisomère 2 obtenu.

[Diastéréoisomère 2 : Mousse

Microanalyse élémentaire :

| | C% | H% | N% |
|---|---|---|---|
| Théorique : | 75,65 | 6,58 | 6,30 |
| Trouvée : | 75,38 | 6,66 | 6,40 ] |

Point de fusion : 170-172°C

**EXEMPLE 40 : 4-[(7-Fluoro-1,2,3,4-tétrahydro-2-naphtalényl)méthyl]-4,5-dihydro- *1H*-imidazole, fumarate (diastéréoisomères 3 et 4 en mélange)**

On procède comme dans l'Exemple 38 en prenant au stade 1 le diastéréoisomère 1 obtenu.

Point de fusion : 170-171°C

| Microanalyse élémentaire : | | | |
|---|---|---|---|
| | C% | H% | N% |
| Théorique | 62,06 | 6,08 | 8,04 |
| Trouvée | 62,31 | 6,14 | 8,07 |

**EXEMPLE 41: 4-[(8-Chloro-2-naphtyl)méthyl]-4,5-dihydro-*1H*-imidazole, fumarate**

On procède comme dans les stades 2, 3 et 4 de l'Exemple 23 à partir du 2-bromométhyl-8-chloronaphtalène.

Point de fusion : 172-175°C

| Microanalyse élémentaire: | | | | |
|---|---|---|---|---|
| | C% | H% | N% | Cl% |
| Théorique | 59,92 | 4,75 | 7,76 | 9,83 |
| Trouvée | 59,55 | 4,79 | 7,58 | 10,00 |

**EXEMPLE 42: 4-[(7-Fluoro-2-naphtyl)méthyl]-4,5-dihydro-*1H*-imidazole, fumarate**

On procède comme dans les stades 2, 3 et 4 de l'Exemple 23 à partir du 2-bromométhyl-7-fluoronaphtalène.

Point de fusion : 157-160°C

| Microanalyse élémentaire: | | | |
|---|---|---|---|
| | C% | H% | N% |
| Théorique | 62,79 | 4,98 | 8,14 |
| Trouvée | 62,41 | 5,08 | 8,11 |

**EXEMPLE 43 : 4-Méthyl-4-(1,2,3,4-tetrahydro-2-naphtalénylméthyl)-4,5-dihydro-*1H*-imidazole, fumarate (diastéréoisomère 1)**

*Stade 1 : 3-(3,4-Dihydro-2-naphtalényl)-2-méthyl-2-[(1-phényléthyl)amino] propane nitrile*

Une solution de 9,50 g (51 mmol) de (3,4-dihydro-naphtalèn-2-yl)-propan-2-one, 6,17 g (51 mmol) de R-α-méthyl-benzylamine et 0,095 g (0,5 mmol) d'acide p-toluène sulfonique dans 250 ml de toluène est chauffée au reflux avec distillation aréotropique du mélange eau/toluène pendant 8 heures. Après refroidissement et évaporation du solvant, le résidu est dissout dans 450 ml de $CH_2Cl_2$ ; à ce mélange refroidi à -35°C, sous atmosphère d'azote sont additionnés par portions 5,50 g (55 mmol) de cyanure de triméthylsilyle. Après 10 heures d'agitation à -35°C, le mélange est concentré sous vide pour donner le composé du titre, sous forme d'huile, utilisée sans purification pour la réaction suivante.

*Stade 2 : N-[2-Amino-1-(3,4-dihydro-2-naphtalénylméthyl)-1-méthyléthyl]-N-(1-phényl-éthyl)amine*

Une solution de 16 g du composé obtenu au stade 1 dans 200 ml de THF est additionnée goutte à goutte à une suspension maintenue sous azote de 2,28 g (60 mmol) de $LiAlH_4$ dans 200 ml de THF. La température du milieu est ensuite remontée à 20°C et l'agitation poursuivie à cette température pendant 3 heures. Le milieu est à nouveau refroidi à 0°C et hydrolysé par additions successives de 20 ml d'iPrOH et 25 ml d'une solution saturée de NaCl. Le précipité formé est essoré et rincé par du THF ; le filtrat est concentré sous vide et le résidu est chromatographié sur silice en éluant avec un mélange $CH_2Cl_2/CH_3OH/NH_4OH$ = 97/3/0,3 pour donner le composé du titre sous forme de 2 diastéréoisomères :

- diastéréoisomère 1 : huile - Rf= 0,18
- diastéréoisomère 2 : huile - Rf= 0,15

<u>Stade 3</u>: *N-[2-Amino-1-méthyl-1-(1,2,3,4-tétrahydro-2-naphtalénylméthyl)éthyl] -N-(1-phényléthyl)amine*

Une suspension de 0,52 g (1,62 mmol) du composé obtenu au stade 2 (diastéréoisomère 1) et de 0,1 g de $Pd/Al_2O_3$ dans 60 ml d'EtOH est hydrogénée à 20°C sous 1 bar pendant 16 heures. Le mélange est ensuite filtré et concentré sous vide pour conduire au produit du titre sous forme de mousse.

<u>Stade 4</u> : *2-Méthyl-3-(1,2,3,4-tétrahydro-2-naphtalényl)-1,2-propanediamine*

Une suspension de 0,50 g (1,55 mmol) du composé obtenu au stade 3 et 0,14 g de $Pd(OH)_2$ dans un mélange de 0,8 ml d'acide acétique et 50 ml d'EtOH est hydrogénée à 20°C, sous 1 bar pendant 20 heures. Après filtration du mélange et concentration sous vide, le résidu est repris dans NaOH aq. 1N (10 ml), extrait par $CH_2Cl_2$ (3 x 10 ml) ; les phases organiques réunies sont lavées par NaCl aq. saturée, séchées et concentrées pour conduire au composé du titre sous forme d'huile utilisée sans autre purification dans la réaction suivante.

<u>Stade 5</u> : *4-Méthyl-4-(1,2,3,4-tétrahydro-2-naphtalénylméthyl)-4,5-dihydro-*1H-*imidazole, fumarate (diastéréoisomère 1)*

On procède comme au stade 5 de l'Exemple 1 à partir du composé obtenu au stade 4.
<u>Point de fusion</u> : 118°C

| Microanalyse élémentaire : | | | |
|---|---|---|---|
| | C% | H% | N% |
| Théorique | 66,26 | 7,02 | 8,13 |
| Trouvée | 65,98 | 7,01 | 8,22 |

**<u>EXEMPLE 44</u>: 4-Méthyl-4-(1,2,3,4-tétrahydro-2-naphtalénylméthyl)-4,5-dihydro-*1H*-imidazole, fumarate (diastéréoisomère 2)**

On procède comme dans l'Exemple 43 en prenant au stade 3 le diastéréoisomère 2 obtenu au stade 2.
<u>Point de fusion</u>: 121°C

| Microanalyse élémentaire : | | | |
|---|---|---|---|
| | C% | H% | N% |
| Théorique | 66,26 | 7,02 | 8,13 |
| Trouvée | 66,11 | 7,11 | 8,16 |

**<u>EXEMPLE 45</u>: 4-{Spiro[cyclopropan-2':2''-(5'',6''-difluoro-indan)]-1'-yl}-4,5-dihydro-1*H*-imidazole, fumarate (diastéréoisomère 1)**

<u>Stade 1</u>: *Ester éthylique de l'acide {spiro[cyclopropan-2:2'-(5',6'-difluoro-1'-indanone)]-1-yl}-carboxylique*

A une solution de 5,6-difluoro-1-indanone (20 g, 120 mmole) et de 2-[(diéthoxy-phosphoryl)oxy]acrylate d'éthyle (36 g, 140 mmole) dans le THF (260 ml), est ajouté par portions NaH (3,43 g, 140 mmole) de manière telle que la température ne dépasse pas 35°C. A la fin de l'addition, le ballon est plongé dans un bain d'huile à 50°C et la température de la réaction monte à 60°C. Lorsque la température revient à 45°C, on agite une heure à cette température. Le mélange réactionnel est versé sur un mélange de glace (1 l) et d'acide chlorhydrique 1N (1 l), le produit est extrait à l'acétate d'éthyle (3 × 600 ml), la solution organique est séchée sur $MgSO_4$ et évaporée. Le produit du titre est purifié par chromatographie sur gel de silice (éluant : cyclohexane/acétate d'éthyle 93/7).

<u>Stade 2</u> : *Ester éthylique de l'acide {spiro[cyclopropan-2:2'-(5',6'-difluoroindan)]-1-yl}-carboxylique*

Le produit obtenu au stade 1 (24,2 g, 90 mmole) est introduit dans un ballon de 1 l muni d'un agitateur mécanique, qui contient 500 ml de 1,2-dichloroéthane, $NaBH_3CN$ (42,7 g, 680 mmole) et $ZnI_2$ (41,5 g, 130 mmole). La suspension est portée au reflux sous agitation, pendant 14 heures. Les sels sont ensuite filtrés, la solution est hydrolysée par un mélange $NH_4Cl$ 10 % (1 l) et HCl 6N (180 ml), la phase organique est décantée après une demi-heure et la phase aqueuse est extraite avec de l'acétate d'éthyle (2 × 500 ml). Les sels sont hydrolysés dans la même phase aqueuse et celle-ci est extraite une nouvelle fois à l'acétate d'éthyle (2 × 300 ml). Les phases organiques réunies sont lavées avec $Na_2CO_3$ (500 ml) et NaCl (500 ml), séchées sur $MgSO_4$ et concentrées sous vide. Le produit du titre est purifié par chromatographie sur gel de silice (éluant : cyclohexane/acétate d'éthyle 95/5).

*Stade 3 : (Spiro[cyclopropan-2:2'-(5',6'-difluoro-indan)]-1-yl}-carbinol*

Le produit obtenu au stade 2 (13,9 g, 53 mmole) en solution dans le THF (250 ml) est ajouté goutte à goutte à une suspension de $LiAlH_4$ (3,1 g, 82 mmole) dans le THF (250 ml) à -18°C. Le mélange est agité 3 heures à température ambiante et décomposé par l'addition successive d'eau (3,1 ml), de NaOH 1N (3,1 ml) et d'eau (6,2 ml). La suspension est agitée une nuit, le solide est filtré et le filtrat est évaporé pour conduire au produit du titre, qui est utilisé sans purification supplémentaire dans l'étape suivante.

*Stade 4 : {Spiro[cyclopropan-2:2'-(5',6'-difluoro-indan)]-1-yl}-carboxaldéhyde*

Le produit obtenu au stade 3 (14,3 g, 68 mmole) en solution dans 140 ml de $CH_2Cl_2$ est ajouté à température ambiante, goutte à goutte à une suspension de chromate de pyridinium préparé à 0°C, à partir de pyridine (68 ml, 670 mmole) et de $CrO_3$ (42 g, 420 mmole) dans 1 l de $CH_2Cl_2$. La suspension est agitée pendant 5 heures, filtrée, le précipité lavé à l'éther, les filtrats évaporés à sec, repris dans l'éther (1 l) et les insolubles filtrés. La phase organique est lavée successivement par NaOH 1N (1 l), HCl 1N (2 × 750 ml), $NaHCO_3$ 10 % (2 × 500 ml), NaCl (500 ml), séchée sur $MgSO_4$ et évaporée pour conduire au produit du titre qui est utilisé sans étape de purification supplémentaire dans l'étape suivante.

*Stade 5 :2-Di(4-méthoxyphényl)-méthylamino-2-{spiro[cyclopropan-2':2"-(5",6 "-difluoro-indan]-1'-yl}-acétonitrile*

Le produit obtenu au stade 4 (12 g, 58 mmole) est agité dans 250 ml de $CH_2Cl_2$ pendant 2 heures en présence de di(4-méthoxyphényl)méthylamine (14 g, 58 mmole) et de tamis moléculaire 4Å (18 g). Puis 6,3 g de $(CH_3)_3SiCN$ (64 mmole) sont alors ajoutés et la solution est agitée 14 heures à température ambiante. Le solide est filtré, la phase organique est lavée par NaOH 0,1 N (500 ml) et NaCl (250 ml), séchée sur $MgSO_4$ et évaporée pour conduire au produit du titre qui est utilisé sans purification supplémentaire dans l'étape suivante.

*Stade 6 : 2-Di{4-méthoxyphényl)-méthylamino-2-{spiro[cyclopropan-2':2"-(5",6"-difluoro-indan)]-1'-yl}-éthylamine (diastéréoisomère 1)*

Le produit obtenu au stade 5 (26,7 g, 68 mmole) en solution dans le THF (125 ml) est ajouté à -10°C, goutte à goutte à une suspension de $LiAlH_4$ (3,3 g, 87 mmole) dans le THF (500 ml). A la fin de l'addition, la température remonte et la suspension est agitée 1 heure avant d'être décomposée par l'addition successive d'eau (3,3 ml), de NaOH 1N (3,3 ml) et d'eau (6,6 ml). Après addition d'éther (300 ml), la suspension est agitée pendant 45 minutes, le solide est filtré et le filtrat évaporé. Les diastéréoisomères sont séparés par chromatographie sur gel de silice en éluant avec un gradient d'ammoniaque (10 % dans l'éthanol absolu) dans $CH_2Cl_2$.

*Stade 7 : 2 Amino-2-{spiro[cyclopropan-2':2"-(5",6"-difluoro-indan)]-1'-yl}-éthylamine (diastéréoisomère 1)*

Le produit obtenu au stade 6 (diastéréoisomère 1) (3,12 g, 6,7 mmole) est dissout dans un mélange AcOH/$H_2O$ (80/20, 200 ml) et la solution est plongée dans un bain d'huile à 90°C pendant 45 minutes. L'acide acétique est ensuite évaporé. Le sirop obtenu est dissout dans HCl 1N (100 ml) et la solution est lavée à l'éther (3 × 75 ml). La phase aqueuse est neutralisée par NaOH 35 % (20 ml), extraite au $CH_2Cl_2$ (3 × 75 ml), sechée sur $K_2CO_3$ et évaporée. Le produit du titre est purifié par chromatographie sur gel de silice (éluant : $CH_2Cl_2$/MeOH/$NH_4OH$ 90/9/1).

*Stade 8 : 4-{Spiro[cyclopropan-2':2"-(5",6"-difluoro-indan)]-1'-yl}-4,5-dihydro-1H-imidazole, fumarate (diastéréoisomère 1)*

A une solution du composé obtenu au stade 7 (1,5 g, 6,3 mmole) dans l'EtOH (100 ml), est additionné l'acétate de formamidine (654 mg, 6,3 mmole). Après 14 heures d'agitation à température ambiante, l'éthanol est évaporé et le solide blanc est repris dans l'acétone (75 ml) et l'isopropanol (10 ml). La solution est filtrée et additionnée d'acide

fumarique (694 mg, 6,3 mmole) dissout au préalable en tiédissant dans l'acétone (25 ml) et l'isopropanol (10 ml). Le composé du titre est obtenu sous la forme d'un solide qui est filtré et séché sous vide.

Point de fusion : 220°C (décomposition)

| Microanalyse élémentaire : | | | |
|---|---|---|---|
| | C% | H% | N% |
| Théorique | 59,34 | 4,98 | 7,69 |
| Trouvée | 59,43 | 5,17 | 7,58 |

**EXEMPLE 46 : 4-(Spiro[cyclopropan-2':2"-(5",6"-difluoro-indan)]-1'-yl}-4,5-dihydro-*1H*-imidazole, fumarate (diastéréoisomère 2)**

On procède comme aux stades 7 et 8 de l'Exemple 45 en partant du deuxième diastéréoisomère isolé au stade 6.

Point de fusion : 240°C (décomposition)

| Microanalyse élémentaire : | | | |
|---|---|---|---|
| | C% | H% | N% |
| Théorique | 59,34 | 4,98 | 7,69 |
| Trouvée | 59,46 | 4,98 | 7,59 |

**EXEMPLE 47 : 4-[Spiro (cyclopropan-2':2"-indan)-1'-yl]-4,5-dihydro-*1H*- imidazole, hemifumarate (diastéréoisomère 1)**

On procède comme aux stades 1 à 8 de l'Exemple 45 en partant de la 1-indanone.

Point de fusion : 212°C

| Microanalyse élémentaire : | | | |
|---|---|---|---|
| | C% | H% | N% |
| Théorique | 71,09 | 6,71 | 10,36 |
| Trouvée | 70,70 | 6,85 | 10,32 |

**EXEMPLE 48 : 4-[Spiro(cyclopropan-2':2"-indan)-1'-yl]-4,5-dihydro-*1H*- imidazole, fumarate (diastéréoisomère 2)**

On procède comme aux stades 1 à 8 de l'Exemple 46 en partant de la 1-indanone.

Point de fusion : 212°C

| Microanalyse élémentaire : | | | |
|---|---|---|---|
| | C% | H% | N% |
| Théorique | 65,84 | 6,14 | 8,53 |
| Trouvée | 65,86 | 6,40 | 8,66 |

**EXEMPLE 49 : 4-{Spiro[cyclopropan-2':2"-(8"-chlore-1",2",3",4"-tetrahydronaphtalene)]-1'-yl}-4,5-dihydro-*1H*-imidazole, fumarate (diastéréoisomère 1)**

On procède comme aux stades 1 à 8 de l'Exemple 45 en partant de la 8-chloro-1-tetralone.

Point de fusion : 175°C

| Microanalyse élémentaire: | | | |
|---|---|---|---|
| | C% | H% | N% |
| Théorique | 60,56 | 5,62 | 7,43 |
| Trouvée | 60,16 | 5,73 | 7,26 |

**EXEMPLE 50: 4-{Spiro[cyclopropan-2':2"-(8"-chloro-1",2",3",4"-tetrahydro- naphtalène)]-1'-yl}-4,5-dihydro-*1H*-imidazole, fumarate (diastéréoisomère 2)**

On procède comme aux stades 1 à 8 de l'Exemple 46 en partant de la 8-chloro-1-tetralone.

Point de fusion : 194°C

| Microanalyse élémentaire : | | | | |
|---|---|---|---|---|
| | C% | H% | N% | Cl% |
| Théorique | 60,56 | 5,62 | 7,43 | 9,41 |
| Trouvée | 60,24 | 5,61 | 7,48 | 9,41 |

**EXEMPLE 51 : 4-{Spiro[cyclopropan-2':2"-(1",2",3",4"-tetrahydro-naphtalène)]-1'-yl}-4,5-dihydro-*1H*-imidazole, fumarate (énantiomère 1 du produit de l'Exemple 30)**

Le deuxième diastéréoisomère du stade 6 de l'Exemple 29 est dissout dans l'éthanol en présence d'un équivalent d'acide R(-) mandélique. Le solide qui cristallise est recristallisé dans l'éthanol. Le sel est décomposé par neutralisation d'une solution aqueuse par NaOH 1N, puis la solution est extraite au dichlorométhane, séchée sur $K_2CO_3$ et évaporée sous vide. Le résidu est traité comme au stade 7 de l'Exemple 29 pour donner le produit du titre.

Point de fusion : 173,6°C

| Microanalyse élémentaire : | | | |
|---|---|---|---|
| | C% | H% | N% |
| Théorique | 66,65 | 6,48 | 8,18 |
| Trouvée | 66,35 | 6,48 | 8,14 |

**EXEMPLE 52: 4-{Spiro[cyclopropan-2':2"-(1",2",3",4"-tetrahydro-naphtalène)]-1'-yl}-4,5-dihydro-*1H*-imidazole, fumarate (énantiomère 2 du produit de l'exempte 30)**

Le deuxième diastéréoisomère du stade 6 de l'Exemple 29 est dissout dans l'éthanol en présence d'un équivalent d'acide S(+) mandélique. Le solide qui cristallise est recristallisé dans l'éthanol. Le sel est décomposé par neutralisation d'une solution aqueuse par le NaOH 1N, puis la solution est extraite au dichlorométhane, séchée sur $K_2CO_3$ et évaporée sous vide. Le résidu est traité comme au stade 7 de l'Exemple 29 pour donner le produit du titre.

Point de fusion : 172,5°C

| Microanalyse élémentaire: | | | |
|---|---|---|---|
| | C% | H% | N% |
| Théorique | 66,65 | 6,48 | 8,18 |
| Trouvée | 66,34 | 6,50 | 8,16 |

**EXEMPLE 53: 4-[(2-Méthyl-1,2,3,4-tétrahydro-2-naphtalényl)méthyl]-4,5-dihydro-*1H*-imidazole, fumarate (diastéréoisomère 1)**

On procède comme aux stades 5 à 8 de l'Exemple 45 à partir du (2-méthyl-1,2,3,4-tétrahydronaphtalèn-2-yl) acétaldéhyde.

Point de fusion : 154°C

| Microanalyse élémentaire : | | | |
|---|---|---|---|
| | C% | H% | N% |
| Théorique | 66,26 | 7,02 | 8,13 |
| Trouvée | 65,72 | 7,17 | 8,28 |

**EXEMPLE 54: 4-[2-(Méthyl-1,2,3,4-tétrahydro-2-naphtalényl)méthyl]4,5-dihydro-*1H*-imidazole, fumarate (diastéréoisomère 2)**

On procède comme aux stades 5 à 8 de l'Exemple 45 à partir du (2-méthyl-1,2,3,4-tétrahydronaphtalèn-2-yl) acétaldéhyde.

Point de fusion : 152°C

| Microanalyse élémentaire: | | | |
|---|---|---|---|
| | C% | H% | N% |
| Théorique | 66,26 | 7,02 | 8,13 |
| Trouvée | 66,22 | 7,36 | 8,27 |

**ETUDE PHARMACOLOGIQUE**

**EXEMPLE A : Détermination de l'affinité pour les récepteurs adrénergiques $\alpha_2$ chez le rat**

L'affinité a été déterminée par des expériences de compétition avec le [$^3$H]-RX 821,002). Les membranes sont préparées à partir de cortex cérébral de rat et incubées en triple avec 0,4 nM de [$^3$H]-RX 821,002 et le composé à tester dans un volume final de 1,0 ml, pendant 60 minutes à 22°C. Le tampon d'incubation contient 50 nM de TRIS-HCl (pH 7,5), 1 mM de EDTA et 100 µM de GppNHp. La fixation non spécifique est déterminée avec 10 µM de phentolamine.

**Analyse de données**

A la fin de l'incubation, le milieu d'incubation est filtré au travers de filtres WHATMAN GF/B imprégnés avec 0,1 % de polyéthylénimine et lavé trois fois avec 5 ml de tampon refroidi. La radioactivité retenue sur les filtres est déterminée par comptage du liquide de scintillation. Les isothermes de binding sont analysées par régression non-linéaire.

**Résultat**

Les composés de l'invention montrent une activité antagoniste spécifique des récepteurs α2-adrénergiques avec, par exemple pour le composé de l'Exemple 22 un pKi de 8,8.

**EXEMPLE B : Détermination de l'affinité pour les sites de recapture de la noradrenaline chez le rat**

L'affinité a été déterminée par des expériences de compétition avec [$^3$H]-nisoxetine. Les membranes sont préparées à partir de cortex frontal de rat et incubées en triple avec 2 nM de [$^3$H]-nisoxetine et le composé à tester dans un volume final de 0,5 ml, pendant 4 heures à 4°C. Le tampon d'incubation contient 50 mM de TRIS-HCl (pH 7,4), 120 mM de NaCl et 5 mM de KCl. La fixation non-spécifique est déterminée avec 10 µM de desipramine.

**Analyse de données**

A la fin de l'incubation, le milieu d'incubation est filtré au travers de filtres WHATMAN GF/B imprégnés avec 0,1 % de polyéthylénimine et lavé trois fois avec 5 ml de tampon refroidi. La radioactivité retenue sur les filtres est déterminée par comptage du liquide de scintillation. Les isothermes de binding sont analysées par régression non-linéaire.

**Résultat**

Les composés de la présente invention montrent une très bonne affinité pour les sites de recapture de la noradrénaline. A titre d'Exemple, le pKi du composé de l'Exemple 2 est de 7,8.

**EXEMPLE C : Détermination de l'affinité pour les sites de recapture de la sérotonine chez le rat**

L'affinité a été déterminée par des expériences de compétition avec le [$^3$H]-paroxetine. Les membranes sont préparées à partir de cortex frontal de rat et incubées en triple avec 0,25 nM de [$^3$H]-paroxetine et le composé à tester dans un volume final de 0,4 ml, pendant 2 heures à 25°C. Le tampon d'incubation contient 50 mM de TRIS-HCl (pH 7,4), 120 mM de NaCl et 5 mM de KCl. La fixation non-spécifique est déterminée avec 10 µM de citalopram.

**Analyse de données**

A la fin de l'incubation, le milieu d'incubation est filtré au travers de filtres WHATMAN GF/B imprégnés avec 0,1 % de polyéthylénimine et lavé trois fois avec 5 ml de tampon refroidi. La radioactivité retenue sur les filtres est déterminée par comptage du liquide de scintillation. Les isothermes de binding sont analysées par régression non-linéaire.

**Résultat**

Les composés de la présente invention montrent une très bonne affinité pour les sites de recapture de la sérotonine. A titre d'Exemple, le pKi du composé de l'Exemple 12 est de 7,8.

**EXEMPLE D : Composition pharmaceutique : Comprimés**

| | |
|---|---|
| 1000 comprimés dosés à 5 mg de 4-[(8-méthoxy-3,4-dihydro-2-naphtalényl)méthyl]-4,5-dihydro-*1H*-imidazole, fumarate (Exemple 12) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

**1.** Composés de formule (I):

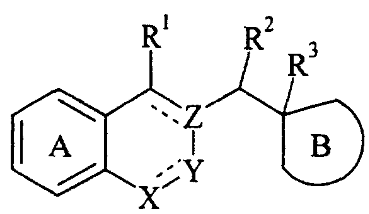

(I)

dans laquelle :

- ♦ A représente un noyau benzénique substitué ou non par 1 à 4 groupements identiques ou différents choisis parmi alkyle ($C_1$-$C_6$) linéaire ou ramifié, alkoxy ($C_1$-$C_6$) linéaire ou ramifié, hydroxy, polyhalogénoalkyles ($C_1$-$C_6$) linéaire ou ramifié, cyano, nitro, amino, alkylamino, dialkylamino, thioalkyle, sulfonylalkyle, sulfinylalkyle, carboxy, alkyloxycarbonyle, alkylcarbonyloxy, formyle, carbamoyle, carboxamide, phényle, benzyle, ou

atomes d'halogène,

- B représente un cycle imidazolinique tel que représenté dans les formules (Ia) ou (Ib)

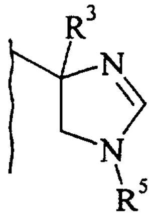

(Ia)

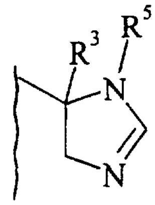

(Ib)

- X représente
  - un groupement $CR^6$ ou $CR^6R^7$ (où $R^6$ et $R^7$, identiques ou différents, représentent un atome d'hydrogène ou un groupement alkyle ($C_1$-$C_6$) linéaire ou ramifié),
  - un atome d'azote ou un groupement $NR^8$ (dans lequel $R^8$ représente un atome d'hydrogène ou un groupement alkyle ($C_1$-$C_6$) linéaire ou ramifié ou benzyle),
  - un atome d'oxygène,
  - un atome de soufre ou un groupement SO ou $SO_2$,

- Y représente un groupement CH ou $CH_2$ ou une liaison simple (et dans ce cas le cycle contenant X, Y et Z est représenté par la formule (Ic):

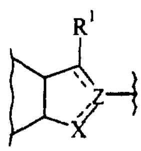

(Ic) )

- Z représente un atome de carbone ou un groupement $CR^4$ dans lequel $R^4$ représente un atome d'hydrogène ou un groupement alkyle ($C_1$-$C_6$) linéaire ou ramifié,

- $R^1$, $R^2$, $R^3$, identiques ou différents, représentent un atome d'hydrogène ou un groupement alkyle ($C_1$-$C_6$) linéaire ou ramifié,
  les groupements ($R^2$ et $R^4$) ou ($R^1$ et $R^4$) pouvant former un cyclopropane,

- $R^5$ représente un atome d'hydrogène, un groupement alkyle ($C_1$-$C_6$) linéaire ou ramifié ou benzyle,

- la représentation ---- signifie que les liaisons peuvent être simples ou doubles, étant entendu que la valence des atomes est respectée,

étant entendu que par alkyle on entend un groupement alkyle linéaire ou ramifié contenant de 1 à 6 atomes de carbone,
leurs tautomères, énantiomères et diastéréoisomères, ainsi que leurs sels d'addition à un acide ou à une base pharmaceutiquement acceptable.

**2.** Composés de formule (I) selon la revendication 1 pour lesquels $R^5$ représente un atome d'hydrogène, leurs tautomères, énantiomères et diastéréoisomères, ainsi que leurs sels d'addition à un acide ou à une base pharmaceutiquement acceptable.

**3.** Composés de formule (I) selon la revendication 1 pour lesquels $R^1$, $R^2$ et $R^3$ représentent simultanément un atome d'hydrogène et Z représente un atome de carbone ou un groupement CH, leurs tautomères, énantiomères et diastéréoisomères, ainsi que leurs sels d'addition à un acide ou à une base pharmaceutiquement acceptable.

**4.** Composés de formule (I) selon la revendication 1 pour lesquels $R^1$ et $R^2$ représentent simultanément un atome d'hydrogène et $R^3$ ou $R^4$ représente un groupement méthyle, leurs tautomères, énantiomères et diastéréoisomères, ainsi que leurs sels d'addition à un acide ou à une base pharmaceutiquement acceptable.

**5.** Composés de formule (I) selon la revendication 1 pour lesquels ($R^1$ et $R^4$) ou ($R^2$ et $R^4$) représentent un cyclopropane, leurs tautomères, énantiomères et diastéréoisomères, ainsi que leurs sels d'addition à un acide ou à une base pharmaceutiquement acceptable.

**6.** Composés de formule (I) selon la revendication 1 pour lesquels le système cyclique

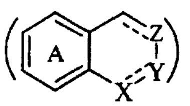

représente le 3,4-dihydronaphtalène, le 1,2,3,4-tétrahydronaphtalène, l'indane, l'indène ou le benzofurane, leurs tautomères, énantiomères et diastéréoisomères, ainsi que leurs sels d'addition à un acide ou à une base pharmaceutiquement acceptable.

**7.** Composés de formule (I) selon la revendication 1 pour lesquels le système cyclique

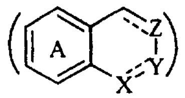

représente le 3,4-dihydronaphtalène, le 1,2,3,4-tétrahydronaphtalène, l'indane, l'indène ou le benzofurane, ces systèmes cycliques étant non substitués, leurs tautomères, énantiomères et diastéréoisomères, ainsi que leurs sels d'addition à un acide ou à une base pharmaceutiquement acceptable.

**8.** Composés de formule (I) selon la revendication 1 pour lesquels le système cyclique

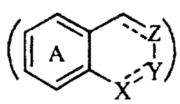

représente le 3,4-dihydronaphtalène, le 1,2,3,4-tétrahydronaphtalène, l'indane, l'indène ou le benzofurane, ces systèmes cycliques étant substitués par un ou plusieurs substituants, identiques ou différents, choisis parmi l'atome d'halogène, les groupements méthyle, méthoxy ou $CF_3$, leurs tautomères, énantiomères et diastéréoisomères, ainsi que leurs sels d'addition à un acide ou à une base pharmaceutiquement acceptable.

**9.** Composé de formule (I) selon la revendication 1 qui est le 4-(3,4-dihydro-2-naphtalénylméthyl)-4,5-dihydro-*1H*-imidazole, ses tautomères, énantiomères et diastéréoisomères, ainsi que ses sels d'addition à un acide ou à une base pharmaceutiquement acceptable.

**10.** Composé de formule (I) selon la revendication 1 qui est le 4-[(8-chloro-3,4-dihydro-2-naphtalényl)méthyl]-4,5-dihydro-*1H*-imidazole, ses tautomères, énantiomères et diastéréoisomères, ainsi que ses sels d'addition à un acide ou à une base pharmaceutiquement acceptable.

**11.** Composé de formule (I) selon la revendication 1 qui est le 4-[(8-méthoxy-3,4-dihydro-2-naphtalényl)méthyl]-4,5-dihydro-*1H*-imidazole, ses tautomères, énantiomères et diastéréoisomères, ainsi que ses sels d'addition à un acide ou à une base pharmaceutiquement acceptable.

**12.** Composé de formule (I) selon la revendication 1 qui est le 4-(benzo[*b*]furan-2-ylméthyl)-4,5-dihydro-*1H*-imidazole, ses tautomères, énantiomères et diastéréoisomères, ainsi que ses sels d'addition à un acide ou à une base pharmaceutiquement acceptable.

**13.** Composés de formule (I) selon la revendication 1 qui sont le 4-{spiro[cyclopropan-2: 2'-(1',2',3',4'-tétrahydronaphtalèn)]-1-yl}-4,5-dihydro-*1H*-imidazole (isomère 1) et le 4-{spiro[cyclopropan-2:2'-(1',2',3',4'-tétrahydronaphtalèn)]-1-yl}-4,5-dihydro-*1H*-imidazole (isomère 2), ses tautomères, énantiomères et diastéréoisomères, ainsi que ses sels d'addition à un acide ou à une base pharmaceutiquement acceptable.

**14.** Composés de formule (I) selon la revendication 1 qui sont le 4-[1,2,3,4-tétrahydro-2-naphtalénylméthyl]-4,5-dihydro-*1H*-imidazole (isomère 1), le 4-[1,2,3,4-tétrahydro-2-naphtalényl-méthyl]-4,5-dihydro-*1H*-imidazole (isomère 2), le 4-[1,2,3,4-tétrahydro-2-naphtalénylméthyl]-4,5-dihydro-*1H*-imidazole (isomère 3), le 4-[1,2,3,4-tétrahydro-2-naphtalénylméthyl]-4,5-dihydro-*1H*-imidazole (isomère 4), leurs tautomères, ainsi que leurs sels d'addition à un acide ou à une base pharmaceutiquement acceptable.

**15.** Composé de formule (I) selon la revendication 1 qui est le 4-(1,3-dihydro-*1H*-2-indénylméthyl)-4,5-dihydro-*1H*-imidazole, ses tautomères, énantiomères et diastéréoisomères, ainsi que ses sels d'addition à un acide ou à une base pharmaceutiquement acceptable.

**16.** Composé de formule (I) selon la revendication 1 qui est le 4-[(3,4-dihydro-4,4-diméthyl-2-naphtalényl)méthyl]-4,5-dihydro-*1H*-imidazole, ses tautomères, énantiomères et diastéréoisomères, ainsi que ses sels d'addition à un acide ou à une base pharmaceutiquement acceptable.

**17.** Composé de formule (I) selon la revendication 1 qui est le 4-[(7-fluoro-3,4-dihydro-2-naphtalényl)méthyl]-4,5-dihydro-*1H*-imidazole, ses tautomères, énantiomères et diastéréoisomères, ainsi que ses sels d'addition à un acide ou à une base pharmaceutiquement acceptable.

**18.** Composé de formule (I) selon la revendication 1 qui est 4-[(8-chloro-2-naphtyl) méthyl]-4,5-dihydro-*1H*-imidazole, ses tautomères, énantiomères et diastéréoisomères, ainsi que ses sels d'addition à un acide ou à une base pharmaceutiquement acceptable.

**19.** Composés de formule (I) selon la revendication 1 qui sont le 4-{spiro[cyclopropan-2':2"-(5",6"-difluoro-indan)]-1'-yl}-4,5-dihydro-*1H*-imidazole (diastéréoisomère 1), le 4-{spiro[cyclopropan-2':2"-(5",6"-difluoro-indan)]-1'-yl}-4,5-dihydro-*1H*-imidazole (diastéréoisomère 2), leurs tautomères, ainsi que leurs sels d'addition à un acide ou à une base pharmaceutiquement acceptable.

**20.** Composé de formule (I) selon la revendication 1 qui est le 4-[(5-fluoro-2,3-dihydro-*1H*-indèn-2-yl)méthyl]-4,5-dihydro-*1H*-imidazole, ses tautomères, énantiomères et diastéréoisomères, ainsi que ses sels d'addition à un acide ou à une base pharmaceutiquement acceptable.

**21.** Composé de formule (I) selon la revendication 1 qui est le 4-[(5,6-Difluoro-2,3-dihydro-*1H*-indèn-2-yl)méthyl]-4,5-dihydro-*1H*-imidazole, ses tautomères, énantiomères et diastéréoisomères, ainsi que ses sels d'addition à un acide ou à une base pharmaceutiquement acceptable.

**22.** Procédé de préparation des composés de formule (I) selon la revendication 1 **caractérisé en ce que** l'on prend comme produit de départ un composé de formule (II):

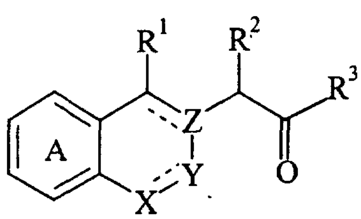

(II)

dans laquelle A, X, Y, Z, $R^1$, $R^2$, $R^3$ et la représentation ---- sont définis comme précédemment,
que l'on soumet soit aux conditions de la réaction de Strecker (KCN, $NH_4Cl$), soit à l'action d'une amine $R_aNH_2$

(dans laquelle $R_a$ représente un atome d'hydrogène ou un groupement protecteur encombré permettant une meilleure séparation, lorsqu'il y a lieu, des diastéréoisomères formés) et de cyanure de triméthylsilyle afin de conduire au composé de formule (III):

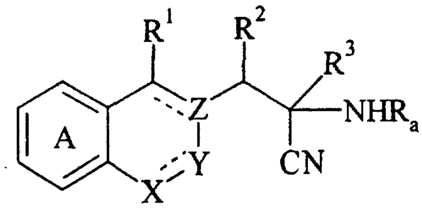

(III)

dans laquelle A, X, Y, Z, $R^1$, $R^2$, $R^3$, $R_a$ et la représentation ---- sont définis comme précédemment, composé de formule (III) qui peut être par ailleurs obtenu, lorsque $R_a$ représente un atome d'hydrogène, à partir du composé de formule (IV) :

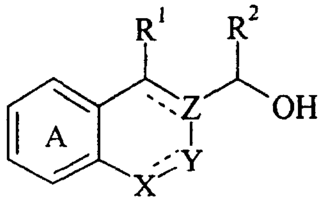

(IV)

dans laquelle A, X, Y, Z, $R^1$, $R^2$ et la représentation ---- sont définis comme précédemment, que l'on soumet à un agent d'halogénation comme $CBr_4/P(Ph)_3$ pour conduire au composé de formule (V):

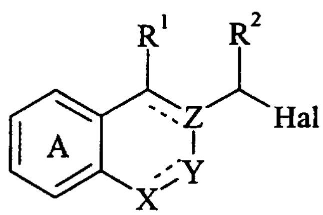

(V)

dans laquelle A, X, Y, Z, $R^1$, $R^2$ et la représentation ---- ont la même définition que précédemment et Hal représente un atome d'halogène,
sur lequel on condense un dérivé de formule (VI):

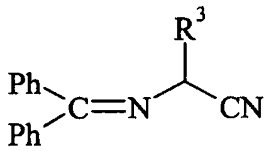

(VI)

dans laquelle $R^3$ est tel que défini précédemment, pour conduire au composé de formule (VII):

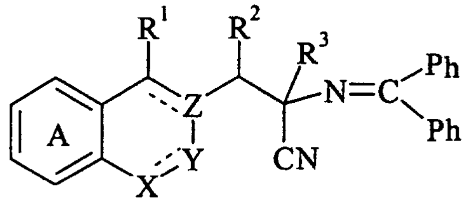

(VII)

dans laquelle A, X, Y, Z, $R^1$, $R^2$, $R^3$ et la représentation ---- sont tels que définis précédemment, que l'on soumet à une hydrolyse acide pour conduire au composé de formule (III/a), cas particulier des composés de formule (III):

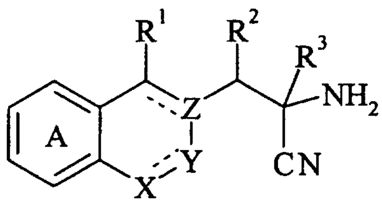

(III/a)

dans laquelle A, X, Y, Z, $R^1$, $R^2$, $R^3$ et la représentation ---- sont définis comme précédemment composé de formule (III) que l'on soumet à l'action de $LiAlH_4$ ou $AlH_3$ ou à une hydrogénation catalytique au nickel ou cobalt, ou palladium sur charbon ou ruthénium ou rhodium pour obtenir le composé de formule (VIII):

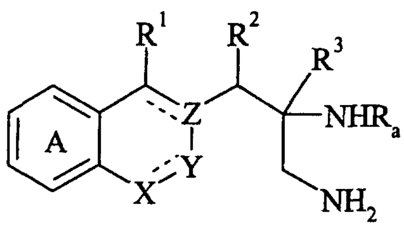

(VIII)

dans laquelle A, X, Y, Z, $R^1$, $R^2$, $R^3$, $R_a$ et la représentation ---- ont la même définition que précédemment, que l'on déprotège dans le cas ou $R_a$ est différent d'un atome d'hydrogène en milieu acide acétique aqueux, ou sous hydrogène en présence de palladium sur charbon avec une quantité catalytique d'acide chlorhydrique, en présence de formiate d'ammonium, ou en présence d'une quantité catalytique de palladium sur charbon dans un solvant tel que l'éthanol pour conduire au composé de formule (IX):

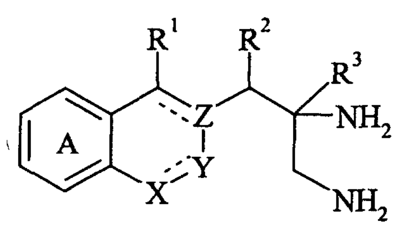

(IX)

dans laquelle A, X, Y, Z, $R^1$, $R^2$, $R^3$ et la représentation ---- sont définis comme précédemment, qui est mis en réaction avec de l'acétate de formamidine ou de l'orthoformiate de méthyle, pour aboutir au composé de formule (I/a), cas particulier des composés de formule (I):

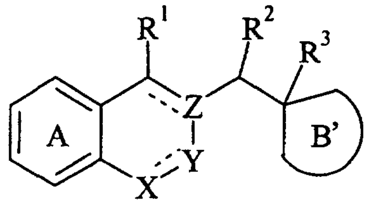

(I/a)

dans laquelle A, X, Y, Z, $R^1$, $R^2$, $R^3$, et la représentation ---- ont la même signification que précédemment, et B' représente un cycle imidazolinique non substitué tel que représenté dans les formules (Ia/a) ou (Ib/a):

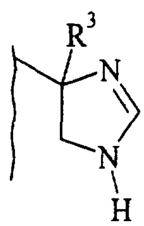

(Ia/a)

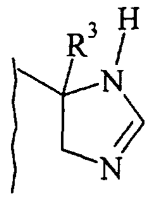

(Ib/a)

que l'on peut soumettre, en présence d'un système basique, à l'action d'un composé de formule (X):

$$R^5_a - J \qquad (X)$$

dans laquelle $R^5_a$ représente un groupement alkyle ($C_1$-$C_6$) linéaire ou ramifié ou benzyle et J représente un groupe partant comme un atome d'halogène ou un groupement tosyle, afin d'obtenir le composé de formule (I/b), cas particulier des composés de formule (I):

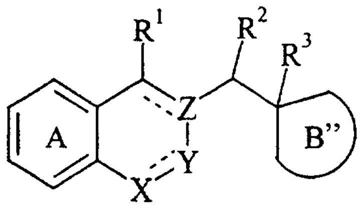

(I/b)

dans laquelle A, X, Y, Z, $R^1$, $R^2$, $R^3$, et la représentation ---- ont la même définition que précédemment, et B" représente un cycle imidazolinique substitué tel que représenté dans les formules (Ia/b) ou (Ib/b)

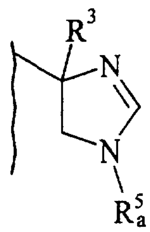

(Ia/b)

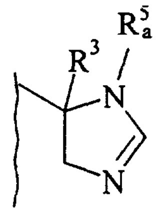

(Ib/b)

où $R^5_a$ est tel que défini précédemment, les composés de formule (I/a) et (I/b) formant l'ensemble des composés de formule (I) et pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

**23.** Procédé de préparation des composés de formule (I/a) selon la revendication 22 **caractérisé en ce que** l'on utilise comme produit de départ le composé de formule (II'):

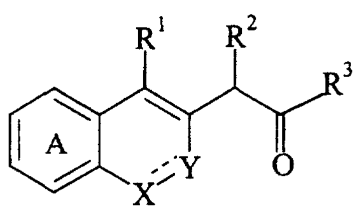

(II')

dans laquelle A, X, Y, $R^1$, $R^2$, $R^3$ et la représentation ---- sont définis comme précédemment, que l'on condense sur un composé aminé chiral de formule (XI):

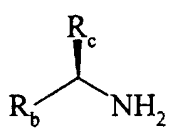

(XI)

dans laquelle $R_b$ et $R_c$, différents, représentent un groupement alkyle ou aryle, pour obtenir le composé de formule (XII):

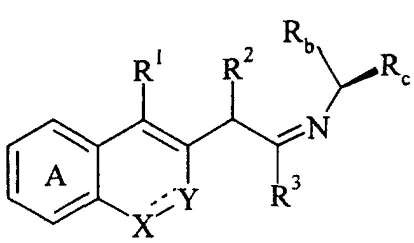

(XII)

dans laquelle A, X, Y, $R^1$, $R^2$, $R^3$, $R_b$, $R_c$ et la représentation ---- ont la même définition que précédemment, que l'on soumet à l'action deN-1-isocyano-cyclohexene et d'acide benzoique pour conduire au composé de formule (XIII):

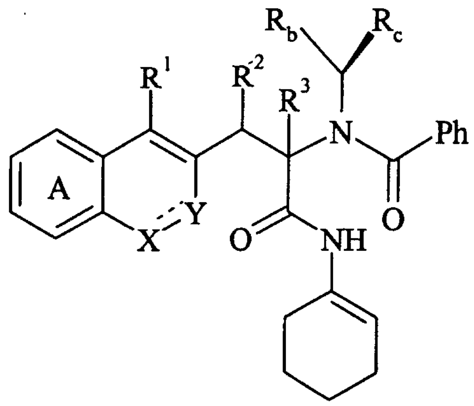

(XIII)

dans laquelle A, X, Y, $R^1$, $R^2$, $R^3$, $R_b$, $R_c$ et la représentation ---- sont tels que définis précédemment, composé de formule (XIII) que l'on chromatographie sur silice pour conduire aux diastéréoisomères de formules ($XIII_a$) et ($XIII_b$):

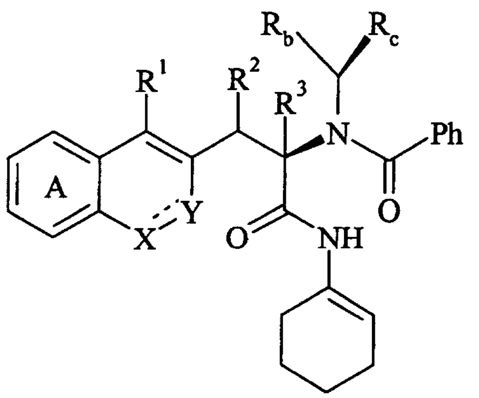

($XIII_a$)

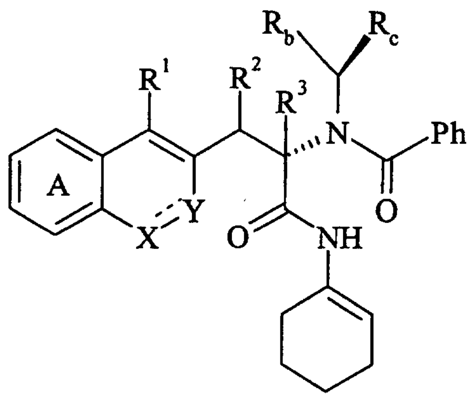

($XIII_b$)

dans lesquelles A, X, Y, $R^1$, $R^2$, $R^3$, $R_b$, $R_c$ et la représentation ---- sont définis comme précédemment, composé de formule ($XIII_a$) que l'on soumet à l'action d'un acide fort tel que l'acide chlorhydrique concentré pour obtenir le composé de formule ($XIV_a$) :

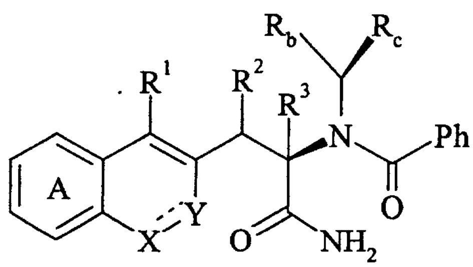

($XIV_a$)

dans laquelle A, X, Y, $R^1$, $R^2$, $R^3$, $R_b$, $R_c$ et la représentation ---- sont tels que définis précédemment,

- que l'on soumet à une hydrogénation catalytique pour conduire au composé de formule ($XV_a$) :

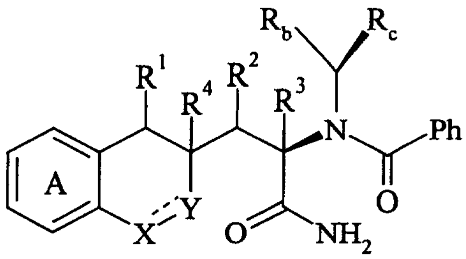

(XV$_a$)

dans laquelle A, X, Y, $R^1$, $R^2$, $R^3$, $R^4$, $R_b$, $R_c$ et la représentation ---- sont tels que définis précédemment, composé de formule (XV$_a$) que l'on chromatographie sur silice pour conduire aux diastéréoisomères de formules (XV$_a$') et (XV$_a$") :

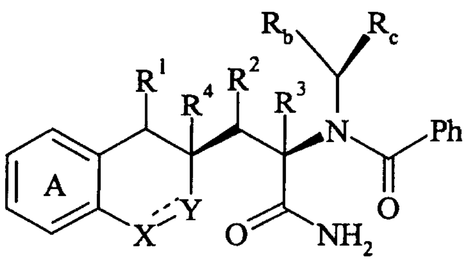

(XV$_a$')

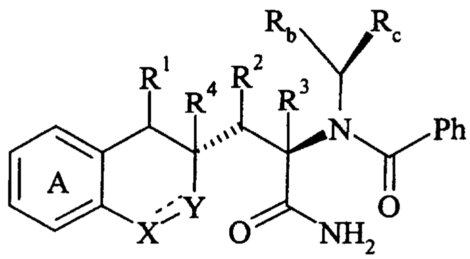

(XV$_a$")

dans lesquelles A, X, Y, $R^1$, $R^2$, $R^3$, $R^4$, $R_b$, $R_c$ et la représentation ---- ont la même signification que précédemment,
composé de formule (XVa') que l'on soumet successivement à l'action de $BH_3$, puis à une hydrogénolyse pour conduire au composé de formule (XVI$_a$') :

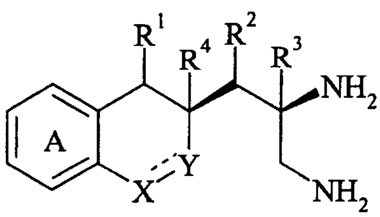

(XVI$_a$')

dans laquelle A, X, Y, $R^1$, $R^2$, $R^3$, $R^4$ et la représentation ---- sont tels que définis précédemment,
qui est mis en réaction avec de l'acétate de formamidine ou de l'orthoformiate de méthyle pour obtenir le composé de formule (XVII$_a$'), cas particulier des composés de formule (I/a):

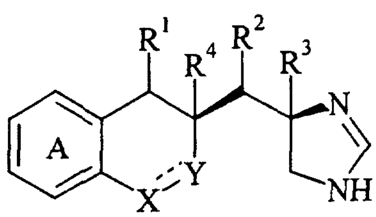

(XVII$_a$')

dans laquelle A, X, Y, $R^1$, $R^2$, $R^3$, $R^4$ et la représentation ---- sont tels que définis précédemment,
la même suite réactionnelle permettant d'obtenir:

- le composé de formule (XVII$_a$"), cas particulier des composés de formule (I/a), obtenu à partir du

diastéréoisomère ($XV_a$"):

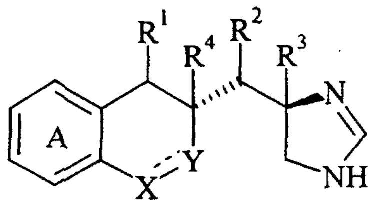

($XVII_a$")

dans laquelle A, X, Y, $R^1$, $R^2$, $R^3$, $R^4$ et la représentation ---- sont tels que définis précédemment,

➢ le composé de formule ($XVII_b$'), cas particulier des composés de formule (I/a), obtenu à partir du diastéréoisomère ($XIII_b$):

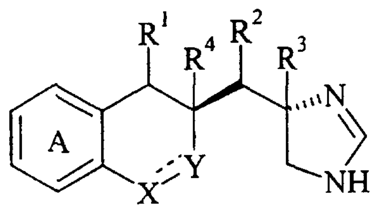

($XVII_b$')

dans laquelle A, X, Y, $R^1$, $R^2$, $R^3$, $R^4$ et la représentation ---- sont tels que définis précédemment,

➢ le composé de formule ($XVII_b$"), cas particulier des composés de formule (I/a), obtenu à partir du diastéréoisomère ($XIII_b$) :

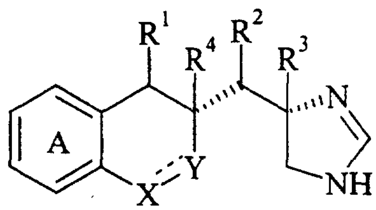

($XVII_b$")

dans laquelle A, X, Y, $R^1$, $R^2$, $R^3$, $R^4$ et la représentation ---- sont tels que définis précédemment,

- ou composé de formule ($XIV_a$) que l'on soumet successivement à l'action de $BH_3$, puis à une hydrogénolyse pour conduire au composé de formule ($XVIII_a$):

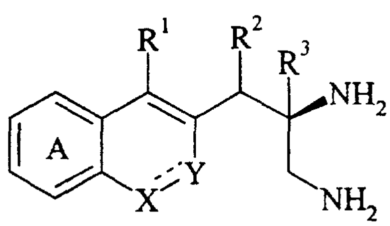

($XVIII_a$)

dans laquelle A, X, Y, $R^1$, $R^2$, $R^3$ et la représentation ---- sont tels que définis précédemment, qui est mis en réaction avec de l'acétate de formamidine ou de l'orthoformiate de méthyle pour obtenir le composé de formule ($XIX_a$), cas particulier des composés de formule (I/a):

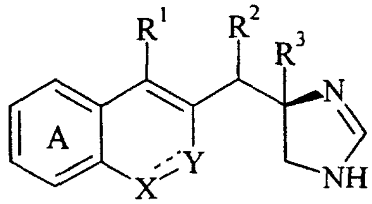

$(XIX_a)$

dans laquelle A, X, Y, $R^1$, $R^2$, $R^3$ et la représentation ----sont tels que définis précédemment, la même suite réactionnelle permettant d'obtenir le composé de formule $(XIX_b)$, cas particulier des composés de formule (I/a) obtenu à partir du composé $(XIV_b)$ correspondant :

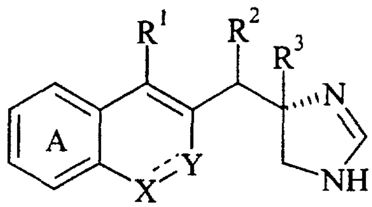

$(XIX_b)$

dans laquelle A, X, Y, $R^1$, $R^2$, $R^3$ et la représentation ---- sont tels que définis précédemment,
les composés de formules $(XVII_a')$, $(XVII_a'')$, $(XVII_b')$, $(XVII_b'')$, $(XIX_a)$ et $(XIX_b)$ pouvant être purifiés selon une technique classique de séparation que l'on transforme, si on le souhaite, en leurs sels d'addition à un acide ou à une base pharmaceutiquement acceptable.

**24.** Compositions pharmaceutiques contenant comme principe actif au moins un des composés de formule (I) selon l'une quelconque des revendications 1 à 21 ou un de ses sels d'addition avec un acide ou une base pharmaceutiquement acceptable en combinaison avec un ou plusieurs excipients pharmaceutiquement acceptables.

**25.** Compositions pharmaceutiques selon la revendication 24 utiles pour la fabrication d'un médicament pour le traitement de la dépression, de l'obésité, des attaques de panique, de l'anxiété, des troubles obsessionnels compulsifs, des troubles cognitifs, des phobies, des troubles impulsifs de l'abus et du sevrage de drogue, des dysfonctionnements sexuels et de la maladie de Parkinson.

**Patentansprüche**

**1.** Verbindungen der Formel (I):

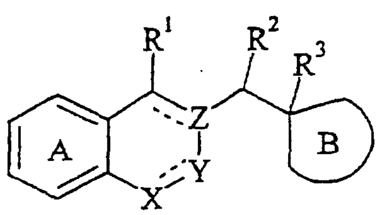

(I)

in der:

- A einen Benzolkern, der gegebenenfalls durch 1 bis 4 gleichartige oder verschiedenartige Gruppen ausgewählt aus geradkettigem oder verzweigtem $(C_1-C_6)$-Alkyl, geradkettigem oder verzweigtem $(C_1-C_6)$-Alkoxy, Hydroxy, geradkettigem oder verzweigtem $(C_1-C_6)$-Polyhalogenalkyl, Cyano, Nitro, Amino, Alkylamino, Dialkylamino, Thioalkyl, Sulfonylalkyl, Sulfinylalkyl, Carboxy, Alkyloxycarbonyl, Alkylcarbonyloxy, Formyl, Carbamoyl, Carboxamid, Phenyl, Benzyl oder Halogenatomen substituiert ist, bedeutet,
- B einen Imidazolinring der Formel (Ia) oder (Ib) bedeutet:

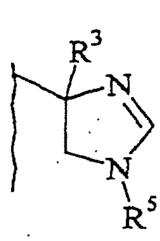

(Ia)

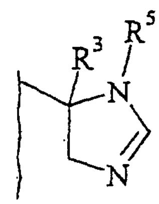

(Ib)

- X:
  - eine Gruppe $CR^6$ oder $CR^6R^7$ (worin $R^6$ und $R^7$, die gleichartig oder verschieden sind, ein Wasserstoffatom oder eine geradkettige oder verzweigte ($C_1$-$C_6$)-Alkylgruppe bedeuten),
  - ein Stickstoffatom oder eine Gruppe $NR^8$ (worin $R^8$ ein Wasserstoffatom oder eine geradkettige oder verzweigte ($C_1$-$C_6$-Alkylgruppe oder eine Benzylgruppe darstellt),
  - ein Sauerstoffatom,
  - ein Schwefelatom oder eine Gruppe SO oder $SO_2$ bedeutet,

- Y eine Gruppe CH oder $CH_2$ oder eine Einfachbindung (wobei in diesem Fall der Ring, der X, Y und Z enthält, durch die Formel (Ic) wiedergegeben wird:

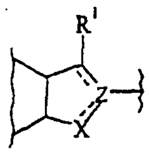

(Ic) )

  bedeutet;
- Z ein Kohlenstoffatom oder eine Gruppe $CR^4$, worin $R^4$ ein Wasserstoffatom oder eine geradkettige oder verzweigte ($C_1$-$C_6$)-Alkylgruppe darstellt, bedeutet,
- $R^1$, $R^2$ und $R^3$, die gleichartig oder verschieden sind, ein Wasserstoffatom oder eine geradkettige oder verzweigte ($C_1$-$C_6$)-Alkylgruppe bedeuten, wobei die Gruppen ($R^2$ und $R^4$) oder ($R^1$ und $R^4$) einen Cyclopropanrest bilden können,
- $R^5$ ein Wasserstoffatom, eine geradkettige oder verzweigte ($C_1$-$C_6$)-Alkylgruppe oder eine Benzylgruppe darstellt,
- das Symbol ---- bedeutet, daß die Bindungen einfach oder doppelt sein können, mit der Maßgabe, daß die Wertigkeit der Atome berücksichtigt ist,

mit der Maßgabe, daß man unter Alkyl eine geradkettige oder verzweigte Alkylgruppe mit 1 bis 6 Kohlenstoffatomen versteht,
deren Tautomere, Enantiomere und Diastereoisomere sowie deren Additionssalze mit einer pharmazeutisch annehmbaren Säure oder Base.

**2.** Verbindungen der Formel (I) nach Anspruch 1, worin $R^5$ ein Wasserstoffatom bedeutet, deren Tautomere, Enantiomere und Diastereoisomere sowie deren Additionssalze mit einer pharmazeutisch annehmbaren Säure oder Base.

**3.** Verbindungen der Formel (I) nach Anspruch 1, worin $R^1$, $R^2$ und $R^3$ gleichzeitig ein Wasserstoffatom und Z ein Kohlenstoffatom oder eine Gruppe CH bedeuten, deren Tautomere, Enantiomere und Diastereoisomere sowie deren Additionssalze mit einer pharmazeutisch annehmbaren Säure oder Base.

**4.** Verbindungen der Formel (I) nach Anspruch 1, worin $R^1$ und $R^2$ gleichzeitig ein Wasserstoffatom und $R^3$ oder $R^4$ eine Methylgruppe bedeuten, deren Tautomere, Enantiomere und Diastereoisomere sowie deren Additionssalze mit einer pharmazeutisch annehmbaren Säure oder Base.

**5.** Verbindungen der Formel (I) nach Anspruch 1, worin ($R^1$ und $R^4$) oder ($R^2$ und $R^4$) einen Cyclopropanrest bedeuten, deren Tautomere, Enantiomere und Diastereoisomere sowie deren Additionssalze mit einer pharmazeutisch annehmbaren Säure oder Base.

**6.** Verbindungen der Formel (I) nach Anspruch 1, worin das cyclische System

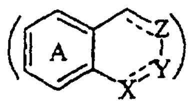

3,4-Dihydronaphthalin, 1,2,3,4-Tetrahydronaphthalin; Indan, Inden oder Benzofuran bedeutet, deren Tautomere, Enantiomere und Diastereoisomere sowie deren Additionssalze mit einer pharmazeutisch annehmbaren Säure oder Base.

**7.** Verbindungen der Formel (I) nach Anspruch 1, worin das cyclische System

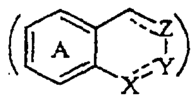

3,4-Dihydronaphthalin, 1,2,3,4-Tetrahydronaphthalin . Indan, Inden oder Benzofuran bedeutet, wobei diese cyclischen Systeme nicht substituiert sind, deren Tautomere, Enantiomere und Diastereoisomere sowie deren Additionssalze mit einer pharmazeutisch annehmbaren Säure oder Base.

**8.** Verbindungen der Formel (I) nach Anspruch 1, worin das cyclische System

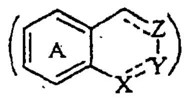

3,4-Dihydronaphthalin, 1,2,3,4-Tetrahydronaphthalin, Indan, Inden oder Benzofuran bedeutet, wobei diese cyclischen Systeme durch einen oder mehrere gleichartige oder verschiedene Substituenten ausgewählt aus Halogenatomen, Methyl-, Methoxy- oder $CF_3$-Gruppen substituiert sind, deren Tautomere, Enantiomere und Diastereoisomere sowie deren Additionssalze mit einer pharmazeutisch annehmbaren Säure oder Base.

**9.** Verbindung der Formel (I) nach Anspruch 1, nämlich 4-(3,4-Dihydro-2-naphthalinylmethyl)-4,5-dihydro-*1H*-imidazol, dessen Tautomere, Enantiomere und Diastereoisomere sowie dessen Additionssalze mit einer pharmazeutisch annehmbaren Säure oder Base.

**10.** Verbindung der Formel (I) nach Anspruch 1, nämlich 4-[(8-Chlor-3,4-dihydro-2-naphthalinyl)-methyl]-4,5-dihydro-*1H*-imidazol, dessen Tautomere, Enantiomere und Diastereoisomere sowie dessen Additionssalze mit einer pharmazeutisch annehmbaren Säure oder Base.

**11.** Verbindung der Formel (I) nach Anspruch 1, nämlich 4-[(8-Methoxy-3,4-dihydro-2-naphthalinyl)-methyl]-4,5-dihydro-*1H*-imidazol, dessen Tautomere, Enantiomere und Diastereoisomere sowie dessen Additionssalze mit einer pharmazeutisch annehmbaren Säure oder Base.

**12.** Verbindung der Formel (I) nach Anspruch 1, nämlich 4-(Benzo[b]furan-2-ylmethyl)-4,5-dihydro-*1H*-imidazol, dessen Tautomere, Enantiomere und Diastereoisomere sowie dessen Additionssalze mit einer pharmazeutisch annehmbaren Säure oder Base.

**13.** Verbindungen der Formel (I) nach Anspruch 1, nämlich 4-{Spiro[cyclopropan-2:2'-(1',2',3',4'-tetrahydronaphthalin))-1-yl)-4,5-dihydro-*1H*-imidazol (Isomeres 1) und 4-{Spiro[cyclopropan-2:2'-(1',2',3',4'-tetrahydronaphthalin)]-1-yl}-4,5-dihydro-*1H*-imidazol (Isomeres 2), deren Tautomere, Enantiomere und Diastereoisomere sowie deren

Additionssalze mit einer pharmazeutisch annehmbaren Säure oder Base.

**14.** Verbindungen der Formel (I) nach Anspruch 1, nämlich 4-[1,2,3,4-Tetrahydro-2-naphthalinylmethyl]-4,5-dihydro-*1H*-imidazol (Isomeres 1), 4-[1, 2, 3, 4-Tetrahydro-2-naphthalinylmethyl]-4,5-dihydro-*1H*-imidazol (Isomeres 2), 4-[1,2,3,4-Tetrahydro-2-naphthalinylmethyl]-4,5-dihydro-*1H*-imidazol (Isomeres 3), 4-[1,2,3,4-Tetrahydro-2-naphthalmylmethyl]-4,5-dihydro-*1H*-imidazol (Isomeres 4), deren Tautomere sowie deren Additionssalze mit einer pharmazeutisch annehmbaren Säure oder Base.

**15.** Verbindung der Formel (I) nach Anspruch 1, nämlich 4-(1,3-Dihydro-*1H*-2-indenylmethyl)-4,5-dihydro-*1H*-imidazol, dessen Tautomere, Enantiomere und Diastereoisomere sowie dessen Additionssalze mit einer pharmazeutisch annehmbaren Säure oder Base.

**16.** Verbindung der Formel (I) nach Anspruch 1, nämlich 4-[(3,4-Dihydro-4,4-dimethyl-2-naphthalinyl)-methy]-4,5-dihydro-*1H*-imidazol, dessen Tautomere, Enantiomere und Diastereoisomere sowie dessen Additionssalze mit einer pharmazeutisch annehmbaren Säure oder Base.

**17.** Verbindung der Formel (I) nach Anspruch 1, nämlich 4-[(7-Fluor-3,4-dihydro-2-naphthalinyl)-methyl] 4,5-dihydro-*1H*-imidazol, dessen Tautomere, Enantiomere und Diastereoisomere sowie dessen Additionssalze mit einer pharmazeutisch annehmbaren Säure oder Base.

**18.** Verbindung der Formel (I) nach Anspruch 1, nämlich 4-[(8-Chlor-2-naphthyl)-methyl]-4,5-dihydro-*1H*-imidazol, dessen Tautomere, Enantiomere und Diastereoisomere sowie dessen Additionssalze mit einer pharmazeutisch annehmbaren Säure oder Base.

**19.** Verbindungen der Formel (I) nach Anspruch 1, nämlich 4-{Spiro[cyclopropan-2':2"-(5",6"-difluor-indan)]-1'-yl}-4,5-dihydro-*1H*-imidazol (Diastereoisomeres 1), 4-{Spiro[cyclopropan-2':2"-(5",6"-difluor-indan)]-1'-yl}-4,5-dihydro-*1H*-imidazol (Diastereoisomeres 2), deren Tautomere, Enantiomere und Diastereoisomere sowie deren Additionssalze mit einer pharmazeutisch annehmbaren Säure oder Base.

**20.** Verbindung der Formel (I) nach Anspruch 1, nämlich 4-[[5-Fluor-2,3-dihydro-*1H*-inden-2-yl)-methyl]-4,5-dihydro-*1H*-imidazol, dessen Tautomere, Enantiomere und Diastereoisomere sowie dessen Additionssalze mit einer pharmazeutisch annehmbaren Säure oder Base.

**21.** Verbindung der Formel (I) nach Anspruch 1, nämlich 4-[(5,6-Difluor-2,3-dihydro-*1H*-inden-2-yl)-methyl]-4,5-dihydro-*1H*-imidazol, dessen Tautomere, Enantiomere und Diastereoisomere sowie dessen Additionssalze mit einer pharmazeutisch annehmbaren Säure oder Base.

**22.** Verfahren zur Herstellung der Verbindungen der Formel (I) nach Anspruch 1, **dadurch gekennzeichnet, daß** man als Ausgangsprodukt eine Verbindung der Formel (II) einsetzt:

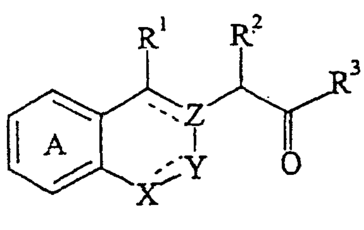

(II)

in der A, X, Y, Z, $R^1$, $R^2$, $R^3$ und das Symbol ---- die oben angegebenen Bedeutungen besitzen, welche man entweder den Bedingungen der Strecker-Reaktion (KCN, $NH_4Cl$) oder der Einwirkung eines Amins $R_aNH_2$ (worin $R_a$ ein Wasserstoffatom oder eine sperrige Schutzgruppe, welche eine bessere Trennung der gebildeten Diastereoisomeren ermöglicht, wenn diese durchgeführt wird) und Trimethylsilylcyanid unterwirft zur Bildung der Verbindung der Formel (III):

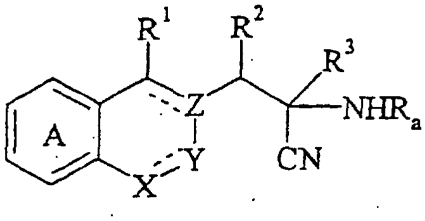

(III)

in der A, X, Y, Z, $R^1$, $R^2$, $R^3$, $R_a$ und das Symbol ---- die oben angegebenen Bedeutungen besitzen, welche Verbindung der Formel (III), die dann, wenn $R_a$ ein Wasserstoffatom bedeutet, auch ausgehend von der Verbindung der Formel (IV):

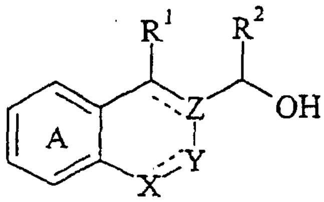

(IV)

in der A, X, Y, Z, $R^1$, $R^2$ und das Symbol ---- die oben angegebenen Bedeutungen besitzen, erhalten werden kann, man der Einwirkung eines Halogenierungsmittels, wie $CBr_4/P(Ph)_3$, unterwirft zur Bildung der Verbindung der Formel (V):

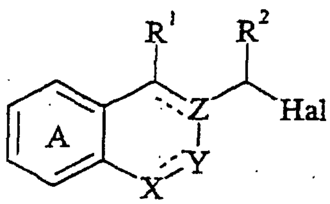

(V)

in der A, X, Y, Z, $R^1$, $R^2$ und das Symbol ---- die oben angegebenen Bedeutungen besitzen und Hal ein Halogenatom darstellt,
welches man mit einem Derivat der Formel (VI):

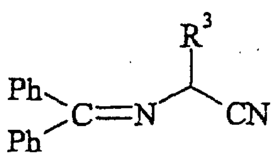

(VI)

in der $R^3$ die oben angegebenen Bedeutungen besitzt, kondensiert zur Bildung der Verbindung der Formel (VII):

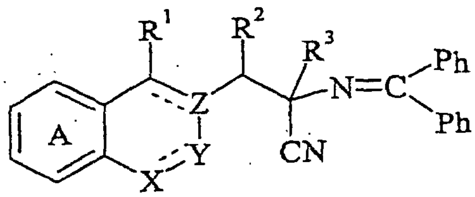

(VII)

in der A, X, Y, Z, $R^1$, $R^2$, $R^3$ und das Symbol ---- die oben angegebenen Bedeutungen besitzen,
welche man einer sauren Hydrolyse unterzieht zur Bildung der Verbindung der Formel (III/a), einem Sonderfall

der Verbindungen der Formel (III):

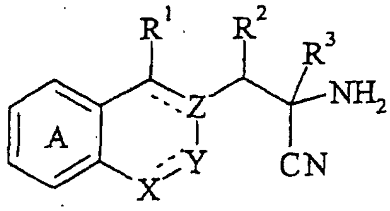

(III/a)

in der A, X, Y, Z, $R^1$, $R^2$, $R^3$ und das Symbol ---- die oben angegebenen Bedeutungen besitzen, welche Verbindung der Formel (III) man der Einwirkung von $LiAlH_4$ oder $AlH_3$ oder einer katalytischen Hydrierung mit Nickel oder Kobalt, oder Palladium-auf-Kohlenstoff oder Ruthenium oder Rhodium unterwirft zur Bildung der Verbindung der Formel (VIII):

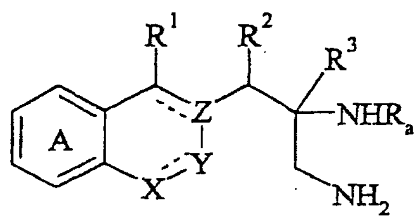

(VIII)

in der A, X, Y, Z, $R^1$, $R^2$, $R^3$, $R_a$ und das Symbol ---- die oben angegebenen Bedeutungen besitzen, von welcher man, wenn $R_a$ von einem Wasserstoff verschieden ist, die Schutzgruppe in essigsaurem Medium oder unter Wasserstoff in Gegenwart von Palladiumauf-Kohlenstoff mit einer katalytischen Menge Chlorwasserstoffsäure, in Gegenwart von Ammoniumformiat oder in Gegenwart einer katalytischen Menge Palladium-auf-Kohlenstoff in einem Lösungsmittel, wie Ethanol, abspaltet zur Bildung der Verbindung der Formel (IX):

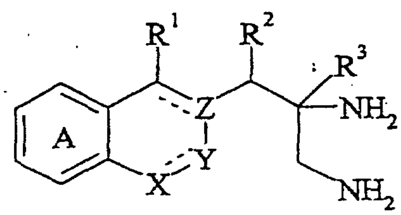

(IX)

in der A, X, Y, Z, $R^1$, $R^2$, $R^3$ und das Symbol ---- die oben angegebenen Bedeutungen besitzen, welche man mit Formamidinacetat oder Orthoameisensäuremethylester umsetzt zur Bildung der Verbindung der Formel (I/a), einem Sonderfall der Verbindungen der Formel (I):

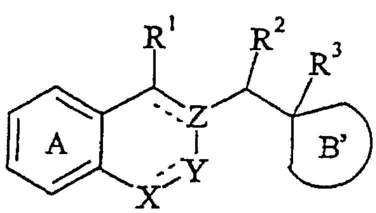

(I/a)

in der A, X, Y, Z, R1, $R^2$, $R^3$ und das Symbol ---- die oben angegebenen Bedeutungen besitzen und B' einen nichtsubstituierten Imidazolinring bedeutet, wie er in den Formeln (Ia/a) oder (Ib/a) dargestellt ist:

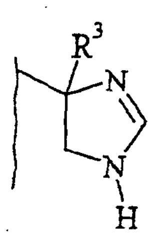

(Ia/a)

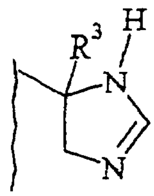

(Ib/a)

welche man in Gegenwart eines basischen Mittels der Einwirkung einer Verbindung der Formel (X):

$$R^5_a\text{-J} \qquad (X)$$

in der $R^5_a$ eine geradkettige oder verzweigte ($C_1$-$C_6$)-Alkylgruppe oder eine Benzylgruppe und J eine austretende Gruppe, wie ein Halogenatom oder eine Tosylgruppe bedeuten, unterwerfen kann zur Bildung der Verbindung der Formel (I/b), einem Sonderfall der Verbindungen der Formel (1):

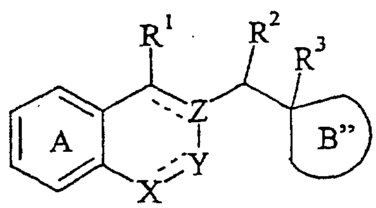

(I/b)

in der A, X, Y, Z, $R^1$, $R^2$, $R^3$ und das Symbol ---- die oben angegebenen Bedeutungen besitzen und B" eine substituierten Imidazolinring bedeutet, wie er durch die Formeln (Ia/b) oder (Ib/b) wiedergegeben wird:

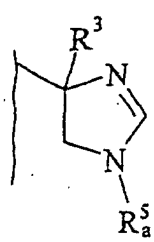

(Ia/b)

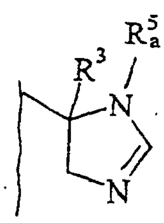

(Ib/b)

worin $R^5_a$ die oben angegebenen Bedeutungen besitzt,
wobei die Verbindungen der Formeln (I/a) und (I/b) die Gesamtheit der Verbindungen der Formel (I) bilden und mit Hilfe einer klassischen Trennmethode gereinigt werden können, gewünschtenfalls in ihre Additionssalze mit einer pharmazeutisch annehmbaren Säure oder Base umgewandelt werden können oder gegebenenfalls mit Hilfe einer klassischen Trennmethode in ihre Isomeren aufgetrennt werden können.

**23.** Verfahren zur Herstellung der Verbindungen der Formel (I/a) nach Anspruch 22, **dadurch gekennzeichnet, daß** als Ausgangsprodukt die Verbindung der Formel (II') verwendet:

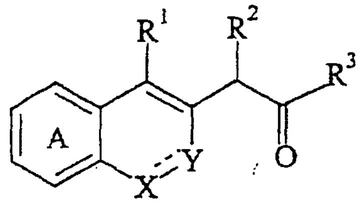

(II')

in der A, X, Y, $R^1$, $R^2$, $R^3$ und das Symbol ---- die oben angegebenen Bedeutungen besitzen, welche man mit einer chiralen Aminverbindung der Formel (XI):

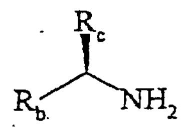

(XI)

in der $R_b$ und $R_c$, die verschieden sind, eine Alkyl- oder Arylgruppe bedeuten, kondensiert, zur Bildung der Verbindung der Formel (XII):

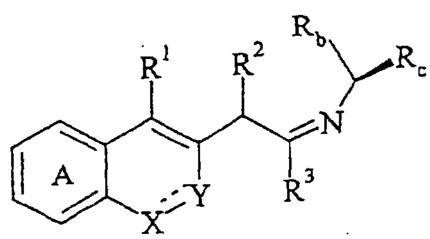

(XII)

in der A, X, Y, $R^1$, $R^2$, $R^3$, $R_b$, $R_c$ und das Symbol ---- die oben angegebenen Bedeutungen besitzen, welche man der Einwirkung von N-1-Isocyano-cyclohexen und Benzoesäure unterwirft zur Bildung der Verbindung der Formel (XIII):

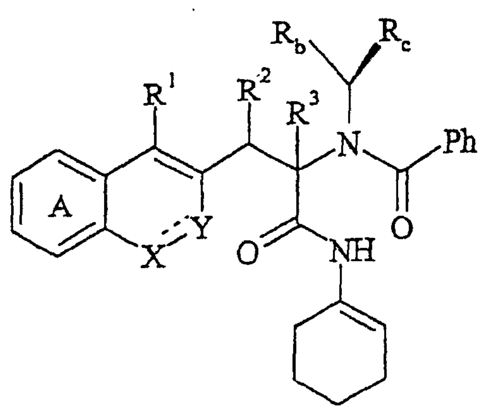

(XIII)

in der A, X, Y, $R^1$, $R^2$, $R^3$, $R_b$, $R_c$ und das Symbol ---- die oben angegebenen Bedeutungen besitzen, welche Verbindung der Formel (XIII) man über Siliciumdioxid chromatographiert zur Bildung der Diastereoisomeren der Formeln ($XIII_a$) und ($XIII_b$):

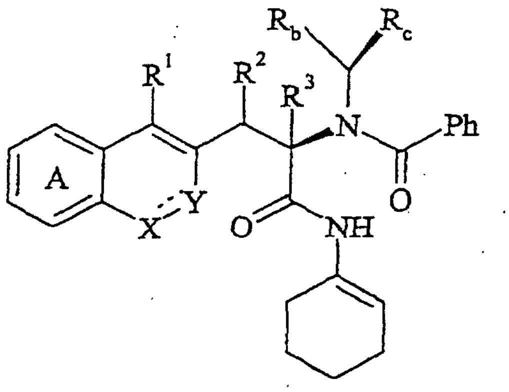

(XIIIa)

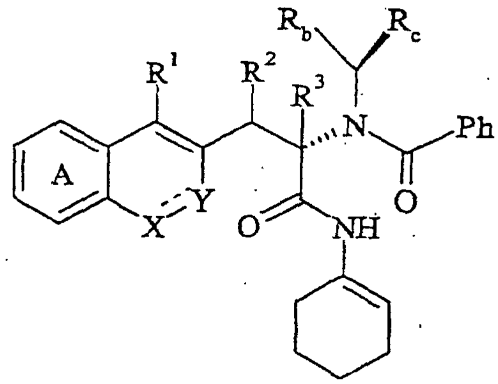

(XIIIb)

worin A, X, Y, $R^1$, $R^2$, $R^3$, $R_b$, $R_c$ und das Symbol ---- die oben angegebenen Bedeutungen besitzen, welche Verbindung der Formel (XIIIa) man der Einwirkung einer starken Säure, wie konzentrierter Chlorwasserstoffsäure, unterwirft zur Bildung der Verbindung der Formel ($XIV_a$):

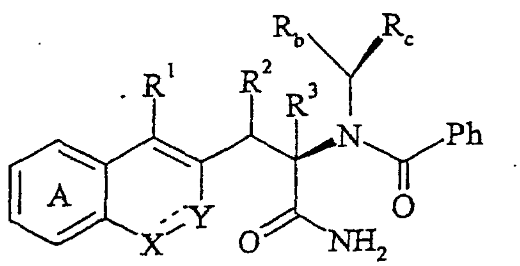

($XIV_a$)

in der A, X, Y, $R^1$, $R^2$, $R^3$, $R_b$, $R_c$ und das Symbol ---- die oben angegebenen Bedeutungen besitzen,

- welche man einer katalytischen Hydrierung unterwirft zur Bildung der Verbindung der Formel ($XV_a$):

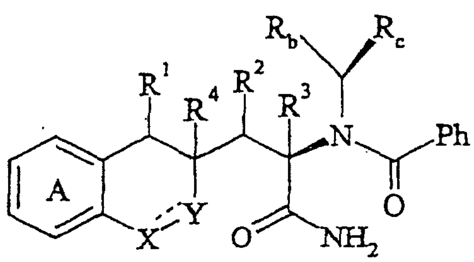

($XV_a$)

in der A, X, Y, $R^1$, $R^2$, $R^3$, $R^4$, $R_b$, $R_c$ und das Symbol ---- die oben angegebenen Bedeutungen besitzen, welche Verbindung der Formel ($XV_a$) man über Siliciumdioxid chromatographiert zur Bildung der Diastereoisomeren der Formeln ($XV_a$') und ($XV_a$''):

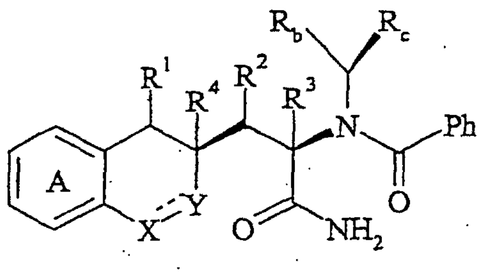

(XV$_a$')

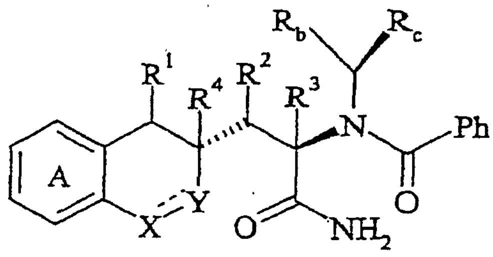

(XV$_a$")

worin A, X, Y, $R^1$, $R^2$, $R^3$, $R^4$, $R_b$, $R_c$ und das Symbol ---- die oben angegebenen Bedeutungen besitzen, welche Verbindung der Formel (XV$_a$') man nacheinander der Einwirkung von $BH_3$ und dann einer Hydrogenolyse unterwirft zur Bildung der Verbindung der Formel (XVIa'):

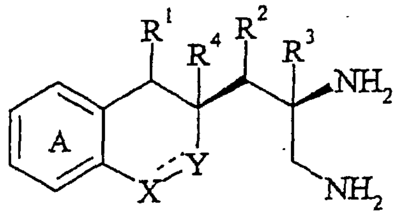

(XVI$_a$')

in der A, X, Y, $R^1$, $R^2$, $R^3$, $R^4$ und das Symbol ---- die oben angegebenen Bedeutungen besitzen, welche man mit Formamidinacetat oder Orthoameisensäuremethylester umsetzt zur Bildung der Verbindung der Formel (XVII$_a$'), einem Sonderfall der Verbindungen der Formel (I/a):

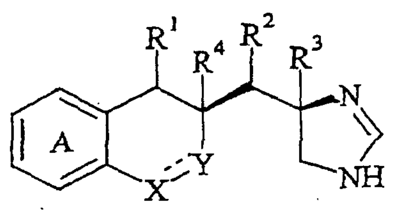

(XVII$_a$')

in der A, X, Y, $R^1$, $R^2$, $R^3$, $R^4$ und das Symbol ---- die oben angegebenen Bedeutungen besitzen, wobei man mit Hilfe der gleichen Reaktionsfolge erhalten kann:

➢ die Verbindung der Formel (XVII$_a$ "), einem Sonderfall der Verbindungen der Formel (I/a), welche man ausgehend von dem Diastereoisomeren (XV$_a$") erhält:

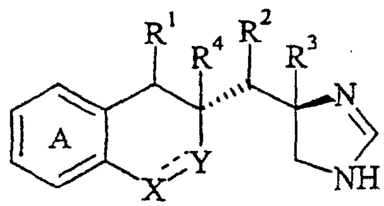

(XVII$_a$")

in der A, X,Y, $R^1$, $R^2$, $R^3$, $R^4$ und das Symbol ---- die oben angegebenen Bedeutungen besitzen,

➢ die Verbindung der Formel (XVII$_b$'), einem Sonderfall der Verbindungen der Formel (I/a), welche man ausgehend von dem Diastereoisomeren (XIII$_b$) erhält:

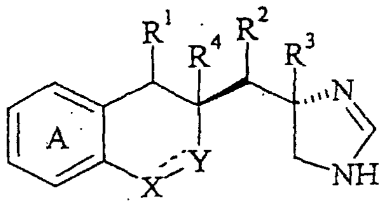

(XVII$_b$')

in der A, X, Y, $R^1$, $R^2$, $R^3$, $R^4$ und das Symbol ---- die oben angegebenen Bedeutungen besitzen,

➢ die Verbindung der Formel (XVII$_b$"), einem Sonderfall der Verbindungen der Formel (I/a), welche man ausgehend von dem Diastereoisomeren (XIII$_b$) erhält:

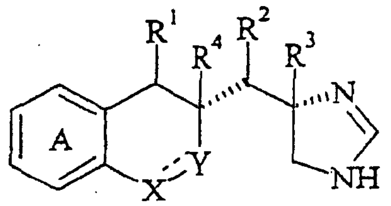

(XVII$_b$")

in der A, X, Y, $R^1$, $R^2$, $R^3$, $R^4$ und das Symbol ---- die oben angegebenen Bedeutungen besitzen,

- oder man die Verbindung der Formel (XIV$_a$) nacheinander der Einwirkung von $BH_3$ und dann einer Hydrogenolyse unterwirft zur Bildung der Verbindung der Formel (XVIII$_a$):

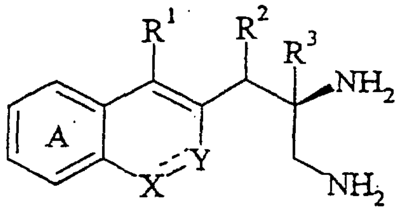

(XVIII$_a$)

in der A, X, Y, $R^1$, $R^2$, $R^3$ und das Symbol ---- die oben angegebenen Bedeutungen besitzen,
welches man mit Formamidinacetat oder Orthoameisensäuremethylester umsetzt zur Bildung der Verbindung der Formel (XIX$_a$), einem Sonderfall der Verbindungen der Formel (I/a):

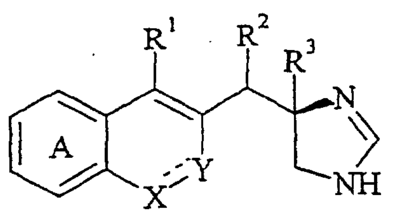

(XIX$_a$)

in der A, X, Y, $R^1$, $R^2$, $R^3$ und das Symbol ---- die oben angegebenen Bedeutungen besitzen,
welche gleiche Reaktionsfolge auch die Bildung der Verbindung der Formel (XIX$_b$) ermöglicht, einem Sonderfall der Verbindungen der Formel (I/a), welche man ausgehend von der Verbindung (XIV$_b$) erhält entsprechend der Formel:

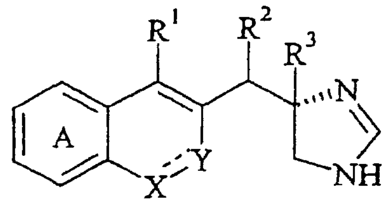

(XIXb)

in der A, X, Y, $R^1$, $R^2$, $R^3$ und das Symbol ---- die oben angegebenen Bedeutungen besitzen,

wobei die Verbindungen der Formeln ($XVII_a$'), ($XVII_a$"), ($XVII_b$'), ($XVII_b$"), ($XIX_a$) und ($XIX_b$) mit Hilfe einer klassischen Trennmethode gereinigt werden können und welche man gewünschtenfalls in ihre Additionssalze mit einer pharmazeutisch annehmbaren Säure oder Base umwandelt.

**24.** Pharmazeutische Zubereitungen enthaltend als Wirkstoff mindestens eine Verbindung der Formel (I) nach einem der Ansprüche 1 bis 21 oder eines ihrer Additionssalze mit einer pharmazeutisch annehmbaren Säure oder Base in Kombination mit einem oder mehreren pharmazeutisch annehmbaren Trägermaterialien.

**25.** Pharmazeutische Zubereitungen nach Anspruch 24 für die Herstellung eines Arzneimittels zur Behandlung von Depressionen, der Fettsucht, von Panikanfällen, der Angst, von krampfartigen Besessenheitsstörungen, von Erkenntnisstörungen, von Phobien, impulsiven Störungen des Drogenmißbrauchs und des Drogenentzugs, sexuellen Dysfunktionen und der Parkinsonschen Krankheit.

## Claims

**1.** Compounds of formula (I):

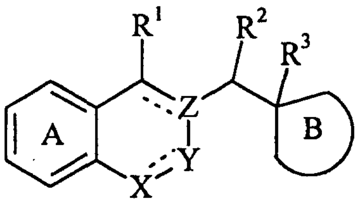

(I),

wherein:

- A represents a benzene ring unsubstituted or substituted by from 1 to 4 identical or different groups selected from linear or branched ($C_1$-$C_6$)alkyl, linear or branched ($C_1$-$C_6$)alkoxy, hydroxy, polyhalo-($C_1$-$C_6$)alkyl in which the alkyl moiety is linear or branched, cyano, nitro, amino, alkylamino, dialkylamino, thioalkyl, sulphonylalkyl, sulphinylalkyl, carboxy, alkoxycarbonyl, alkylcarbonyloxy, formyl, carbamoyl, carboxamide, phenyl, benzyl, and halogen atoms,

- B represents an imidazoline ring as represented in formula (Ia) or (Ib) :

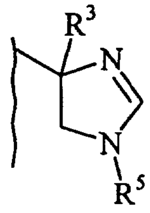

(Ia)

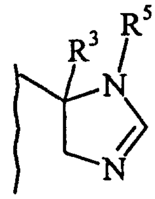

(Ib),

- ♦ X represents
  - a $CR^6$ or $CR^6R^7$ group (wherein $R^6$ and $R^7$, which may be the same or different, each represent a hydrogen atom or a linear or branched $(C_1-C_6)$alkyl group),
  - a nitrogen atom or an $NR^8$ group (wherein $R^8$ represents a hydrogen atom or a linear or branched $(C_1-C_6)$ alkyl group or a benzyl group),
  - an oxygen atom,
  - a sulphur atom or an SO or $SO_2$ group,

- ♦ Y represents a CH or $CH_2$ group or a single bond (and, in that case, the ring containing X, Y and Z is represented by the formula (Ic):

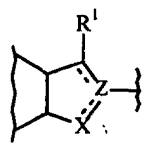

(Ic) ),

- ♦ Z represents a carbon atom or a $CR^4$ group wherein $R^4$ represents a hydrogen atom or a linear or branched $(C_1-C_6)$alkyl group,

- ♦ $R^1$, $R^2$, $R^3$, which may be the same or different, each represent a hydrogen atom or a linear or branched $(C_1-C_6)$alkyl group,
  it being possible for the groups ($R^2$ and $R^4$) or ($R^1$ and $R^4$) to form a cyclopropane group,

- ♦ $R^5$ represents a hydrogen atom, a linear or branched $(C_1-C_6)$alkyl group or a benzyl group,

- ♦ the symbol ---- means that the bonds can be single or double, it being understood that the valency of the atoms is respected,

wherein alkyl is understood to mean a linear or branched alkyl group containing from 1 to 6 carbon atoms,
their tautomers, enantiomers and diastereoisomers, and addition salts thereof with a pharmaceutically acceptable acid or base.

**2.** Compounds of formula (I) according to claim 1, wherein $R^5$ represents a hydrogen atom, their tautomers, enantiomers and diastereoisomers, and addition salts thereof with a pharmaceutically acceptable acid or base.

**3.** Compounds of formula (I) according to claim 1, wherein $R^1$, $R^2$ and $R^3$ each simultaneously represent a hydrogen atom and Z represents a carbon atom or a CH group, their tautomers, enantiomers and diastereoisomers, and addition salts thereof with a pharmaceutically acceptable acid or base.

**4.** Compounds of formula (I) according to claim 1, wherein $R^1$ and $R^2$ each simultaneously represent a hydrogen atom, and $R^3$ or $R^4$ represents a methyl group, their tautomers, enantiomers and diastereoisomers, and addition salts thereof with a pharmaceutically acceptable acid or base.

5. Compounds of formula (I) according to claim 1, wherein ($R^1$ and $R^4$) or ($R^2$ and $R^4$) represent a cyclopropane group, their tautomers, enantiomers and diastereoisomers, and addition salts thereof with a pharmaceutically acceptable acid or base.

6. Compounds of formula (I) according to claim 1, wherein the ring system

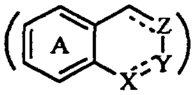

represents 3,4-dihydronaphthalene, 1,2,3,4-tetrahydronaphthalene, indan, indene or benzofuran, their tautomers, enantiomers and diastereoisomers, and addition salts thereof with a pharmaceutically acceptable acid or base.

7. Compounds of formula (I) according to claim 1, wherein the ring system

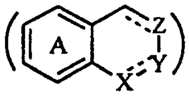

represents 3,4-dihydronaphthalene, 1,2,3,4-tetrahydronaphthalene, indan, indene or benzofuran, those ring systems being unsubstituted, their tautomers, enantiomers and diastereoisomers, and addition salts thereof with a pharmaceutically acceptable acid or base.

8. Compounds of formula (I) according to claim 1, wherein the ring system

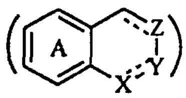

represents 3,4-dihydronaphthalene, 1,2,3,4-tetrahydronaphthalene, indan, indene or benzofuran, those ring systems being substituted by one or more identical or different substituents selected from halogen atoms and methyl, methoxy and $CF_3$ groups, their tautomers, enantiomers and diastereoisomers, and addition salts thereof with a pharmaceutically acceptable acid or base.

9. Compound of formula (I) according to claim 1 which is 4-(3,4-dihydro-2-naphthylmethyl)-4,5-dihydro-*1H*-imidazole, its tautomers, enantiomers and diastereoisomers, and addition salts thereof with a pharmaceutically acceptable acid or base.

10. Compound of formula (I) according to claim 1 which is 4-[(8-chloro-3,4-dihydro-2-naphthyl)methyl]-4,5-dihydro-*1H*-imidazole, its tautomers, enantiomers and diastereoisomers, and addition salts thereof with a pharmaceutically acceptable acid or base.

11. Compound of formula (I) according to claim 1 which is 4-[(8-methoxy-3,4-dihydro-2-naphthyl)methyl]4,5-dihydro-*1H*-imidazole, its tautomers, enantiomers and diastereoisomers, and addition salts thereof with a pharmaceutically acceptable acid or base.

12. Compound of formula (I) according to claim 1 which is 4-(benzo[A]furan-2-ylmethyl)-4,5-dihydro-*1H*-imidazole, its tautomers, enantiomers and diastereoisomers, and addition salts thereof with a pharmaceutically acceptable acid or base.

13. Compounds of formula (I) according to claim 1 which are 4-{spiro[cyclopropan-2:2'-(1',2',3',4'-tetrahydronaphth)]-1-yl}-4,5-dihydro-1*H*-imidazole (isomer 1) and 4-{spiro[cyclopropan-2:2'-(1',2',3',4'-tetrahydronaphth)] 1-yl )-4,5-dihydro-*1H*-imidazole (isomer 2), their tautomers, enantiomers and diastereoisomers, and addition salts thereof with a pharmaceutically acceptable acid or base.

**14.** Compounds of formula (I) according to claim 1 which are 4-[1,2,3,4-tetrahydro-2-naphthylmethyl]-4,5-dihydro-*1H*-imidazole (isomer 1), 4-[1,2,3,4-tetrahydro-2-naphthylmethyl]-4,5-dihydro-*1H*-imidazole (isomer 2), 4-[1,2,3,4-tetrahydro-2-naphthylmethyl]-4,5-dihydro-*1H*-imidazole (isomer 3), 4-[1,2,3,4-tetrahydro-2-naphthylmethyl]-4,5-dihydro-*1H*-imidazole (isomer 4), their tautomers, and addition salts thereof with a pharmaceutically acceptable acid or base.

**15.** Compound of formula (I) according to claim 1 which is 4-(1,3-dihydro-*1H*-2-indenylmethyl)-4,5-dihydro-*1H*-imidazole, its tautomers, enantiomers and diastereoisomers, and addition salts thereof with a pharmaceutically acceptable acid or base.

**16.** Compound of formula (I) according to claim 1 which is 4-[(3,4-dihydro-4,4-dimethyl-2-naphthyl)methyl]-4,5-dihydro-*1H*-imidazole, its tautomers, enantiomers and diastereoisomers, and addition salts thereof with a pharmaceutically acceptable acid or base.

**17.** Compound of formula (I) according to claim 1 which is 4-[(7-fluoro-3,4-dihydro-2-naphthyl)methyl]-4,5-dihydro-*1H*-imidazole, its tautomers, enantiomers and diastereoisomers, and addition salts thereof with a pharmaceutically acceptable acid or base.

**18.** Compound of formula (I) according to claim 1 which is 4-[(8-chloro-2-naphthyl)-methyl]-4,5-dihydro-*1H*-imidazole, its tautomers, enantiomers and diastereoisomers, and addition salts thereof with a pharmaceutically acceptable acid or base.

**19.** Compounds of formula (I) according to claim 1 which are 4-{spiro[cyclopropan-2':2"-(5",6"-difluoro-indan)]-1'-yl}-4,5-dihydro-*1H*-imidazole (diastereoisomer 1), 4-{spiro[cyclopropan-2':2"-(5",6"-difluoro-indan)]-1'-yl}-4,5-dihydro-*1H*-imidazole (diastereoisomer 2), their tautomers, and addition salts thereof with a pharmaceutically acceptable acid or base.

**20.** Compound of formula (I) according to claim 1 which is 4-[(5-fluoro-2,3-dihydro-*1H*-inden-2-yl)methyl]-4,5-dihydro-*1H*-imidazole, its tautomers, enantiomers and diastereoisomers, and addition salts thereof with a pharmaceutically acceptable acid or base.

**21.** Compound of formula (I) according to claim 1 which is 4-[(5,6-difluoro-2,3-dihydro-*1H*-inden-2-yl)methyl]-4,5-dihydro-*1H*-imidazole, its tautomers, enantiomers and diastereoisomers, and addition salts thereof with a pharmaceutically acceptable acid or base.

**22.** Process for the preparation of compounds of formula (I) according to claim 1, **characterised in that** there is used as starting material a compound of formula (II) :

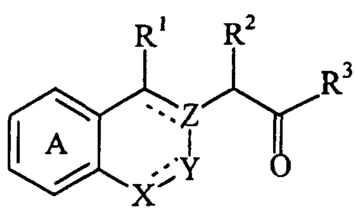

(II),

wherein A, X, Y, Z, $R^1$, $R^2$, $R^3$ and the symbol ---- are as defined hereinbefore,
which is subjected either to the conditions of a Strecker reaction (KCN, $NH_4Cl$) or to the action of an amine $R_aNH_2$ (wherein $R_a$ represents a hydrogen atom or a hindered protecting group allowing better separation, where applicable, of the diastereoisomers formed) and of trimethylsilyl cyanide to yield a compound of formula (III):

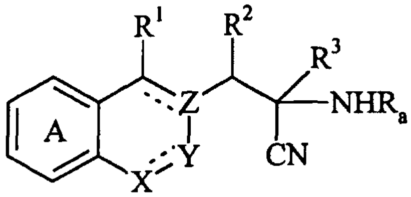

(III),

wherein A, X, Y, Z, $R^1$, $R^2$, $R^3$, $R_a$ and the symbol ---- are as defined hereinbefore, which compound of formula (III), when $R_a$ represents a hydrogen atom, may alternatively be obtained starting from a compound of formula (IV) :

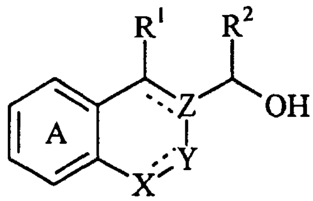

(IV),

wherein A, X, Y, Z, $R^1$, $R^2$ and the symbol ---- are as defined hereinbefore, which is subjected to a halogenating agent such as $CBr_4/P(Ph)_3$ to yield a compound of formula (V):

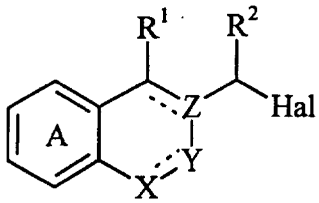

(V),

wherein A, X, Y, Z, $R^1$, $R^2$ and the symbol ---- are as defined hereinbefore and Hal represents a halogen atom, with which there is condensed a compound of formula (VI):

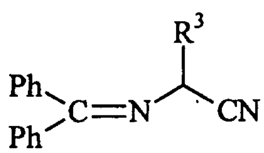

(VI),

wherein $R^3$ is as defined hereinbefore, to yield a compound of formula (VII) :

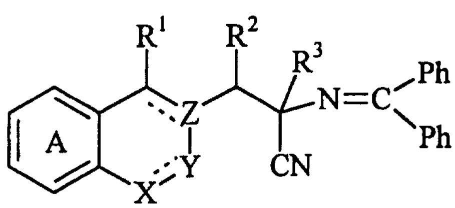

(VII),

wherein A, X, Y, Z, $R^1$, $R^2$, $R^3$ and the symbol ---- are as defined hereinbefore, which is subjected to acid hydrolysis to yield a compound of formula (III/a), a particular case of the compounds of formula (III):

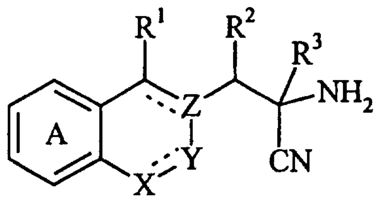

(III/a),

wherein A, X, Y, Z, $R^1$, $R^2$, $R^3$ and the symbol ---- are as defined hereinbefore, which compound of formula (III) is subjected to the action of $LiAlH_4$ or $AlH_3$ or to catalytic hydrogenation using nickel or cobalt or palladium-on-carbon or ruthenium or rhodium to obtain a compound of formula (VIII) :

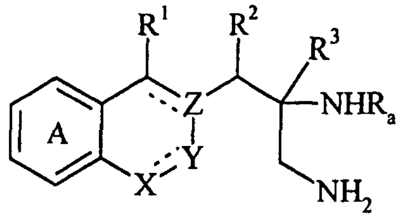

(VIII),

wherein A, X, Y, Z, $R^1$, $R^2$, $R^3$, $R_a$ and the symbol ---- are as defined hereinbefore, which, in the case where $R_a$ is other than a hydrogen atom, is deprotected in an aqueous acetic acid medium, or under hydrogen in the presence of palladium-on-carbon with a catalytic amount of hydrochloric acid, in the presence of ammonium formate, or in the presence of a catalytic amount of palladium-on-carbon in a solvent such as ethanol to yield a compound of formula (IX):

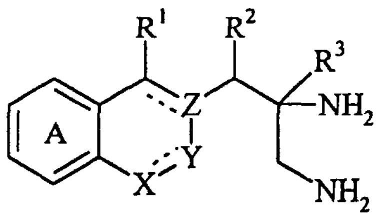

(IX),

wherein A, X, Y, Z, $R^1$, $R^2$, $R^3$ and the symbol ---- are as defined hereinbefore, which is reacted with formamidine acetate or methyl orthoformate to give a compound of formula (I/a), a particular case of the compounds of formula (I) :

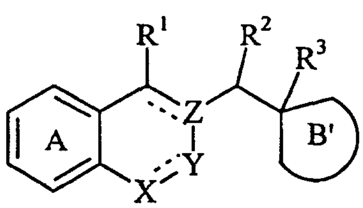

(I/a),

wherein A, X, Y, Z, $R^1$, $R^2$, $R^3$ and the symbol ---- are as defined hereinbefore and B' represents an unsubstituted imidazoline ring as represented in formula (Ia/a) or (Ib/a) :

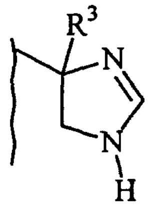

(Ia/a)

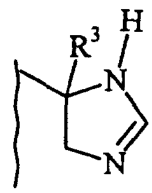

(Ib/a),

which can be subjected, in the presence of a basic system, to the action of a compound of formula (X):

$$R^5_a\text{- J} \qquad \text{(X)},$$

wherein $R^5_a$ represents a linear or branched ($C_1$-$C_6$)alkyl group or a benzyl group and J represents a leaving group such as a halogen atom or a tosyl group, to obtain a compound of formula (I/b), a particular case of the compounds of formula (I):

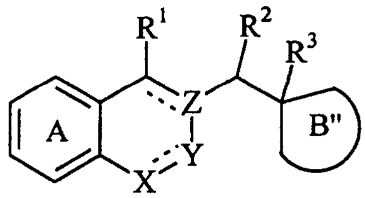

(I/b),

wherein A, X, Y, Z, $R^1$, $R^2$, $R^3$ and the symbol ---- are as defined hereinbefore and B" represents a substituted imidazoline ring as represented in formula (Ia/b) or (Ib/b) :

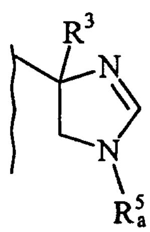

(Ia/b)

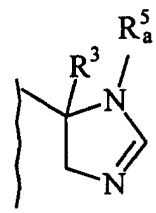

(Ib/b),

wherein $R^5_a$ is as defined hereinbefore,
which compounds of formulae (I/a) and (I/b) constitute the totality of the compounds of formula (I) and may be purified according to a conventional separation technique, are converted, if desired, into their addition salts with a pharmaceutically acceptable acid or base and are separated, where appropriate, into their isomers according to a conventional separation technique.

**23.** Process for the preparation of compounds of formula (I/a) according to claim 22, **characterised in that** there is used as starting material a compound of formula (II'):

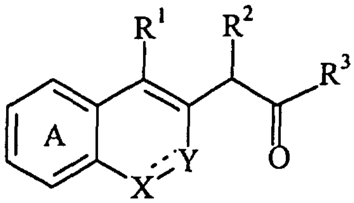

(II'),

wherein A, X, Y, $R^1$, $R^2$, $R^3$ and the symbol ---- are as defined hereinbefore, which is condensed with a chiral amine compound of formula (XI) :

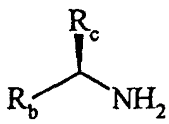

(XI),

wherein $R_b$ and $R_c$, which are different, represent an alkyl or aryl group, to obtain a compound of formula (XII):

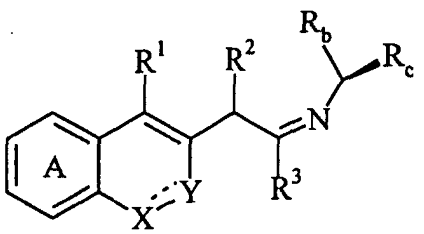

(XII),

wherein A, X, Y, $R^1$, $R^2$, $R^3$, $R_b$, $R_c$ and the symbol ---- are as defined hereinbefore, which is subjected to the action of N-1-isocyano-cyclohexene and benzoic acid to yield a compound of formula (XIII) :

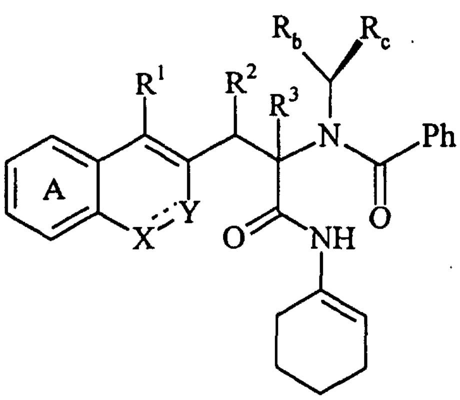

(XIII),

wherein A, X, Y, $R^1$, $R^2$, $R^3$, $R_b$, $R_c$ and the symbol ---- are as defined hereinbefore, which compound of formula (XIII) is chromatographed on silica to yield the diastereoisomers of formulae ($XIII_a$) and ($XIII_b$):

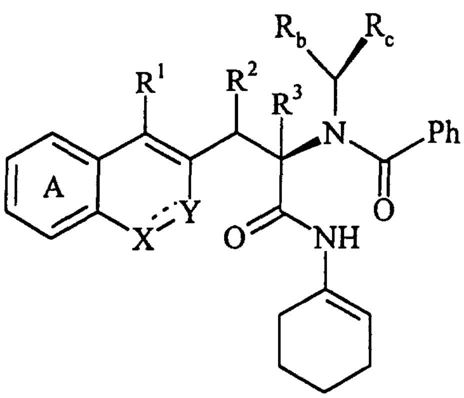

(XIII$_a$)

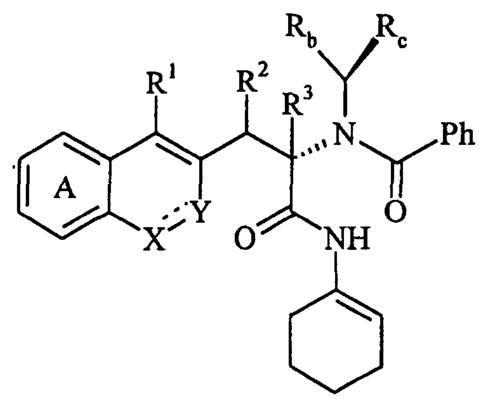

(XIII$_b$),

wherein A, X, Y, $R^1$, $R^2$, $R^3$, $R_b$, $R_c$ and the symbol ---- are as defined hereinbefore, which compound of formula (XIII$_a$) is subjected to the action of a strong acid such as concentrated hydrochloric acid to obtain a compound of formula (XIV$_a$) :

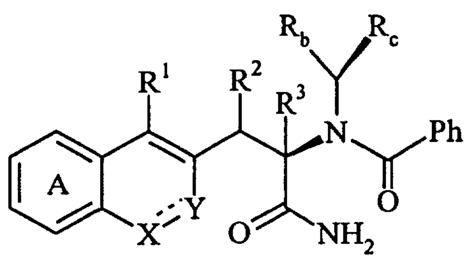

(XIV$_a$),

wherein A, X, Y, $R^1$, $R^2$, $R^3$, $R_b$, $R_c$ and the symbol ---- are as defined hereinbefore,

- which is subjected to catalytic hydrogenation to yield a compound of formula (XV$_a$) :

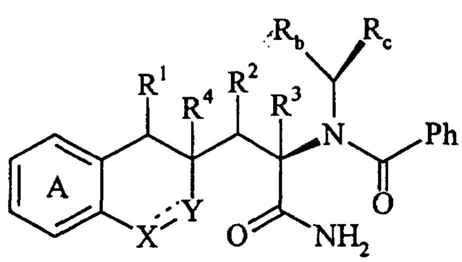

(XV$_a$),

wherein A, X, Y, $R^1$, $R^2$, $R^3$, $R^4$, $R_b$, $R_c$ and the symbol ---- are as defined hereinbefore, which compound of formula (XV$_a$) is chromatographed on silica to yield the diastereoisomers of formulae (XV$_a$') and (XV$_a$"):

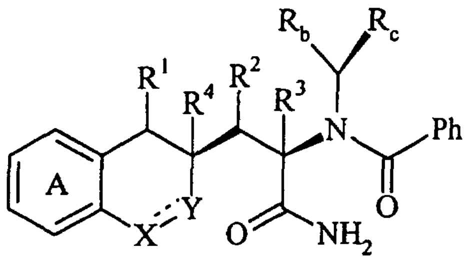

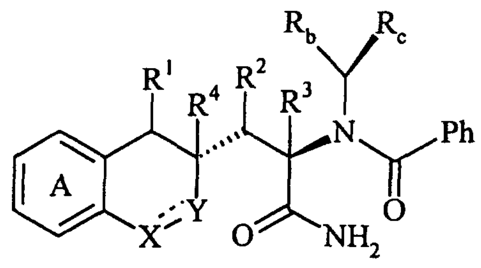

$(XV_a')$ $(XV_a'')$,

wherein A, X, Y, $R^1$, $R^2$, $R^3$, $R^4$, $R_b$, $R_c$ and the symbol ---- are as defined hereinbefore, which compound of formula $(XV_a')$ is subjected, in succession, to the action of $BH_3$ and then to hydrogenolysis to yield a compound of formula $(XVI_a')$:

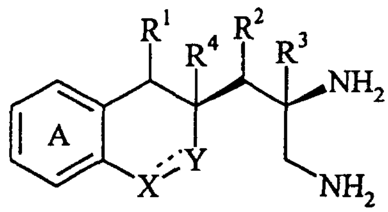

$(XVI_a')$,

wherein A, X, Y, $R^1$, $R^2$, $R^3$, $R^4$ and the symbol ---- are as defined hereinbefore, which is reacted with formamidine acetate or methyl orthoformate to obtain a compound of formula $(XVII_a')$, a particular case of the compounds of formula (I/a) :

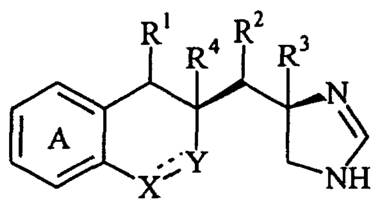

$(XVII_a')$,

wherein A, X, Y, $R^1$, $R^2$, $R^3$, $R^4$ and the symbol ---- are as defined hereinbefore, it being possible, using the same reaction sequence, to obtain:

➢ a compound of formula $(XVII_a'')$, a particular case of the compounds of formula (I/a), obtained starting from the diastereoisomer $(XV_a'')$ :

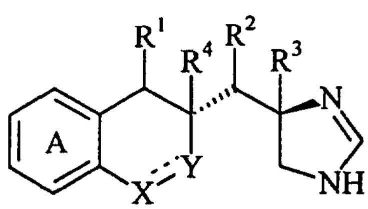

$(XVII_a'')$,

wherein A, X, Y, $R^1$, $R^2$, $R^3$, $R^4$ and the symbol ---- are as defined hereinbefore,

➢ a compound of formula $(XVII_b')$, a particular case of the compounds of formula (I/a), obtained starting from the diastereoisomer $(XIII_b)$:

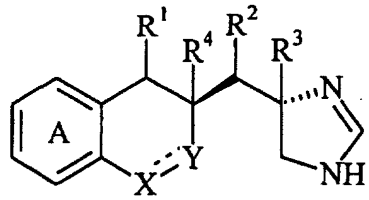

(XVII$_b$'),

wherein A, X, Y, $R^1$, $R^2$, $R^3$, $R^4$ and the symbol ---- are as defined hereinbefore,

➢ a compound of formula (XVII$_b$"), a particular case of the compounds of formula (I/a), obtained starting from the diastereoisomer (XIII$_b$):

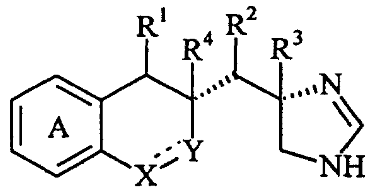

(XVII$_b$"),

wherein A, X, Y, $R^1$, $R^2$, $R^3$, $R^4$ and the symbol ---- are as defined hereinbefore,

- or which compound of formula (XIV$_a$) is subjected, in succession, to the action of $BH_3$ and then to hydrogenolysis to yield a compound of formula (XVIII$_a$) :

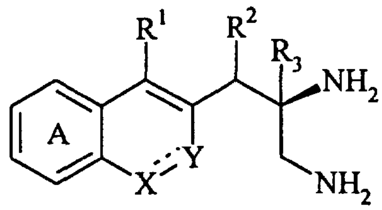

(XVIII$_a$),

wherein A, X, Y, $R^1$, $R^2$, $R^3$ and the symbol ---- are as defined hereinbefore,
which is reacted with formamidine acetate or methyl orthoformate to obtain a compound of formula (XIX$_a$), a particular case of the compounds of formula (I/a):

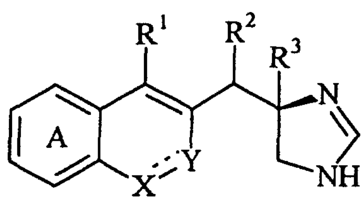

(XIX$_a$),

wherein A, X, Y, $R^1$, $R^2$, $R^3$ and the symbol ---- are as defined hereinbefore,
it being possible, using the same reaction sequence, to obtain a compound of formula (XIX$_b$), a particular case of the compounds of formula (I/a), obtained starting from the corresponding compound (XIV$_b$):

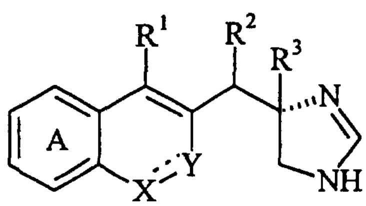

(XIX$_b$),

wherein A, X, Y, $R^1$, $R^2$, $R^3$ and the symbol ---- are as defined hereinbefore,
which compounds of formulae ($XVII_a'$), ($XVII_a''$), ($XVII_b'$), ($XVII_b''$), ($XIX_a$) and ($XIX_b$) may be purified according to a conventional separation technique and converted, if desired, into their addition salts with a pharmaceutically acceptable acid or base.

**24.** Pharmaceutical compositions comprising as active ingredient at least one compound of formula (I) according to any one of claims 1 to 21 or an addition salt thereof with a pharmaceutically acceptable acid or base in combination with one or more pharmaceutically acceptable excipients.

**25.** Pharmaceutical compositions according to claim 24 for use in the manufacture of a medicament for the treatment of depression, obesity, panic attacks, anxiety, obsessive-compulsive disorders, cognitive disorders, phobias, impulsive disorders associated with the abuse of drugs and withdrawal therefrom, sexual dysfunctions and Parkinson's disease.